# EUROPÄISCHE PATENTSCHRIFT

(11) **EP 2 354 235 B1**
(45) Veröffentlichungstag und Bekanntmachung des Hinweises auf die Patenterteilung: **19.11.2014**
(21) Anmeldenummer: 11152912.9
(22) Anmeldetag: 27.09.2006
(51) Int. Cl.: C12P 13/08, C12P 13/22, C12N 1/20, C12P 13/04

(54) **Verfahren zur fermentativen Herstellung von L-Aminosäuren unter Verwendung coryneformer Bakterien**
Method for the fermentative production of L-amino acids involving the use of coryneform bacteria
Méthode de production d'acides aminés L par fermentation au moyen de bactéries corynéformes

(30) Priorität: 05.10.2005 DE 102005047596
(43) Veröffentlichungstag der Anmeldung: 10.08.2011
(62) Teilanmeldung aus: 06806854.3
(73) Patentinhaber: Evonik Degussa GmbH, 45128 Essen (DE); Forschungszentrum Jülich GmbH, 52425 Jülich (DE)
(72) Erfinder: Wendisch, Volker F., 33617 Bielefeld (DE); Rittmann, Doris, 52428 Jülich (DE); Sahm, Hermann, 52428 Jülich (DE); Gerth, Caroline, 33813 Oerlinghausen (DE)

(56) Entgegenhaltungen:
- EP-A2- 1 174 508
- WO-A-02/061093
- WO-A-2005/042750
- WO-A2-02/02777
- DE-A1- 10 344 739
- KALINOWSKI J ET AL: "THE COMPLETE CORYNEBACTERIUM GLUTAMICUM ATCC 13032 GENONE SEQUENCE AND ITS IMPACT ON THE PRODUCTION OF L-ASPARTATE-DERIVED AMINO ACIDS AND VITAMINS", JOURNAL OF BIOTECHNOLOGY, ELSEVIER SCIENCE PUBLISHERS, AMSTERDAM, NL, Bd. 104, Nr. 1-03, 4. September 2003 (2003-09-04), Seiten 5-25, XP001184752, ISSN: 0168-1656, DOI: DOI:10.1016/S0168-1656(03)00154-8
- EIKMANNS B J: "IDENTIFICATION, SEQUENCE ANALYSIS, AND EXPRESSION OF A CORYNEBACTERIUM GLUTAMICUM GENE CLUSTER ENCODING THE THREE GLYCOLYTIC ENZYMES GLYCERALDEHYDE-3-PHOSPHATE DEHYDROGENASE, 3-PHOSPHOGLYCERATE KINASE, AND TRIOSEPHOSPHATE ISOMERASE", JOURNAL OF BACTERIOLOGY, AMERICAN SOCIETY FOR MICROBIOLOGY, WASHINGTON, DC; US, Bd. 174, Nr. 19, 1. Oktober 1992 (1992-10-01), Seiten 6076-6086, XP000979491, ISSN: 0021-9193

## Beschreibung

Gegenstand der Erfindung ist sind rekombinante coryneforme Bakterien, in denen mindestens eines oder mehrere der heterologen Gene des Glycerin-Stoffwechsels (glycerol metabolism), ausgewählt aus der Gruppe glpA, glpB, glpC, glpD, glpE, glpF, glpG, glpK, glpQ, glpT, glpX, gldA, dhaK, dhaL, dhaM, dhaR, fsa und talC exprimiert wird (werden) sowie ein Verfahren zur fermentativen Herstellung von L-Aminosäuren, insbesondere L-Lysin und L-Tryptophan, wobei das Medium Glycerin als Kohlenstoffquelle enthält, unter Verwendung dieser Bakterien. Diese Bakterien zeigen die Fähigkeit zur Nutzung von Glycerin und somit zur wirkungsvollen Bildung und Anreicherung der L-Aminosäuren.

### Stand der Technik

Chemische Verbindungen, mit denen insbesondere L-Aminosäuren, Vitamine, Nukleoside und Nukleotide und D-Aminosäuren gemeint sind, finden in der Humanmedizin, in der pharmazeutischen Industrie, in der Kosmetik, in der Lebensmittelindustrie und in der Tierernährung Anwendung.

Zahlreiche dieser Verbindungen werden durch Fermentation von Stämmen coryneformer Bakterien, insbesondere Corynebacterium glutamicum, hergestellt. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Bakterien werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder das Tryptophan-Analogon 5-Fluorotryptophan oder auxotroph für regulatorisch bedeutsame Metabolite sind und die L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht. Eine zusammenfassende Darstellung über verschiedenste Aspekte der Genetik, des Stoffwechsels und der Biotechnologie von Corynebacterium glutamicum findet sich bei Pühler ((chief ed.) Journal of Biotechnology 104 (1-3), 1-338 (2003)) und Eggeling und Bott ((editors) Handbook of Corynebacterium glutamicum, CRC Press, Taylor & Francis Group, Boca Raton (2005)).

Die gesamte Fermentationsindustrie zur Herstellung von L-Aminosäuren verwendet heute zumeist Glukose oder Saccharose als Kohlenstoffquelle, die bei der Verarbeitung von Agrarerzeugnissen erhalten werden. Da jedoch der Preis dieser Kohlenstoffquellen steigt, ist eine technologisch durchführbare Alternative für das Produzieren der L-Aminosäuren, bevorzugt L-Lysin und L-Tryptophan, die Nutzung eines preiswerteren Materials als alternativer Rohstoff zur Fermentation, wünschenswert.

Glycerin (Propantriol) ist ein natürlicher Bestandteil von Ölen und Fetten und verbindet als "Brücke" die Fettsäuremoleküle in den Triglyceriden. Das Glycerin-Molekül ist stark polar und daher gut wasserlöslich. Als Kuppelprodukt entsteht dieser wertvolle Rohstoff bei der Biodieselherstellung (z.B. für Rapsölmethylester, RME) und wird in Kosmetika, pharmazeutischen Produkten, Lebensmitteln und für technische Anwendungen eingesetzt. Entscheidend für den Einsatz von Glycerin als Rohstoff zur Herstellung von Futtermittelkomponenten ist die Preiswürdigkeit. Es ist davon auszugehen, dass mit zunehmender Biodieselproduktion Glycerin für die Herstellung von Futtermitteladditiven interessanter wird.

Corynebacterium glutamicum Wildtyp verwendet eine Vielzahl an monomeren und oligomeren Zuckern wie Glukose, Saccharose oder Maltose als Kohlenstoffquelle (Vahjen et al., FEMS Microbiology Ecology 18: 317-328 (1995)), wächst aber nicht auf Glycerin als alleiniger Kohlenstoffquelle.

Corynebacterium glutamicum Wildtyp verfügt über einige Gene mit Homologie zu bekannten Genen des Glycerin-Stoffwechsels, bisher konnte aber nicht geklärt werden, warum das Wachstum auf Glycerin trotzdem nicht möglich ist.

### Aufgabe der Erfindung

Die Erfinder haben sich die Aufgabe gestellt, neue coryneformen Bakterien bereitzustellen, die in der Lage sind Glycerin möglichst als alleinige Kohlenstoffquelle zu nutzen. Eine weitere damit in unmittelbarem Zusammenhang stehende Aufgabe war es, ein verbessertes Verfahren zur fermentativen Herstellung von L-Aminosäuren, insbesondere L-Lysin und L-Tryptophan, mit Hilfe solcher coryneformen Bakterien bereitzustellen. Insbesondere sollte dadurch Glycerin für die fermentative Herstellung von L-Aminosäuren in möglichst wirtschaftlicher Weise nutzbar gemacht werden.

### Beschreibung der Erfindung

Gelöst werden diese sowie weitere nicht explizit genannte Aufgaben, die jedoch aus den hierin diskutierten Zusammenhängen ohne weiteres ableitbar oder erschließbar sind durch ein Verfahren nach Anspruch 1. Zweckmäßige Abwandlungen und Ausformungen der Erfindung werden in den auf Anspruch 1 rückbezogenen Unteransprüchen unter Schutz gestellt.

Die Beschreibung offenbart rekombinante coryneforme Bakterien, die insbesondere bereits L-Aminosäuren ausscheiden, und in denen mindestens eine oder mehrere der für die heterologen Genprodukte des Glycerin-Stoffwechsels (glycerol metabolism) kodierende(n) Nukleotidsequenz(en), ausgewählt aus der Gruppe glpA, glpB, glpC, glpD, glpE, glpF, glpG, glpK, glpQ, glpT, glpX, gldA, dhaK, dhaL, dhaM, dhaR, fsa und talC, exprimiert wird (werden). Diese Bakterien zeigen die Fähigkeit zur Nutzung von Glycerin.

Zu den verwendeten Bakterien gehören insbesondere coryneforme Bakterien, in denen mindestens ein heterologes Polynukleotid exprimiert wird, das für ein Polypeptid kodiert, dessen Aminosäuresequenz zu mindestens 80% oder zu mindestens 90%, insbesondere zu mindestens 95%, bevorzugt zu mindestens 98%, besonders bevorzugt zu mindestens 99% und ganz besonders bevorzugt zu 100% identisch ist mit einer Aminosäuresequenz, ausgewählt aus der Gruppe bestehend aus SEQ ID No. 2, SEQ ID No. 4, SEQ ID No. 6, SEQ ID No. 8, SEQ ID No. 10, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 32, SEQ ID No. 34 und SEQ ID No. 36 .

Die genannten Bakterien enthalten bevorzugt mindestens ein hetereologes Polynukleotid, ausgewählt aus der Gruppe, bestehend aus:
a) Polynukleotid mit der Nukleotidsequenz SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 und dazu komplementäre Nukleotidsequenzen
b) Polynukleotid mit einer Nukleotidsequenz, die der SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 im Rahmen der Degeneration des genetischen Codes entspricht;
c) Polynukleotidsequenz mit einer Sequenz, die mit der zur Sequenz SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35, die funktionsneutrale Sinnmutationen enthält,
wobei die Polynukleotide für Enzyme des Glycerin-Stoffwechsels (glycerol metabolism) kodieren.

Die Beschreibung offenbart ebenso ein Verfahren zur fermentativen Herstellung von L-Aminosäuren, insbesondere L-Lysin und L-Tryptophan, wobei das Medium Glycerin als Kohlenstoffquelle enthält, unter Verwendung von rekombinanten coryneformen Bakterien, die insbesondere bereits L-Aminosäuren produzieren, und in denen mindestens eines oder mehrere der heterologen Gene des Glycerin-Stoffwechsels (glycerol metabolism), ausgewählt aus der Gruppe glpA, glpB, glpC, glpD, glpE, glpF, glpG, glpK, glpQ, glpT, glpX, gldA, dhaK, dhaL, dhaM, dhaR, fsa und talC oder für deren Genprodukte kodierende Nukleotidsequenzen exprimiert wird bzw. werden.

Bevorzugt werden die in der Beschreibung offenbarten Bakterien eingesetzt.

Werden im folgenden L-Aminosäuren oder Aminosäuren erwähnt, sind damit eine oder mehrere der proteinogenen Aminosäuren einschließlich ihrer Salze, ausgewählt aus der Gruppe L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin und L-Prolin gemeint. Besonders bevorzugt ist L-Lysin und L-Tryptophan. Zu den L-Aminosäuren zählt auch das L-Homoserin.

Unter proteinogenen Aminosäuren versteht man die Aminosäuren die in natürlichen Proteinen, das heißt in Proteinen von Mikroorganismen, Pflanzen, Tieren und Menschen vorkommen.

Werden im Folgenden Aminosäuren erwähnt, umfasst der Begriff auch deren Salze wie beispielsweise das Lysin-Monohydrochlorid oder Lysin-Sulfat im Falle der Aminosäure L-Lysin.

Die "heterologen Gene" oder "heterologen Nukleotidsequenzen" können aus jedem prokaryontischen Spenderorganismus außer aus Vertretern der Gattung Corynebacterium stammen. Bevorzugt werden die Gene aus Escherichia coli eingesetzt.

Der Begriff "Expression heterologer Gene" beschreibt in diesem Zusammenhang die Klonierung entsprechender Gene und ihre Expression im heterologen System, was zu einer Etablierung der intrazellulären Aktivität oder Konzentration eines oder mehrerer Enzyme oder Proteine in einem Mikroorganismus führt, die durch die entsprechende DNA kodiert werden, indem man beispielsweise einen Vektor erzeugt, der das gewünschte Gen oder ein Allel dieses Gens und einen die Exprimierung des Gens ermöglichenden Promotor enthält und durch Transformation, Transduktion oder Konjugation in den Mikroorganismus überträgt und gegebenenfalls diese Maßnahmen kombiniert.

Unter Allelen versteht man alternative Formen eines gegebenen Gens. Die Formen zeichnen sich durch Unterschiede in der Nukleotidsequenz aus.

Als Genprodukt bezeichnet man das von einer Nukleotidsequenz, d.h. einem Gen oder einem Allel kodierte Protein oder die kodierte Ribonukleinsäure.

Gegenstand dieser Erfindung ist ein Verfahren zur fermentativen Herstellung von L-Aminosäuren, dadurch gekennzeichnet, dass man folgende Schritte durchführt:
a) Kultivierung der die gewünschte L-Aminosäure produzierenden rekombinanten coryneformen Bakterien, in denen mindestens das heterologe Polynukleotid glpK exprimiert oder das endogene Polynukleotid glpk überexprimiert vorliegt, in einem Glycerin oder gegebenenfalls zusätzlich eine oder mehrere weitere C-Quellen enthaltenden Medium unter Bedingungen, bei denen die gewünschte L-Aminosäure im Medium oder in den Zellen angereichert wird, und gegebenenfalls
b) Isolierung der gewünschten L-Aminosäure, wobei gegebenenfalls Bestandteile der Fermentationsbrühe und/oder der Biomasse in ihrer Gesamtheit oder in Anteilen (> 0 bis 100%) im Endprodukt verbleiben.
wobei das Polypeptid mit der Aktivität einer Glycerinkinase, kodiert vom heterologen Polynukleotid glpK, eine Aminosäuresequenz aufweist, die zu mindestens 80% identisch ist mit der Aminosäuresequenz von SEQ ID NO:16
und wobei das Polypeptid mit der Aktivität einer Glycerinkinase, kodiert vom endogenen Polynukleotid glpK, die Aminosäuresequenz von SEQ ID NO:38 aufweist.

Die eingesetzten coryneformen Bakterien produzieren bevorzugt bereits vor der Expression eines oder mehrerer der Gene des Glycerin-Stoffwechsels auf den herkömmlichen Kohlenstoffquellen wie zum Beispiel Glukose oder Saccharose L-Aminosäuren, insbesondere L-Lysin und L-Tryptophan. Das eingesetzte Glycerin kann einzeln oder als Mischung verwendet werden, wobei der Anteil des Glycerins bevorzugt ≥ 10 bis 100% betragen soll.

Es wurde gefunden, dass coryneforme Bakterien nach heterologer Expression eines oder mehrerer der Gene des Glycerin-Stoffwechsels L-Aminosäuren, insbesondere L-Lysin und L-Tryptophan, aus Glycerin als alleiniger Kohlenstoffquelle produzieren.

Erfindungsgemäße rekombinante Bakterien werden beispielsweise durch Transformation, Transduktion oder Konjugation, oder einer Kombination dieser Methoden, mit einem Vektor erzeugt, der das gewünschte Gen, ein Allel dieses Gens oder Teile davon und einen die Exprimierung des Gens ermöglichenden Promotor enthält. Die heterologe Expression wird insbesondere durch Integration des Gens oder der Allele in das Chromosom der Mikroorganismen oder einen extrachromosomal replizierenden Vektor erzielt.

Bei dem Promotor kann es sich um die eigene, upstream des Gens befindliche regulatorische Sequenz handeln, oder es wird ein Promotor aus coryneformen Bakterien mit dem Gen fusioniert. Eine Übersicht über bekannte Promotoren aus Corynebacterium glutamicum ist von Pátek et al. (Journal of Biotechnology 104, 311-323 (2003)) beschrieben.

Die Bakterien, an denen die Maßnahmen der Erfindung durchgeführt werden und die somit Ausgangspunkt der vorliegenden Erfindung sind, können Aminosäuren aus Glucose, Saccharose, Lactose, Fructose, Maltose, Melasse, Stärke, Cellulose oder aus Ethanol herstellen. Es handelt sich um Vertreter coryneformer Bakterien.

Bei den coryneformen Bakterien wird die Gattung Corynebacterium bevorzugt. Besonders bevorzugt sind Aminosäure-ausscheidende Stämme, die auf folgenden Arten beruhen:
Corynebacterium efficiens, wie zum Beispiel der Stamm DSM44549,
Corynebacterium glutamicum, wie zum Beispiel der Stamm ATCC13032,
Corynebacterium thermoaminogenes wie zum Beispiel der Stamm FERM BP-1539, und
Corynebacterium ammoniagenes, wie zum Beispiel der Stamm ATCC6871,
wobei die Art Corynbacterium glutamicum ganz besonders bevorzugt wird.

Einige Vertreter der Art Corynebacterium glutamicum sind im Stand der Technik auch unter anderen Artbezeichnungen bekannt. Hierzu gehören beispielsweise:
Corynebacterium acetoacidophilum ATCC13870
Corynebacterium lilium DSM20137
Corynebacterium melassecola ATCC17965
Brevibacterium flavum ATCC14067
Brevibacterium lactofermentum ATCC13869 und
Brevibacterium divaricatum ATCC14020

Bekannte Vertreter Aminosäure ausscheidender Stämme coryneformer Bakterien sind beispielsweise die L-Lysin produzierenden Stämme
Corynebacterium glutamicum DM58-1/pDM6 (= DSM4697) beschrieben in EP 0 358 940
Corynebacterium glutamicum MH20 (= DSM5719) beschrieben in EP 0 435 132
Corynebacterium glutamicum AHP-3 (= FermBP-7382) beschrieben in EP 1 108 790
Corynebacterium thermoaminogenes AJ12521 (= FERM BP-3304) beschrieben in US 5,250,423
oder die L-Tryptophan produzierenden Stämme
Corynebacterium glutamicum K76 (=FermBP-1847) beschrieben in US 5,563,052
Corynebacterium glutamicum BPS13 (=FermBP-1777) beschrieben in US 5,605,818
Corynebacterium glutamicum FermBP-3055 beschrieben in US 5,235,940

Angaben zur taxonomischen Einordnung von Stämmen dieser Gruppe von Bakterien findet man unter anderem bei Seiler (Journal of General Microbiology 129, 1433-1477 (1983)), Kämpfer und Kroppenstedt (Canadian Journal of Microbiology 42, 989-1005 (1996)), Liebl et al (International Journal of Systematic Bacteriology 41, 255-260 (1991)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Der genannte Stamm von Corynebacterium thermoaminogenes (FERM BP-1539) ist in der US-A-5.250.434 beschrieben.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden.

Aus den ebenfalls zur Familie Enterobacteriaceae gehörenden Salmonella typhimurium (Accession No.: NC 003197 (Sequenz des gesamten Genoms)) und Shigella flexneri (Accession No.: NC 004337 (Sequenz des gesamten Genoms)) ist die Nukleotidsequenz für die Gene glpA, glpB, glpC, glpD, glpE, glpF, glpG, glpK, glpQ, glpT und glpX beispielhaft ebenso bekannt. Desweiteren ist aus Salmonella typhimurium (Accession No.: NC 003197 (Sequenz des gesamten Genoms)) die Nukleotidsequenz für die Gene gldA und talC und aus Shigella flexneri (Accession No.: NC 004337 (Sequenz des gesamten Genoms)) die Nukleotidsequenz für die Gene dhaK, dhaL, dhaM, dhaR und fsa bekannt.

Die Gene und Aktivitäten des Glycerin-Stoffwechsels (glycerol metabolism) sind zusammenfassend auch bei Lin (In: Neidhardt (ed), Escherichia coli und Salmonella, American Society for Microbiology, Washington, D.C., USA: 307-342 (1996)) beschrieben.

Das die Gene des Glycerintransport und -metabolismus enthaltende Glycerophosphatregulon (glp) besteht aus fünf Operons, die an drei verschiedenen Genorten auf dem E. coli-Chromosom liegen (Cozzarelli et al., Journal of Molecular Biology 31: 371-387 (1968)).

Ein Regulon ist eine Einheit von Genen, die zwar an verschiedenen Orten eines Genoms lokalisiert sind, deren Expression aber durch die gleichen Regulatorproteine gesteuert wird. Ein Operon ist eine Einheit von gemeinsam regulierten Genen an einem Genort.

Bei 88 min findet man das Operon glpFKX (Cozzarelli und Lin, Journal of Bacteriology 91: 1763-1766 (1966); Weissenborn et al., Journal of Biological Chemistry 267: 6122-6131 (1992)), das für den Glycerinfascilitator GlpF und die Glycerinkinase GlpK und eine Fructose-1,6-Bisphosphatase II GlpX (Donahue et al., Journal of Bacteriology 182(19): 5624-5627 (2000)) kodiert. In dem bei 49 min kartierten Operon sind die Gene glpT (Glycerin-3-Phosphat-Permease) und glpQ (periplasmatische Glycerinphosphodiesterase) zusammengefasst, die im Gegenuhrzeigersinn transkribiert werden. Gegenläufig dazu ist das glpABC-Operon angeordnet, dessen Gene für die drei Untereinheiten der heterotrimeren sn-Glycerin-3-Phosphat-Dehydrogenase, wirksam unter Ausschluß von Luftsauerstoff (anaerob), kodieren (Cole et al., Journal of Bacteriology 170: 2448-2456 (1988); Ehrmann et al., Journal of Bacteriology 169: 526-532 (1987)). Bei 75 min auf dem Chromosom liegt das glpDEG-Operon, das für die Glycerin-3-Phosphat-Dehydrogenase GlpD, wirksam in Gegenwart von Luftsauerstoff (aerob) (Cozzarelli et al., Journal of Molecular Biology 31: 371-387 (1968)), die Schwefeltransferase GlpE (Cozzarelli et al., Journal of Molecular Biology 31: 371-387 (1968)) und das glpG-Gen mit unbekannter Funktion (Zeng et al., Journal of Bacteriology 178: 7080-7089 (1996)) kodiert.

Eine kurze Zusammenfassung der Gene und Aktivitäten des Glycerin-Stoffwechsels gibt die folgende Auflistung:
- glpA-Gen: Bezeichnung:: große Untereinheit der sn-Glycerin-3-Phosphat-Dehydrogenase (anaerob)
- Funktion:: Im anaeroben Milieu wird Glycerin-3-Phosphat zur Energiegewinnung von einer FAD-abhängigen Glycerin-3-Phosphat-Dehydrogenase (GlpABC) zu Dihydroxyaceton-Phosphat oxidiert, welches als Intermediat in die Glykolyse eingehen kann. Die bei dieser Oxidationsreaktion frei werdenden Reduktionsäquivalente werden von dem Flavoenzym auf einen membranassoziierten Cytochromkomplex übertragen, wobei Fumarat oder Nitrat als terminaler Elektronenakzeptor dient (Lin, in: Neidhardt (ed), Escherichia coli und Salmonella, American Society for Microbiology, Washington, D.C., USA: 307-342 (1996)). Während die Elektronen durch den Cytochromkomplex geschleust werden, wird die frei werdende Energie dazu genutzt, Protonen durch die Membran von der cytoplasmatischen auf die periplasmatische Seite zu pumpen. Mit dem an der Membran erzeugten Protonengradienten ändert sich sowohl das elektrische als auch das chemische Potential, womit die membrangebundene ATPase angetrieben wird und dadurch ATP erzeugen kann.
- EC-Nr.:: 1.1.99.5
- Referenz:: Blattner et al.; Science 277(5331): 1453-1474 (1997)
- SEQ ID No.:: 1
- Accession No.:: U00096 (Region: 2350669-2352297)
- Alternativer Genname:: b2241
- glpB-Gen: Bezeichnung:: Untereinheit zur Membranverankerung (membrane anchor) der sn-Glycerin-3-Phosphat-Dehydrogenase (anaerob)
- Funktion:: siehe glpA
- EC-Nr.:: 1.1.99.5
- Referenz:: Blattner et al.; Science 277(5331): 1453-1474 (1997)
- SEQ ID No.:: 3
- Accession No.:: U00096 (Region: 2352287-2353546)
- Alternativer Genname:: b2242
- glpC-Gen: Bezeichnung:: kleine Untereinheit der sn-Glycerin-3-Phosphat-Dehydrogenase (anaerob)
- Funktion:: siehe glpA
- EC-Nr.:: 1.1.99.5
- Referenz:: Blattner et al.; Science 277(5331): 1453-1474 (1997)
- SEQ ID No.:: 5
- Accession No.:: U00096 (Region: 2353543-2354733)
- Alternativer Genname:: b2243
- glpD-Gen: Bezeichnung:: Glycerin-3-Phosphat-Dehydrogenase (aerob)
- Funktion:: GlpD wurde als aerobe Glycerin-3-Phosphat-Dehydrogenase (Cozzarelli et al., Journal of Molecular Biology 31: 371-387 (1968)) identifiziert. Im aeroben Milieu wird Glycerin-3-Phosphat zur Energiegewinnung von dieser FAD-abhängigen Glycerin-3-Phosphat-Dehydrogenase (GlpD) zu Dihydroxyaceton-Phosphat oxidiert, welches als Intermediat in die Glykolyse eingehen kann. Die bei dieser Oxidationsreaktion frei werdenden Reduktionsäquivalente werden von dem Flavoenzym auf einen membranassoziierten Cytochromkomplex übertragen, wobei molekularer Sauerstoff bzw. Nitrat als terminaler Elektronenakzeptor dient (Lin, in: Neidhardt (ed), Escherichia coli und Salmonella, American Society for Microbiology, Washington, D.C., USA: 307-342 (1996)). Während die Elektronen durch den Cytochromkomplex geschleust werden, wird die frei werdende Energie dazu genutzt, Protonen durch die Membran von der cytoplasmatischen auf die periplasmatische Seite zu pumpen. Mit dem an der Membran erzeugten Protonengradienten ändert sich sowohl das elektrische als auch das chemische Potential, womit die membrangebundene ATPase angetrieben wird und dadurch ATP erzeugen kann. Das offene Leseraster des glpD-Gens besteht aus 501 Codons und die translatierte Sequenz kodiert ein Protein mit einem Molekulargewicht von 57 kDa (Austin und Larson, Journal of Bacteriology 173: 101-107 (1991)).
- EC-Nr.:: 1.1.99.5
- Referenz:: Blattner et al.; Science 277(5331): 1453-1474 (1997)
- SEQ ID No.:: 7
- Accession No.:: U00096 (Region: 3560036-3561541)
- Alternativer Genname:: b3426, glyD
- glpE-Gen: Bezeichnung:: Schwefeltransferase; saure, cytoplasmatische Rhodanese
- Funktion:: GlpE wurde als Schwefeltransferase (Cozzarelli et al., Journal of Molecular Biology 31: 371-387 (1968)) identifiziert. Die vom glpE-Gen kodierte saure, cytoplasmatische Rhodanese mit einem Molekulargewicht von 12 kDa katalysiert als Dimer die Übertragung von Schwefel auf den Schwefelakzeptor Thioredoxin 1 (Ray et al., Journal of Bacteriology 182: 2277-2284 (2000)).
- EC-Nr.:: 2.8.1.1
- Referenz:: Blattner et al.; Science 277(5331): 1453-1474 (1997)
- SEQ ID No.:: 9
- Accession No.:: U00096 (Region: 3559520-3559846)
- Alternativer Genname:: b3425
- glpF-Gen: Bezeichnung:: Glycerinfascilitator GlpF
- Funktion:: Die erleichterte Diffusion von Glycerin aus dem Nährmedium wird von dem Glycerinfascilitator GlpF katalysiert (Borgnia und Agre, Proc. Natl. Acad. Sci. U.S.A. 98: 2888-2893 (2001)), der einen substratspezifischen Kanal mit einer Porengröße von 0.4 nm bildet (Heller et al., Journal of Bacteriology 144: 274-278 (1980)).
- EC-Nr.:: -
- Referenz:: Blattner et al.; Science 277(5331): 1453-1474 (1997)
- SEQ ID No.:: 11
- Accession No.:: U00096 (Region: 4115268-4116113)
- Alternativer Genname:: b3927
- glpG-Gen: Bezeichnung:: Gen des glp Regulons
- Funktion:: Die physiologische Funktion von glpG ist noch unbekannt. Das glpG-Genprodukt ist ein basisches, cytoplasmatisches oder membranassoziiertes Protein mit einem Molekulargewicht von 28 kDa (Zeng et al., Journal of Bacteriology 178: 7080-7089 (1996)).
- EC-Nr.:: -
- Referenz:: Blattner et al.; Science 277(5331): 1453-1474 (1997)
- SEQ ID No.:: 13
- Accession No.:: U00096 (Region: 3558645-3559475)
- Alternativer Genname:: b3424
- glpK-Gen: Bezeichnung:: Glycerinkinase GlpK (ATP-abhängig)
- Funktion:: Cytoplasmatisches Glycerin wird sofort von der ATP-abhängigen Glycerinkinase K phosphoryliert, die in ihrer enzymatisch aktiven Form mit dem Glycerinfascilitator GlpF assoziiert vorliegt (Voegele et al., Journal of Bacteriology 175: 1087-1094 (1993)).
- EC-Nr.:: 2.7.1.30
- Referenz:: Blattner et al.; Science 277(5331): 1453-1474 (1997)
- SEQ ID No.:: 15
- Accession No.:: U00096 (Region: 4113737-4115245)
- Alternativer Genname:: b3926
- glpQ-Gen: Bezeichnung:: Glycerinphosphodiesterase
- Funktion:: Glycerophosphatdiester, die deacetylierten Abbauprodukte der Phospholipide (Lin, in: Neidhardt (ed), Escherichia coli und Salmonella, American Society for Microbiology, Washington, D.C., USA: 307-342 (1996)), werden im Periplasma von der dort lokalisierten Phosphodiesterase GlpQ zu Alkohol und Glycerin-3-Phosphat hydrolysiert (Larson et al., Journal of Biological Chemistry 258: 5428-5432 (1983)). Da der Glycerinphosphodiesterase somit eine extracytoplasmatische Wirkweise zugeschrieben wird, muss das von dem heterologen glpQ-Gen abgeleitete Genprodukt in den beanspruchten Bakterien ein Leaderpeptid umfassen, wie es für exkretierte Proteine Grampositiver Bakterien typisch ist (Nielsen et al., Protein Engineering Design and Selection 10:1-6 (1997)). Diese charakteristische Signalsequenz ermöglicht einen durch den allgemeinen Sekretionsapparat (Sec-System) vermittelten Export des GlpQ-Proteins über die Cytoplasmamembran (Kell und Young, Current opinion in microbiology 3:238-243 (2000); Mukamolova et al., Molecular Microbiology 46:611-621 (2002a); Molecular Microbiology 46:613-635 (2002b)).
- EC-Nr.:: 3.1.4.46
- Referenz:: Blattner et al.; Science 277(5331): 1453-1474 (1997)
- SEQ ID No.:: 17
- Accession No.:: U00096 (Region: 2347957-2349033)
- Alternativer Genname:: b2239
- glpT-Gen: Bezeichnung:: Glycerin-3-Phosphat-Permease
- Funktion:: Glycerin-3-Phosphat wird durch die Permease GlpT (Eiglmeier et al., Molecular Microbiology 1: 251-258 (1987); Larson et al., Journal of Bacteriology 152: 1008-1021 (1982)) im Austausch gegen anorganisches Phosphat ins Zellinnere transportiert (Auer et al., Biochemistry 40: 6628-6635 (2001)). Die für den Transport benötigte Energie wird durch diesen Antiport bereitgestellt und gleichzeitig wird eine Akkumulation des toxisch wirkenden Phosphats verhindert (Xavier et al., Journal of Bacteriology 177: 699-704. (1995)).
- EC-Nr.:: -
- Referenz:: Blattner et al.; Science 277(5331): 1453-1474 (1997)
- SEQ ID No.:: 19
- Accession No.:: U00096 (Region: 2349038-2350396)
- Alternativer Genname:: b2240
- glpX-Gen: Bezeichnung:: Fructose-1,6-Bisphosphatase II
- Funktion:: Bisher konnte keine physiologische Rolle des Enzyms GlpX gefunden werden. Eine Mutation in fbp, dem Gen für die Fructose-1,6-Bisphosphatase I, konnte durch GlpX nicht komplementiert werden. Doch scheint eine funktionelle Bedeutung trotzdem sicher, da Mutanten, die in der Glykolyse aufgrund einer pfkA (Phosphofructokinase A) Mutation gestört sind, ohne GlpX sehr viel langsamer wachsen (Donahue et al.; Journal of Bacteriology 182 (19) : 5624 - 5627 (2000)).
- EC-Nr.:: 3.1.3.11
- Referenz:: Blattner et al.; Science 277(5331): 1453-1474 (1997)
- SEQ ID No.:: 21
- Accession No.:: U00096 (Region: 4112592-4113602)
- Alternativer Genname:: 3925

Neben den Genen des Glycerin-Transports und -Stoffwechsels (glycerol metabolism) können die folgenden Gene des Abbaus verschiedener Zwischen- und Endprodukte des besagten Stoffwechsels heterolog exprimiert werden:
- gldA-Gen: Bezeichnung:: Glycerin-Dehydrogenase (NAD)
- Funktion:: Die Glycerin-Dehydrogenase katalysiert die reversible NAD-abhängige Reaktion von Glycerin zu Dihydroxyaceton (Truniger und Boos, Journal of Bacteriology 176(6): 1796-1800 (1994))
- EC-Nr.:: 1.1.1.6
- Referenz:: Blattner et al.; Science 277(5331): 1453-1474 (1997)
- SEQ ID No.:: 23
- Accession No.:: U00096 (Region: 4135955-4137097)
- Alternativer Genname:: b3945
- dhaK-Gen: Bezeichnung:: Untereinheit der Dihydroxyaceton-Kinase, N-terminale Domäne
- Funktion:: Die Dihydroxyaceton-Kinase katalysiert in ihrer Funktion als Phosphoenolpyruvat (PEP)-abhängige Dihydroxyaceton-Phosphotransferase die Reaktion von Dihydroxyaceton zu Dihydroxyaceton-Phosphat (Gutknecht et al., The EMBO Journal 20(10): 2480-2486 (2001)). DhaK trägt die Dihydroxyaceton-Bindestelle.
- EC-Nr.:: 2.7.1.29
- Referenz:: Blattner et al.; Science 277(5331): 1453-1474 (1997)
- SEQ ID No.:: 25
- Accession No.:: U00096 (Region: 1248991-1250091)
- Alternativer Genname:: dhak1, ycgT, b1200
- dhaL-Gen: Bezeichnung:: Untereinheit der Dihydroxyaceton-Kinase, C-terminale Domäne
- Funktion:: Die Dihydroxyaceton-Kinase katalysiert in ihrer Funktion als PEP-abhängige Dihydroxyaceton-Phosphotransferase die Reaktion von Dihydroxyaceton zu Dihydroxyaceton-Phosphat (Gutknecht et al., The EMBO Journal 20(10): 2480-2486 (2001)). DhaL trägt ADP als Cofaktor für den Transfer von Phosphat von DhaM auf Dihydroxyaceton.
- EC-Nr.:: 2.7.1.29
- Referenz:: Blattner et al.; Science 277(5331): 1453-1474 (1997)
- SEQ ID No.:: 27
- Accession No.:: U00096 (Region: 1248348-1248980)
- Alternativer Genname:: dhaK2, ycgS, b1199
- dhaM-Gen: Bezeichnung:: PTS-Protein-Untereinheit der Dihydroxyaceton-Kinase, Multi-Phosphoryl-Transferprotein
- Funktion:: Die Dihydroxyaceton-Kinase katalysiert in ihrer Funktion als PEP-abhängige Dihydroxyaceton-Phosphotransferase die Reaktion von Dihydroxyaceton zu Dihydroxyaceton-Phosphat (Gutknecht et al., The EMBO Journal 20(10): 2480-2486 (2001)). DhaM besteht aus drei Domänen mit Ähnlichkeit zu den drei Domänen des Phosphoenolpyruvat-abhängigen Phosphotransferase-Systems. Phosphoryliertes DhaM überträgt das Phosphat auf das am DhaL gebundene ADP.
- EC-Nr.:: 2.7.1.29
- Referenz:: Blattner et al.; Science 277(5331): 1453-1474 (1997)
- SEQ ID No.:: 29
- Accession No.:: U00096 (Region: 1246919-1248340)
- Alternativer Genname:: dhaH, ycgC, b1198
- dhaR-Gen: Bezeichnung:: Aktivator des dha-Operons (dhaKLM)
- Funktion:: transkriptioneller Aktivator, stimuliert die Transkription des dha-Operons von einem sigma70-Promotor (Bachler et al., The EMBO Journal 24 (2): 283-293 (2005))
- EC-Nr.:: -
- Referenz:: Blattner et al.; Science 277(5331): 1453-1474 (1997)
- SEQ ID No.:: 31
- Accession No.:: U00096 (Region: 1250280-1252208)
- Alternativer Genname:: ycgU, b1201
- fsa-Gen: Bezeichnung:: Fruktose-6-Phosphat-Aldolase I
- Funktion:: Die Fruktose-6-Phosphat-Aldolase I katalysiert eine Aldolspaltung des Fruktose-6-Phosphats, Substrate des Enzyms sind Dihydroxyaceton sowie Fruktose-6-Phosphat und Glycerinaldehyd-3-Phosphat, nicht genutzt werden Fruktose, Fruktose-1-Phosphat, Fruktose-1,6-bisphosphat oder Dihydroxyaceton-Phosphat (Schurmann und Sprenger, Journal of Biological Chemistry 276(14): 11055-11061 (2001))
- EC-Nr.:: 4.1.2.-
- Referenz:: Blattner et al.; Science 277(5331): 1453-1474 (1997)
- SEQ ID No.:: 33
- Accession No.:: U00096 (Region: 862793-863527)
- Alternativer Genname:: mipB, ybiZ, B0825
- talC-Gen: Bezeichnung:: Fruktose-6-Phosphat-Aldolase II
- Funktion:: Die Fruktose-6-Phosphat-Aldolase II katalysiert eine Aldolspaltung des Fruktose-6-Phosphats (Schurmann und Sprenger, Journal of Biological Chemistry 276(14): 11055-11061 (2001)). Das talC-Gen liegt direkt neben dem gldA-Gen.
- EC-Nr.:: 4.1.2.-
- Referenz:: Blattner et al.; Science 277(5331): 1453-1474 (1997)
- SEQ ID No.:: 35
- Accession No.:: U00096 (Region: 4137069-4137731)
- Alternativer Genname:: fsaB, yijG, b3946

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannten Sequenzen zu den behandelten Genen von Escherichia coli unter den SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 und SEQ ID No. 35 dargestellt.

Die in den angegebenen Textstellen beschriebenen offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben.

Zu den Allelen der behandelten Gene, welche funktionsneutrale Sinnmutationen enthalten, zählen unter anderem solche, die zu höchstens 13 oder zu höchstens 10, bevorzugt zu höchstens 7 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 13, 10, 7, 5, 3 oder 2 Aminosäuren oder Aminosäurereste.

Es ist bekannt, dass durch wirtseigene Enzyme, sogenannte Aminopeptidasen, das endständige Methionin bei der Proteinsynthese entfernt wird.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 4, 5 in keinem Falle aber mehr als 6 Aminosäuren betreffen.

Zu den geeigneten Allelen gehören ferner solche die durch Hybridisierung, insbesondere unter stringenten Bedingungen unter Verwendung von SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 59°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Auch Polynukleotide von Bacillus subtilis (Accession No.: NC 000964 (Sequenz des gesamten Genoms))und Streptomyces coelicolor (Accession No.: NC 003888 (Sequenz des gesamten Genoms)), die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zu den in SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 dargestellten Nukleotidsequenzen besitzen und die beschriebenen Funktionen aufweisen, können eingesetzt werden. Die aus den Genomprojekten bekannten Nukleotidsequenzen der Gene und ORF's gehören zum Stand der Technik und können verschiedenen Veröffentlichungen, Patentanmeldungen sowie der Datenbank des National Center for Biotechnology Information (NCBI) der National Library of Medicin (Bethesda, MD, USA) entnommen werden.

Die Expression der Gene des Glycerinstoffwechsels ist mit Hilfe von 1- und 2-dimensionaler Proteingelauftrennung und anschließender optischer Identifizierung der Proteinkonzentration mit entsprechender Auswertesoftware im Gel nachweisbar. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließende optische Auswertung mit entsprechender Software zur Konzentrationsbestimmung (Lohaus und Meyer (1998) Biospektrum 5:32-39; Lottspeich (1999) Angewandte Chemie 111:2630-2647) analysiert werden. Die Aktivität von DNA-bindenden Proteinen kann mittels DNA Band-Shift-Assays (auch als Gelretardation bezeichnet) gemessen werden (Wilson et al. (2001) Journal of Bacteriology 183:2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular cloning: a laboratory manual. 2nd Ed. Cold Spring Harbor Laboratory Press, Cold Spring Harbor, N.Y., 1989). Die intrazellulären enzymatischen Aktivitäten können nach verschiedenen beschriebenen Methoden (Donahue et al. (2000) Journal of Bacteriology 182(19):5624-5627; Ray et al. (2000) Journal of Bacteriology 182(8):2277-2284; Freedberg et al. (1973) Journal of Bacteriology 115(3):816-823) bestimmt werden.

Die heterologen Gene werden zum Beispiel mit Hilfe von episomalen Plasmiden exprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden. Ein Überblick über native Plasmide aus Aminosäure-produzierenden coryneformen Bakterien ist im Handbook of Corynebacterium glutamicum (Tauch, Chapter 4, 57-80, editors: Eggeling und Bott, CRC Press, Taylor & Francis Group, Boca Raton (2005)) verfügbar.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons oder in der WO03/040373 beschrieben wurde. Bei dieser Methode wird das Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)), pEM1 (Schrumpf et al, 1991, Journal of Bacteriology 173:4510-4516) oder pBGS8 (Spratt et al.,1986, Gene 41: 337-342) in Frage. Der Plasmidvektor, der das zu amplifizierende heterologe Gen, gegebenenfalls einschließlich der Expressions- und/oder Regulationssignale, und die Randbereiche eines nicht essentiellen homologen Gens enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiology Letters 123, 343-347 (1994)) beschrieben. Nach homologer Rekombination mittels eines "cross over"-Ereignisses enthält der resultierende Stamm eine Kopie des heterologen Gens einschließlich des Plasmidvektors an dem gewünschten Genort des C. glutamicum Chromosoms, der über die homologen Nukleotidsequenzen auf dem Plasmid vorbestimmt war. Mittels eines geeigneten zweiten, eine Excision bewirkenden "cross-over"-Ereignisses im Zielgen bzw. in der Zielsequenz erreicht man den Einbau nur des heterologen Gens. Dabei verbleibt am jeweiligen natürlichen Genort keine zur episomalen Replikation in Mikroorganismen befähigende Nukleotidsequenz, keine zur Transposition befähigende Nukleotidsequenz und keine Resistenz gegen Antibiotika vermittelnde Nukleotidsequenz.

Zusätzlich kann es für die Produktion von L-Aminosäuren vorteilhaft sein, zusätzlich zur funktionellen Expression eines oder mehrerer der heterologen Gene des Glycerin-Stoffwechsels (glycerol metabolism), ausgewählt aus der Gruppe glpA, glpB, glpC, glpD, glpE, glpF, glpG, glpK, glpQ, glpT, glpX, gldA, dhaK, dhaL, dhaM, dhaR, fsa und talC, eines oder mehrere Enzyme der Stoffwechselwege, welche die Bildung der gewünschten Aminosäure erhöhen oder verringern, wie z.B. des Biosyntheseweges, der Glykolyse, der Anaplerotik, des Zitronensäure-Zyklus, des Pentosephosphat-Zyklus, des Aminosäure-Exports und gegebenenfalls regulatorische Proteine entweder zu verstärken, insbesondere überzuexprimieren, oder abzuschwächen, insbesondere die Expression zu verringern.

Der Begriff "Verstärkung" bzw. "Verstärken" beschreibt in diesem Zusammenhang die Erhöhung der intrazellulären Aktivität oder Konzentration eines oder mehrerer Enzyme bzw. Proteine in einem Mikroorganismus, die durch die entsprechende DNA kodiert werden, indem man beispielsweise die Kopienzahl des Gens bzw. der Gene erhöht, einen starken Promotor oder ein Gen bzw. Allel verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise der Aktivität oder Konzentration des Proteins im Ausgangs-Mikroorganismus erhöht.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Zur Verstärkung werden Gene zum Beispiel mit Hilfe von episomalen Plasmiden überexprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)), pEM1 (Schrumpf et al, 1991, Journal of Bacteriology 173:4510-4516) oder pBGS8 (Spratt et al.,1986, Gene 41: 337-342) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiology Letters 123, 343-347 (1994)) beschrieben. Nach homologer Rekombination mittels eines "cross over"-Ereignisses enthält der resultierende Stamm mindestens zwei Kopien des betreffenden Gens.

Eine gebräuchliche Methode, eine oder mehrere zusätzliche Kopien eines Gens von C. glutamicum in das Chromosom des gewünschten coryneformen Bakteriums einzubauen, ist die in der WO03/014330 und der WO03/040373 beschriebene Methode der Gen-Verdopplung. Dazu wird in der WO03/040373 die Nukleotidsequenz des gewünschten ORF's, Gens oder Allels, gegebenenfalls einschließlich der Expressions- und/oder Regulationssignale isoliert und zwei Kopien, bevorzugt in Tandem-Anordnung, in einen für C. glutamicum nicht replikativen Vektor wie beispielsweise pK18mobsacB oder pK19mobsacB (Jäger et al., Journal of Bacteriology 174: 5462-65 (1992))kloniert. Der Vektor wird anschließend durch Transformation oder Konjugation in das gewünschte coryneforme Bakterium überführt. Nach homologer Rekombination mittels eines ersten, Integration bewirkenden "cross-over"-Ereignisses und eines geeigneten zweiten, eine Excision bewirkenden "cross-over"-Ereignisses im Zielgen bzw. in der Zielsequenz erreicht man den Einbau der zusätzlichen Gen-Kopie. Danach werden solche Bakterien isoliert, bei denen zwei Kopien des ORF's, Gens oder Allels am jeweiligen natürlichen Ort anstelle der ursprünglich vorhandenen singulären Kopie vorliegen. Dabei verbleibt am jeweiligen natürlichen Genort keine zur episomalen Replikation in Mikroorganismen befähigende Nukleotidsequenz, keine zur Transposition befähigende Nukleotidsequenz und keine Resistenz gegen Antibiotika vermittelnde Nukleotidsequenz.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym bzw. Protein mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Die Erhöhung bzw. Erniedrigung der Proteinkonzentration ist über die bereits vorher genannten Methoden nachweisbar (Hermann et al., Electrophoresis, 22:1712-23 (2001); Lohaus und Meyer, Biospektrum 5:32-39 (1998); Lottspeich, Angewandte Chemie 111:2630-2647 (1999); Wilson et al., Journal of Bacteriology 183:2151-2155 (2001)).

Die Verwendung endogener Gene wird im Allgemeinen bevorzugt. Unter "endogenen Genen" oder "endogenen Nukleotidsequenzen" versteht man die in der Population einer Art vorhandenen Gene beziehungsweise Nukleotidsequenzen.

So kann beispielsweise für die Herstellung von L-Lysin neben der Expression eines oder mehrerer der heterologen Gene des Glycerin-Stoffwechsels (glycerol metabolism), ausgewählt aus der Gruppe glpA, glpB, glpC, glpD, glpE, glpF, glpG, glpK, glpQ, glpT, glpX, gldA, dhaK, dhaL, dhaM, dhaR, fsa und talC, eines oder mehrere der Gene ausgewählt aus der Gruppe der Gene oder Allele der Lysinproduktion verstärkt, insbesondere überexprimiert werden. Unter "Gene oder Allele der Lysinproduktion sind sämtliche, bevorzugt endogene, offene Leserahmen, Gene oder Allele zu verstehen, deren Verstärkung/Überexpression eine Verbesserung der Lysinproduktion bewirken kann.

Hierzu gehören unter anderem folgende Gene oder Allele: accBC, accDA, cstA, cysD, cysE, cysH, cysK, cysN, cysQ, dapA, dapB, dapC, dapD, dapE, dapF, ddh, dps, eno, gap, gap2, gdh, gnd,lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf und zwf A243T. Diese sind in Tabelle 1 zusammengefasst und erläutert.

**Tabelle 1**

| Gene und Allele der Lysinproduktion | | | |
|---|---|---|---|
| Name | Bezeichnung des kodierten Enzyms oder Proteins | Referenz | Zugangsnummer |
| accBC | Acyl-CoA Carboxylase | Jäger et al. Archives of Microbiology (1996) 166:76-82 | U35023 |
| | EC 6.3.4.14 | | |
| | (acyl-CoA carboxylase) | | |
| | | EP1108790; | AX123524 |
| | | WO0100805 | AX066441 |
| accDA | Acetyl-CoA Carboxylase | EP1055725 | |
| | EC 6.4.1.2 | EP1108790 | AX121013 |
| | (acetyl-CoA carboxylase) | WO0100805 | AX066443 |
| CStA | Carbon Starvation Protein A | EP1108790 | AX120811 |
| | (carbon starvation protein A) | WO0100804 | AX066109 |
| cysD | Sulfat-Adenylyltransferase Untereinheit II | EP1108790 | AX123177 |
| | EC 2.7.7.4 | | |
| | (sulfate adenylyltransferase small chain) | | |
| cysE | Serinacetyltransferase | EP1108790 | AX122902 |
| | EC 2.3.1.30 | WO0100843 | AX063961 |
| | (serine acetyltransferase) | | |
| cysH | 3'-Phosphoadenylsulfat Reduktase | EP1108790 | AX123178 |
| | EC 1.8.99.4 | WO0100892 | AX066001 |
| | (3'-phosphoadenosine 5'-phosphosulfate reductase) | | |
| cysK | Cystein-Synthase | EP1108790 | AX122901 |
| | EC 4.2.99.8 (cysteine synthase) | MO0100843 | AX063963 |
| cysN | Sulfat-Adenylyltransferase Untereinheit. I | EP1103790 | AX123176 |
| | | | AX127152 |
| | EC 2.7.7.4 | | |
| | (sulfate adenylyltransferase) | | |
| cysQ | Transportprotein CysQ | EP1108790 | AX127145 |
| | (transporter cysQ) | WO0100805 | AX066423 |
| dapA | Dihydrodipicolinat-Synthase | Bonnassie et al. Nucleic | X53993 |
| | EC 4.2.1.52 | | |
| | (dihydrodipicolinate synthase) | Acids Research 18:6421 (1990) | |
| | | Pisabarro et al., Journal of Bacteriology 175:2743-2749(1993) | Z21502 |
| | | EP1108790 | |
| | | WO0100805 | |
| | | EP0435132 | |
| | | EP1067192 | AX123560 |
| | | EP1067193 | AX063773 |
| dapB | Dihydrodipicolinat-Reduktase | EP1108790 | AX127149 |
| | EC 1.3.1.26 | WO0100843 | AX063753 |
| | (dihydrodipicolinat reductase) | EP1067192 | AX137723 |
| | | EP1067193 | AX137602 |
| | | Pisabarro et al., Journal of Bacteriology 175:2743-2749(1993) | X67737 |
| | | | Z21502 |
| | | JP1998215883 | |
| | | JP1997322774 | E16749 |
| | | JP1997070291 | E14520 |
| | | JP1995075578 | E12773 |
| | | | E08900 |
| dapC | N-Succinyl-Aminoketopimelat-Transaminase | EP1108790 | AX127146 |
| | | WO0100843 | AX064219 |
| | EC 2.6.1.17 | EP1136559 | |
| | (N-succinyl-diaminopimelate transaminase) | | |
| dapD | Tetrahydrodipicolinat-Succinylase | EP1108790 | AX127146 |
| | EC 2.3.1.117 | WO0100843 | AX063757 |
| | (tetrahydrodipicolinat succinylase) | Wehrmann et al. Journal of Bacteriology 180:3159-3165(1998) | AJ004934 |
| dapE | N-Succinyl-Diaminopimelat-Desuccinylase | EP1108790 | AX127146 |
| | | WO0100843 | AX063749 |
| | EC 3.5.1.18 | Wehrmann et al. Microbiology 140:3349-3356 (1994) | X81379 |
| | (N-succinyl-diaminopimelate desuccinylase) | | |
| dapF | Diaminopimelat Epimerase | EP1108790 | AX127149 |
| | EC 5.1.1.7 | WO0100843 | AX063719 |
| | (diaminopimelate epimerase) | EP1085094 | AX137620 |
| ddh | Diaminopimelate Dehydrogenase | EP1108790 | AX127152 |
| | EC 1.4.1.16 | WO0100843 | AX063759 |
| | (diaminopimelate dehydrogenase) | Ishino et al., Nucleic Acids Research 15:3917-3917 (1987) JP1997322774 | Y00151 |
| | | JP1993284970 | E14511 |
| | | Kim et al., Journal of Microbiology and Biotechnology 5:250-256(1995) | E05776 |
| | | | D87976 |
| dps | DNA-Protection Protein | EP1108790 | AX127153 |
| | (protection during starvation protein) | | |
| eno | Enolase | EP1108790 | AX127146 |
| | EC 4.2.1.11 | WO0100844 | AX064945 |
| | (enolase) | EP1090998 Hermann et al., Electrophoresis 19:3217-3221 (1998) | AX136862 |
| gap | Glyceraldehyd-3-Phosphat | EP1103790 | AX127148 |
| | Dehydrogenase | WO0100844 | AX064941 |
| | EC 1.2.1.12 | Eikmanns et al., Journal of Bacteriology 174:6076-6086(1992) | X59403 |
| | (glyceraldehyde-3-phosphate dehydrogenase) | | |
| gap2 | Glyceraldehyd-3-Phosphate | EP1108790 | AX127146 |
| | Dehydrogenase | WO0100844 | AX064939 |
| | EC 1.2.1.12 | | |
| | (glyceraldehyde-3-phosphate dehydrogenase 2) | | |
| gdh | Glutamat Dehydrogenase | EP1108790 | AX127150 |
| | EC 1.4.1.4 | WO0100844 | AX063811 |
| | (glutamate dehydrogenase) | Boermann et al., Molecular Microbiology | X59404 |
| | | 6:317-326 (1992). | X72855 |
| gnd | 6-Phosphogluconat, Dehydrogenase | EP1103790 | AX127147 |
| | EC 1.1.1.44 | | AX121689 |
| | (6-phosphogluconate dehydrogenase) | WO0100844 | AX065125 |
| lysC | Aspartatkinase | EP1108790 | AX120365 |
| | EC 2.7.2.4 | WO0100844 | AX063743 |
| | (aspartate kinase) | Kalinowski et al., Molecular Microbiology 5:1197-204 (1991) | X57226 |
| lysE | Lysinexporter | EP1108790 | AX123539 |
| | (lysine exporter protein) | WO0100843 | AX123539 |
| | | Vrljić et al., Molecular Microbiology 22:815-326 (1996) | X96471 |
| msiK | Zuckerimporter | EP1108790 | AX120892 |
| | (multiple sugar Import protein) | | |
| OpCA | Glukose-6-Phosphat-Dehydrogenase | WO0104325 | AX076272 |
| | (subunit of glucose-6-phosphate dehydrogenase) | | |
| OxyR | Transcriptionsregulator | EP1108790 | AX122198 |
| | (transcriptional regulator) | | AX127149 |
| ppc^{FBR} | Phosphoenolpyruvat Carboxylase feedback resistent | EP0723011 | |
| | | WO0100852 | |
| | EC 4.1.1.31 | | |
| | (phosphoenolpyruvate carboxylase feedback resistant) | | |
| ppc | Phosphoenolpyruvat Carboxylase | EP1108790 | AX127148 |
| | EC 4.1.1.31 | | AX123554 |
| | (phosphoenolpyruvate carboxylase) | O'Reagan et al., Gene 77(2):237-251(1989) | M25819 |
| pgk | Phosphoglycerat Kinase | EP1108790 | AX121838 |
| | EC 2.7.2.3 | | AX127148 |
| | (phosphoglycerate kinase) | WO0100844 | AX064943 |
| | | Eikmanns, Journal of Bacteriology 174:6076-6086 (1992) | X59403 |
| pknA | Protein kinase A | EP1108790 | AX120131 |
| | (protein kinase A) | | AX120035 |
| pknB | Protein kinase B | EP1108790 | AX120130 |
| | (protein kinase B) | | AX120085 |
| pknD | Protein kinase D | EP1108790 | AX127150 |
| | (protein kinase D) | | AX122469 |
| | | | AX122468 |
| pknG | Protein kinase G | EP1108790 | AX127152 |
| | (protein kinase G) | | AX123109 |
| ppsA | Phosphoenolpyruvat-Synthase | EP1103790 | AX127144 |
| | EC 2.7.9.2 | | AX120700 |
| | (phosphoenolpyruvate synthase) | | AX122469 |
| ptsH | Phosphotransferase System Protein H | EP1108790 | AX122210 |
| | EC 2.7.1.69 | | AX127149 |
| | (phosphotransferase System component H) | WO0100844 | AX069154 |
| ptsI | Phosphotransferase System Enzym I | EP1108790 | AX122206 |
| | EC 2.7.3.9 | | AX127149 |
| | (phosphotransferase system enzyme I) | | |
| ptSM | Glukose spezifisches Phosphotransferase System Enzym II | Lee et al., FEMS | L18874 |
| | EC 2.7.1.69 | Microbiology Letters 119(1-2):137-145 (1994) | |
| | (glucose-phosphotransferase-system enzyme II) | | |
| pyc | Pyrvat-Carboxylase | WO9918228 | A97276 |
| | EC 6.9.1.1 | Peters-Wendisch et al., Microbiology 144:915-927 (1998) | Y09543 |
| | (pyrvate Carboxylase) | | |
| pyc P458S | Pyrvat-Carboxylase | EP1108790 | |
| | EC 6.4.1.1 | | |
| | (pyrvate carboxylase) Aminosäureaustausch P458S | | |
| sigC | Sigmafaktor C | EP108790 | AX120368 |
| | EC 2.7.7.6 | | AX120085 |
| | (extracytoplasmic function alternative sigma factor C) | | |
| sigD | RNA Polymerase Sigmafaktor D | EP1108790 | AX120753 |
| | EC 2.7.7.6 | | AX127144 |
| | (RNA polymerase sigma factor) | | |
| sigE | Sigmafaktor E | EP1108790 | AX127146 |
| | EC 2.7.7.6 | | AX121325 |
| | (extracytoplasmic function alternative sigma factor E) | | |
| sigH | Sigmafaktor H | EP1108790 | AX127145 |
| | EC 2.7.7.6 | | AX120939 |
| | (sigma factor SigH) | | |
| sigM | Sigmafaktor M | EP108790 | AX123500 |
| | EC 2.7.7.6 | | AX127153 |
| | (sigma factor SigM) | | |
| tal | Transaldolase | WO0104325 | AX076272 |
| | EC 2.2.1.2 | | |
| | (transaldolase) | | |
| thyA | Thymidylat Synthase | EP1108790 | AX121026 |
| | EC 2.1.1.45 (thymidylate synthase) | | AX127145 |
| tkt | Transketolase EC 2.2.1.1 (transketolase) | Ikeda et al., NCBI | AB023377 |
| tpi | Triose-phosphat Isomerase | Eikmanns, Journal of Bacteriology 174:6076-6086 (1992) | X59403 |
| | EC 5.3.1.1 | | |
| | (triose-phosphate isomerase) | | |
| zwal | Zellwachstumsfaktor 1 (growth factor 1) | EP1111062 | AX133781 |
| zwf | Glukose-6-phosphat-1-Dehydrogenase | EP108790 | AX127148 |
| | EC 1.1.1.49 | | AX121827 |
| | (glucose-6-phosphate-1-dehydrogenase) | WO0104325 | AX076272 |
| zwf A213T | Glukose-6-phosphat-1-Dehydrogenase | EP1108790 | |
| | EC 1.1.1.49 | | |
| | (glucose-6-phosphate-1-dehydrogenase) Aminosäureaustausch A213T | | |

Weiterhin kann es für die Produktion von L-Lysin vorteilhaft sein, wenn man zusätzlich zur funktionellen Expression eines oder mehrerer der heterologen Gene des Glycerin-Stoffwechsels (glycerol metabolism), ausgewählt aus der Gruppe glpA, glpB, glpC, glpD, glpE, glpF, glpG, glpK, glpQ, glpT, glpX, gldA, dhaK, dhaL, dhaM, dhaR, fsa und talC, gleichzeitig eines oder mehrere der Gene, ausgewählt aus der Gruppe der Gene oder Allele, die für das Wachstum oder die Lysinproduktion nicht essentiell sind, abschwächt, insbesondere ausschaltet oder deren Expression verringert.

Hierzu gehören unter anderem folgende offene Leserahmen, Gene oder Allele: aecD, ccpA1, ccpA2, citA, citB, citE, fda, gluA, gluB, gluC, gluD, luxR, luxS, lysR1, lysR2, lysR3, menE, mqo, pck, pgi, poxB und zwa2, welche in Tabelle 2 zusammengefasst und erläutert sind.

**Tabelle 2:**

| Gene und Allele, die für die Lysinproduktion nicht essentiell sind | | | |
|---|---|---|---|
| Genname | Bezeichnung des kodierten Enzyms oder Proteins | Referenz | Zugangs nummer |
| aecD | beta C-S Lyase | Rossol et al., Journal of Bacteriology 179(9):2969-77 (1992) | M89931 |
| | EC 2.6.1.1 | | |
| | (beta C-S lyase) | | |
| ccpA1 | Katabolit-Kontroll-Protein | WO0100844 | AX065267 |
| | | EP1108790 | AX127147 |
| | (catabolite control protein A1) | WO 02/18419 | |
| ccpA2 | Katabolit-Kontroll-Protein (catabolite control protein A2) | WO0100844 | AX065267 |
| | | EP1108790 | AX121594 |
| citA | Sensor-Kinase CitA (sensor kinase CitA) | EP1108790 | AX120161 |
| citB | Transkriptionsregulator CitB | EP1108790 | AX120163 |
| | (transcription regulator CitB) | | |
| citE | Citrat-Lyase | WO0100344 | AX065421 |
| | EC 4.1.3.6 | EP1108790 | AX127146 |
| | (citrate lyase) | | |
| fda | Fruktose-bispluosphat-Aldolase | von der Osten et al., Molecular Microbiology 3(11):1625-37 (1989) | X17313 |
| | EC 4.1.2.13 | | |
| | (fructose 1,6-bisphosphate aldolase) | | |
| gluA | Glutamat-Transport ATP-bindendes Protein | Krorieirteyer et al., Journal of Bacteriology 177(5):1152-8 (1995) | X81191 |
| | (glutamate transport ATP-binding protein) | | |
| gluB | Glutamat-bindendes Protein | Kronemeyer et al., Journal of Bacteriology 177(5):1152-8 (1995) | X81191 |
| | (glutamate binding protein) | | |
| gluC | Glutamat-Transport Permease | Kronemeyer et al., Journal of Bacteriology 177(5):1152-8 (1995) | X81191 |
| | (glutamate transport system permease) | | |
| gluD | Glutamat-Transport Permease | Kronemeyer et al., Journal of Bacteriology 177 (5) : 1152-8 (1995) | X81191 |
| | (glutamate transport system permease) | | |
| luxR | Transkriptionsregulator | WO0100842 | AX065953 |
| | LuxR (transcription regulator LuxR) | EP1108790 | AX123320 |
| luxS | Histidin-Kinase LuxS | EP1108790 | AX123323 |
| | (histidine kinase LuxS) | | AX127153 |
| lysR1 | Transkriptionsregulator | EP1108790 | AX064673 |
| | LysR1 | | AX127144 |
| | (transcription regulator LysR1) | | |
| lysR2 | Transkriptionsaktivator LysR2 | EP1108790 | AX123312 |
| | (transcription regulator LysR2) | | |
| lysR3 | Transkriptionsregulator | WO0100842 | AX065957 |
| | LysR3 | EP1108790 | AX127150 |
| | (transcription regulator LysR3) | | |
| menE | O-Succinylbenzoesäure- | WO0100843 | AX064599 |
| | CoA-Ligase | EP1108790 | AX064193 |
| | EC 6.2.1.26 | | AX127144 |
| | (O-succinylbenzoate-CoA ligase) | | |
| mqo | Malat-Chinon-Oxidoreduktase | Molenaar et al., Eur. Journal of Biochemistry 1;254(2):395-403 (1998) | AJ224946 |
| | (malate-quinone-oxidoreductase) | | |
| pck | Phosphoenolpyruvat-Carboxykinase | WO0100844 | AJ269506 |
| | | EP-A-1099111 | AX065053 |
| | (phosphoenolpyruvate carboxykinase) | | |
| pgi | Glucose-6-Phosphat-Isomerase | EP1087015 | AX136015 |
| | | EP1103790 | AX127146 |
| | Sec 5.3.1.9 | WO 01/07626 | |
| | (glucose-6-phosphate isomerase) | | |
| poxB | Pyruvat-Oxidase | WO0100844 | AX064959 |
| | EC 1.2.3.3 | EP1096013 | AX137665 |
| | (pyruvate oxidase) | | |
| zwa2 | Zellwachstumsfaktor 2 | EP1106693 | AX113822 |
| | (growth factor 2) | EP1108790 | AX127146 |

Auch endogene Polynukleotide von Corynebacterium glutamicum (Accession No.: NC 006958 bzw. NC 003450 (Sequenz des gesamten Genoms)), die beispielsweise mindestens 45% oder mindestens 50% oder mindestens 60% oder mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zu den in SEQ ID No. 1, SEQ ID No. 3, SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No. 27, SEQ ID No. 29, SEQ ID No. 31, SEQ ID No. 33 oder SEQ ID No. 35 dargestellten Nukleotidsequenzen besitzen und die beschriebenen Funktionen aufweisen, können verstärkt werden. Die aus den Genomprojekten bekannten Nukleotidsequenzen der Gene und ORF's gehören zum Stand der Technik und können verschiedenen Veröffentlichungen, Patentanmeldungen sowie der Datenbank des National Center for Biotechnology Information (NCBI) der National Library of Medicin (Bethesda, MD, USA) entnommen werden.

Es wurde gefunden, dass coryneforme Bakterien auch nach gemeinsamer Überexpression der endogenen Polynukleotide glpK (58% Identität zu SEQ ID No. 15), kodierend für die ATP-abhängigen Glycerinkinase K, und glpD (47% Identität zu SEQ ID No. 7), kodierend für die Glycerin-3-Phosphat-Dehydrogenase des Glycerin-Stoffwechsels (glycerol metabolism) L-Aminosäuren, insbesondere L-Lysin und L-Tryptophan, aus Glycerin als alleiniger Kohlenstoffquelle produzieren.

Der besseren Übersichtlichkeit halber sind die bekannten Sequenzen zu den behandelten Genen von Corynebacterium glutamicum unter den SEQ ID No. 37 und SEQ ID No. 39 dargestellt.

Die erfindungsgemäßen Bakterien können kontinuierlich - wie beispielsweise in der PCT/EP2004/008882 beschrieben - oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle wird Glycerin eingesetzt. Dieses kann einzeln oder als Mischung verwendet werden. Zugesetzt werden können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Ethanol und Methanol und organische Säuren wie z.B. Essigsäure, wobei der Anteil des Glycerins mindestens größer gleich (≥) 10%, oder mindestens ≥ 25%, oder mindestens ≥ 50%, oder mindestens ≥ 75%, oder mindestens ≥ 90%, oder mindestens ≥ 95%, mindestens ≥ 99%, bevorzugt 100% betragen soll.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht. Bei kontinuierlichen Verfahren sind längere Kultivierungszeiten möglich.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

## Patentansprüche

1. Verfahren zur fermentativen Herstellung von L-Aminosäuren,
**dadurch gekennzeichnet,**
**dass** man folgende Schritte durchführt:
a) Kultivierung der die gewünschte L-Aminosäure produzierenden rekombinanten coryneformen Bakterien, in denen mindestens das heterologe Polynukleotid glpK kodierend für ein Polypeptid mit der Aktivität Glycerinkinase exprimiert oder das endogene Polynukleotid glpK überexprimiert vorliegt, in einem Glycerin oder gegebenenfalls zusätzlich eine oder mehrere weitere C- Quellen enthaltenden Medium unter Bedingungen, bei denen die gewünschte L-Aminosäure im Medium oder in den Zellen angereichert wird, und gegebenenfalls
b) Isolierung der gewünschten L-Aminosäure, wobei gegebenenfalls Bestandteile der Fermentationsbrühe und/oder Biomasse in ihrer Gesamtheit oder in Anteilen (> 0 bis 100%) im Endprodukt verbleiben,
wobei das Polypeptid mit der Aktivität einer Glycerinkinase, kodiert vom heterologen Polynukleotid glpK, eine Aminosäuresequenz aufweist, die zu mindestens 80% identisch ist mit der Aminosäuresequenz von SEQ ID NO:16
und wobei das Polypeptid mit der Aktivität einer Glycerinkinase, kodiert vom endogenen Polynukleotid glpK, die Aminosäuresequenz von SEQ ID NO:38 aufweist.

2. Verfahren gemäss Anspruch 1, **dadurch gekennzeichnet, dass** das heterologe Polypeptid mit der Aktivität einer Glycerinkinase eine Aminosäuresequenz aufweist, die zu 100% identisch ist mit der Aminosäuresequenz von SEQ ID NO:16.

3. Verfahren gemäss Anspruch 1, **dadurch gekennzeichnet, dass** in den die gewünschte L-Aminosäure produzierenden rekombinanten Bakterien der Art Corynebacterium glutamicum zusätzlich das heterologe Polynukleotid glpD kodierend für ein Polypeptid mit der Aktivität Glycerin-3-Phosphat-Dehydrogenase oder das endogene Polynukleotid glpD überexprimiert vorliegt, wobei das Polypeptid mit der Aktivität einer Glycerin-3-Phosphat-Dehydrogenase, kodiert von dem heterologen Polynukleotid glpD, eine Aminosäuresequenz aufweist, die zu mindestens 80% identisch ist mit der Aminosäuresequenz von SEQ ID NO:8 und wobei das Polypeptid mit der Aktivität einer Glycerin-3-Phosphat-Dehydrogenase, kodiert vom endogenen Polynukleotid glpD, die Aminosäuresequenz von SEQ ID NO:40 aufweist.

4. Verfahren gemäss Anspruch 4, **dadurch gekennzeichnet, dass** das heterologe Polypeptid mit der Aktivität einer Glycerin-3-Phosphat-Dehydrogenase eine Aminosäuresequenz aufweist, die zu 100% identisch ist mit der Aminosäuresequenz von SEQ ID NO:8.

5. Verfahren nach einem oder mehreren der Ansprüche 1 bis 4, in denen das exprimierte heterologe Polynukleotid aus einem prokaryontischen Organismus, vorzugsweise aus Enterobacteriaceae, ganz besonders beovorzugt aus Escherichia coli stammt.

6. Verfahren gemäss Anspruch 1, **dadurch gekennzeichnet, dass** man als C- Quelle zu ≥ 10 bis 100% Glycerin einsetzt.

7. Verfahren gemäß Anspruch 1 bis 6, **dadurch gekennzeichnet, dass** man L- Lysin oder L- Tryptophan herstellt.

8. Verfahren nach einem oder mehreren der Ansprüche 1 bis 7, **dadurch gekennzeichnet, dass** man Bakterien einsetzt, in denen die Stoffwechselwege zumindest teilweise verstärkt sind, die die Bildung der gewünschten L-Aminosäure erhöhen.

9. Verfahren nach einem oder mehreren der Ansprüche 1 bis 8, **dadurch gekennzeichnet, dass** man Bakterien einsetzt, in denen die Stoffwechselwege zumindest teilweise ausgeschaltet sind, die die Bildung der gewünschten L-Aminosäure verringern.

10. Verfahren gemäß Anspruch 8, **dadurch gekennzeichnet, daß** man für die Herstellung von L-Aminosäuren, insbesondere L-Lysin und L-Tryptophan, Bakterien der Art Corynebacterium glutamicum fermentiert, in denen man gleichzeitig eines oder mehrere der Gene, ausgewählt aus der Gruppe accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf, zwf A243T, glpA, glpB, glpC, glpE, glpF, glpG, glpQ, glpT, glpX, gldA, dhaK, dhaL, dhaM, dhaR, fsa und talC verstärkt, insbesondere überexprimiert.

11. Verfahren gemäß Anspruch 8, **dadurch gekennzeichnet, dass** die Aktivität oder Konzentration des (der) Protein(e), für das das (die) verstärkte(n) Gen(e) kodiert(en), um jeweils 10 bis 2000 % zunimmt, bezogen auf die des Wildtyp-Proteins beziehungsweise die Aktivität oder Konzentration des Proteins im Ausgangs-Mikroorganismus.

12. Verfahren nach Anspruch 9, **dadurch gekennzeichnet, dass** man zur Herstellung von L-Aminosäuren, insbesondere L-Lysin, Bakterien der Art Corynebacterium glutamicum fermentiert, in denen man gleichzeitig eines oder mehrere der Gene, ausgewählt aus der Gruppe aecD, ccpA1, ccpA2, citA, citB, citE, fda, gluA, gluB, gluC, gluD, luxR, luxS, lysR1, lysR2, lysR3, menE, mqo, pck, pgi, poxB und zwa2 abschwächt, insbesondere ausschaltet oder die Expression verringert.

13. Verfahren gemäß Anspruch 12, **dadurch gekennzeichnet, dass** die Aktivität oder Konzentration des (der) Protein(e), für das das (die) abgeschwächte(n) Gen(e) kodiert(en), auf jeweils 0 bis 75 % absinkt, bezogen auf die des Wildtyp-Proteins beziehungsweise die Aktivität oder Konzentration des Proteins im Ausgangs-Mikroorganismus.

## Claims

1. Method for the fermentative production of L-amino acids, **characterized in that** the following steps are carried out:
a) culturing the recombinant coryneform bacteria producing the desired L-amino acid, in which bacteria at least the heterologous polynucleotide glpK encoding a polypeptide having glycerol kinase activity is expressed, or the endogenous polynucleotide glpK is overexpressed, in a medium containing glycerol or, optionally, in addition one or more further carbon sources under conditions in which the desired L-amino acid accumulates in the medium or in the cells, and, optionally,
b) isolating the desired L-amino acid, in which optionally components of the fermentation broth and/or biomass remain in the end product in their totality or in fractions (> 0 to 100%),
wherein the polypeptide with the activity of glycerol kinase encoded by the heterologous polynucleotide glpK has an amino acid sequence which is at least 80% identical to the amino acid sequence of SEQ ID NO: 16
and wherein the polypeptide with the activity of a glycerol kinase encoded by the endogenous polynucleotide glpK has the amino acid sequence of SEQ ID NO: 38.

2. Method according to Claim 1, **characterized in that** the heterologous polypeptide with the activity of a glycerol kinase has an amino acid sequence which is 100% identical to the amino acid sequence of SEQ ID NO: 16.

3. Method according to Claim 1, **characterized in that**, in the recombinant bacteria of the species Corynebacterium glutamicum producing the desired L-amino acid, in addition the heterologous polynucleotide glpD encoding a polypeptide with glycerol-3-phosphate dehydrogenase activity or the endogenous polynucleotide glpD is overexpressed, wherein the polypeptide having the activity of a glycerol-3-phosphate dehydrogenase encoded by the heterologous polynucleotide glpD has an amino acid sequence at least 80% identical to the amino acid sequence of SEQ ID NO: 8, and wherein the polypeptide with the activity of a glycerol-3-phosphate dehydrogenase encoded by the endogenous polynucleotide glpD has the amino acid sequence of SEQ ID NO: 40.

4. Method according to Claim 4, **characterized in that** the heterologous polypeptide with the activity of a glycerol-3-phosphate dehydrogenase has an amino acid sequence which is 100% identical to the amino acid sequence of SEQ ID NO: 8.

5. Method according to one or more of Claims 1 to 4, in which the heterologous polynucleotide expressed originates from a prokaryotic organism, preferably from Enterobacteriaceae, very particularly preferably from Escherichia coli.

6. Method according to Claim 1, **characterized in that**, as carbon source, use is made of ≥ 10 to 100% glycerol.

7. Method according to Claims 1 to 6, **characterized in that** L-lysine or L-tryptophan is produced.

8. Method according to one or more of Claims 1 to 7, **characterized in that** use is made of bacteria in which the metabolic pathways which increase the formation of the desired L-amino acid have been at least in part amplified.

9. Method according to one or more of Claims 1 to 8, **characterized in that** use is made of bacteria in which the metabolic pathways which decrease the formation of the desired L-amino acid have been at least in part switched off.

10. Method according to Claim 8, **characterized in that**, for the production of L-amino acids, in particular L-lysine and L-tryptophan, bacteria of the species Corynebacterium glutamicum are fermented in which at the same time one or more of the genes selected from the group accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf, zwf A243T, glpA, glpB, glpC, glpE, glpF, glpG, glpQ, glpT, glpX, gldA, dhaK, dhaL, dhaM, dhaR, fsa and talC is or are amplified, in particular overexpressed.

11. Method according to Claim 8, **characterized in that** the activity or concentration of the protein(s) which is (are) encoded by the amplified gene(s) increases by in each case 10 to 2000%, based on that of the wild type protein or the activity or concentration of the protein in the starting microorganism.

12. Method according to Claim 9, **characterized in that**, for production of L-amino acids, in particular L-lysine, bacteria of the species Corynebacterium glutamicum are fermented in which at the same time one or more of the genes selected from the group aecD, ccpA1, ccpA2, citA, citB, citE, fda, gluA, gluB, gluC, gluD, luxR, luxS, lysR1 lysR2, lysR3, menE, mqo, pck, pgi, poxB and zwa2 is or are attenuated, in particular switched off, or the expression is decreased.

13. Method according to Claim 12, **characterized in that** the activity or concentration of the protein(s) which is (are) encoded by the attenuated gene(s) falls in each case to 0 to 75%, based on that of the wild type protein or the activity or concentration of the protein in the starting microorganism.

## Revendications

1. Procédé pour la production par fermentation d'acides aminés L,
**caractérisé en ce**
**qu'**on effectue les étapes suivantes :
a) culture des bactéries corynéformes recombinantes, produisant l'acide aminé L recherché, chez lesquelles au moins le polynucléotide glpK hétérologue codant pour un polypeptide ayant l'activité glycérol kinase se trouve exprimé ou le polynucléotide glpK endogène se trouve surexprimé, dans un milieu contenant du glycérol ou éventuellement en plus une ou plusieurs autres sources de carbone, dans des conditions dans lesquelles l'acide aminé L recherché est accumulé dans le milieu ou dans les cellules, et éventuellement
b) isolement de l'acide aminé L recherché, éventuellement des composants du bouillon de fermentation et/ou la biomasse restant en leur totalité ou en certaines proportions (> 0 à 100 %) dans le produit final,
le polypeptide ayant l'activité d'une glycérol kinase, codé par le polynucléotide glpK hétérologue, présentant une séquence d'acides aminés qui est identique à raison d'au moins 80 % à la séquence d'acides aminés de SEQ ID N° 16
et le polypeptide ayant l'activité d'une glycérol kinase, codé par le polynucléotide glpK endogène, présentant la séquence d'acides aminés de SEQ ID N° 38.

2. Procédé selon la revendication 1, **caractérisé en ce que** le polypeptide hétérologue ayant l'activité d'une glycérol kinase présente une séquence d'acides aminés qui est identique à raison de 100 % à la séquence d'acides aminés de SEQ ID N° 16.

3. Procédé selon la revendication 1, **caractérisé en ce que** chez les bactéries recombinantes appartenant à l'espèce *Corynebacterium glutamicum,* produisant l'acide aminé L recherché, en outre le polynucléotide glpD hétérologue codant pour un polypeptide ayant l'activité glycérol-3-phosphate déshydrogénase ou le polynucléotide glpD endogène se trouve surexprimé,
le polypeptide ayant l'activité d'une glycérol-3-phosphate déshydrogénase, codé par le polynucléotide glpD hétérologue, présentant une séquence d'acides aminés qui est identique à raison d'au moins 80 % à la séquence d'acides aminés de SEQ ID N° 8
et le polypeptide ayant l'activité d'une glycérol-3-phosphate déshydrogénase, codé par le polynucléotide glpD endogène, présentant la séquence d'acides aminés de SEQ ID N° 40.

4. Procédé selon la revendication 1, **caractérisé en ce que** le polypeptide hétérologue ayant l'activité d'une glycérol-3-phosphate déshydrogénase présente une séquence d'acides aminés qui est identique à raison de 100 % à la séquence d'acides aminés de SEQ ID N° 8.

5. Procédé selon une ou plusieurs des revendications 1 à 4, dans lequel le polynucléotide hétérologue exprimé provient d'un organisme procaryote, de préférence d'*Enterobacteriaceae,* de façon tout particulièrement préférée *d'Escherichia coli.*

6. Procédé selon la revendication 1, **caractérisé en ce qu'**on utilise comme source de carbone à raison de ≥ 10 à 100 % du glycérol.

7. Procédé selon l'une quelconque des revendications 1 à 6, **caractérisé en ce qu'**on produit de la L-lysine ou du L-tryptophane.

8. Procédé selon une ou plusieurs des revendications 1 à 7, **caractérisé en ce qu'**on utilise des bactéries chez lesquelles les voies métaboliques qui augmentent la formation de l'acide aminé L recherché sont au moins en partie renforcées.

9. Procédé selon une ou plusieurs des revendications 1 à 8, **caractérisé en ce qu'**on utilise des bactéries chez lesquelles les voies métaboliques qui réduisent la formation de l'acide aminé L recherché sont au moins en partie inactivées.

10. Procédé selon la revendication 8, **caractérisé en ce que** pour la production d'acides aminés L, en particulier la L-lysine et le L-tryptophane, on cultive par fermentation des bactéries appartenant à l'espèce *Corynebacterium glutamicum,* chez lesquelles on renforce, en particulier surexprime, simultanément un ou plusieurs des gènes choisis dans le groupe constitué par accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf, zwf A243T, glpA, glpB, glpC, glpE, glpF, glpG, glpQ, glpT, glpX, gldA, dhaK, dhaL, dhaM, dhaR, fsa et talC.

11. Procédé selon la revendication 8, **caractérisé en ce que** l'activité ou la concentration de la (des) protéine(s), pour laquelle (lesquelles) code(nt) le(s) gène(s) renforcé(s), augmente respectivement de 10 à 2000 %, par rapport à celle de la protéine de type sauvage ou à l'activité ou la concentration de la protéine chez l'organisme de départ.

12. Procédé selon la revendication 9, **caractérisé en ce que** pour la production d'acides aminés L, en particulier la L-lysine, on cultive par fermentation des bactéries appartenant à l'espèce *Corynebacterium glutamicum,* chez lesquelles on affaiblit, en particulier inactive, simultanément un ou plusieurs des gènes choisis dans le groupe constitué par aecD, ccpA1, ccpA2, citA, citB, citE, fda, gluA, gluB, gluC, gluD, luxR, luxS, lysR1, lysR2, lysR3, menE, mqo, pck, pgi, poxB et zwa2 ou diminue leur expression.

13. Procédé selon la revendication 12, **caractérisé en ce que** l'activité ou la concentration de la (des) protéine(s), pour laquelle (lesquelles) code(nt) le(s) gène(s) affaibli(s), est réduite respectivement de 0 à 75 %, par rapport à celle de la protéine de type sauvage ou à l'activité ou la concentration de la protéine chez l'organisme de départ.


### SEQUENCE LISTING

<110> Evonik Degssa GmbH
   Forschungszentrum Jülich GmbH
<120> Verfahren zur fermentativen Herstellung von L-Aminosäuren unter Verwendung coryneformer Bakterien, in denen Gene des Glycerin-Stoffwechsels heterolog exprimiert werden
<130> 2005P10064 WE01
<160> 40
<170> PatentIn version 3.3
<210> 1
   <211> 1629
   <212> DNA
   <213> Escherichia coli
<220>
   <221> CDS
   <222> (1)..(1629)
   <223> glpA Kodierregion
<400> 1
   atg aaa act cgc gac tcg caa tca agt gac gtg att atc att ggc ggc        48
   Met Lys Thr Arg Asp Ser Gln Ser Ser Asp Val Ile Ile Ile Gly Gly
   1               5                   10                  15
   ggc gca acg gga gcc ggg att gcc cgc gac tgt gcc ctg cgc ggg ctg        96
   Gly Ala Thr Gly Ala Gly Ile Ala Arg Asp Cys Ala Leu Arg Gly Leu
               20                  25                  30
   cgc gtg att ttg gtt gag cgc cac gac atc gca acc ggt gcc acc ggg       144
   Arg Val Ile Leu Val Glu Arg His Asp Ile Ala Thr Gly Ala Thr Gly
           35                  40                  45
   cgt aac cac ggc ctg ctg cac agc ggt gcg cgc tat gcg gta acc gat       192
   Arg Asn His Gly Leu Leu His Ser Gly Ala Arg Tyr Ala Val Thr Asp
       50                  55                  60
   gcg gaa tcg gcc cgc gaa tgc att agt gaa aac cag atc ctg aaa cgc       240
   Ala Glu Ser Ala Arg Glu Cys Ile Ser Glu Asn Gln Ile Leu Lys Arg
   65                  70                  75                  80
   att gca cgt cac tgc gtt gaa cca acc aac ggc ctg ttt atc acc ctg       288
   Ile Ala Arg His Cys Val Glu Pro Thr Asn Gly Leu Phe Ile Thr Leu
                   85                  90                  95
   ccg gaa gat gac ctc tcc ttc cag gcc act ttt att cgc gcc tgc gaa       336
   Pro Glu Asp Asp Leu Ser Phe Gln Ala Thr Phe Ile Arg Ala Cys Glu
               100                 105                 110
   gaa gca ggg atc agc gca gaa gct ata gac ccg cag caa gcg cgc att       384
   Glu Ala Gly Ile Ser Ala Glu Ala Ile Asp Pro Gln Gln Ala Arg Ile
           115                 120                 125
   atc gaa cct gcc gtt aac ccg gca ctg att ggc gcg gtg aaa gtt ccg      432
   Ile Glu Pro Ala Val Asn Pro Ala Leu Ile Gly Ala Val Lys Val Pro
       130                 135                 140
   gat ggc acc gtt gat cca ttt cgt ctg acc gca gca aac atg ctg gat      480
   Asp Gly Thr Val Asp Pro Phe Arg Leu Thr Ala Ala Asn Met Leu Asp
   145                 150                 155                 160
   gcc aaa gaa cac ggt gcc gtt atc ctt acc gct cat gaa gtc acg ggg      528
   Ala Lys Glu His Gly Ala Val Ile Leu Thr Ala His Glu Val Thr Gly
                   165                 170                 175
   ctg att cgt gaa ggc gcg acg gtg tgc ggt gtt cgt gta cgt aac cat      576
   Leu Ile Arg Glu Gly Ala Thr Val Cys Gly Val Arg Val Arg Asn His
               180                 185                 190
   ctc acc ggc gaa act cag gcc ctt cat gca cct gtc gtg gtt aat gcc      624
   Leu Thr Gly Glu Thr Gln Ala Leu His Ala Pro Val Val Val Asn Ala
           195                 200                 205
   gct ggg atc tgg ggg caa cac att gcc gaa tat gcc gat ctg cgc att      672
   Ala Gly Ile Trp Gly Gln His Ile Ala Glu Tyr Ala Asp Leu Arg Ile
       210                 215                 220
   cgc atg ttc ccg gcg aaa gga tcg ctg ctg atc atg gat cac cgc att      720
   Arg Met Phe Pro Ala Lys Gly Ser Leu Leu Ile Met Asp His Arg Ile
   225                 230                 235                 240
   aac cag cat gtg atc aac cgc tgc cgt aaa cct tcc gac gcc gat att      768
   Asn Gln His Val Ile Asn Arg Cys Arg Lys Pro Ser Asp Ala Asp Ile
                   245                 250                 255
   ctg gtg cct ggc gat acc att tcg ctg att ggt acc acc tct tta cgt      816
   Leu Val Pro Gly Asp Thr Ile Ser Leu Ile Gly Thr Thr Ser Leu Arg
               260                 265                 270
   att gat tac aac gag att gac gat aat cga gtg acg gca gaa gag gtt      864
   Ile Asp Tyr Asn Glu Ile Asp Asp Asn Arg Val Thr Ala Glu Glu Val
           275                 280                 285
   gat att ctg ctg cgt gaa ggg gaa aaa ctg gcc ccc gtg atg gcg aaa      912
   Asp Ile Leu Leu Arg Glu Gly Glu Lys Leu Ala Pro Val Met Ala Lys
       290                 295                 300
   acg cgc att ttg cgg gcc tat tct ggc gtg cgc ccg ctg gtt gcc agc      960
   Thr Arg Ile Leu Arg Ala Tyr Ser Gly Val Arg Pro Leu Val Ala Ser
   305                 310                 315                 320
   gat gac gac ccg agc gga cgt aac gtc agc cgt ggc atc gtg ctg ctc     1008
   Asp Asp Asp Pro Ser Gly Arg Asn Val Ser Arg Gly Ile Val Leu Leu
                   325                 330                 335
   gac cat gct gaa cgc gat ggt ctg gac gga ttt atc acc atc acc ggt     1056
   Asp His Ala Glu Arg Asp Gly Leu Asp Gly Phe Ile Thr Ile Thr Gly
               340                 345                 350
   ggc aaa ctg atg acc tat cgg ctg atg gct gaa tgg gct acc gac gcg     1104
   Gly Lys Leu Met Thr Tyr Arg Leu Met Ala Glu Trp Ala Thr Asp Ala
           355                 360                 365
   gta tgc cgc aaa ctg ggc aac acg cgc ccc tgt acg act gcc gat ctg     1152
   Val Cys Arg Lys Leu Gly Asn Thr Arg Pro Cys Thr Thr Ala Asp Leu
       370                 375                 380
   gca ctg cct ggt tca caa gaa ccc gct gaa gtt acc ttg cgt aaa gtc      1200
   Ala Leu Pro Gly Ser Gln Glu Pro Ala Glu Val Thr Leu Arg Lys Val
   385                 390                 395                 400
   atc tcc ctg cct gcc ccg ctg cgc ggt tct gcg gtt tat cgt cat ggc      1248
   Ile Ser Leu Pro Ala Pro Leu Arg Gly Ser Ala Val Tyr Arg His Gly
                   405                 410                 415
   gat cgc acg cct gcc tgg ctg agc gaa ggc cgt ctg cac cgt agc ctg      1296
   Asp Arg Thr Pro Ala Trp Leu Ser Glu Gly Arg Leu His Arg Ser Leu
               420                 425                 430
   gta tgt gag tgc gaa gcg gta act gcg ggt gaa gtg cag tac gcg gta      1344
   Val Cys Glu Cys Glu Ala Val Thr Ala Gly Glu Val Gln Tyr Ala Val
           435                 440                 445
   gaa aat tta aac gtt aat agc ctg ctg gat tta cgc cgt cgt acc cgt      1392
   Glu Asn Leu Asn Val Asn Ser Leu Leu Asp Leu Arg Arg Arg Thr Arg
       450                 455                 460
   gtg ggg atg ggc acc tgc cag ggc gaa ctc tgc gcc tgc cgc gct gcc      1440
   Val Gly Met Gly Thr Cys Gln Gly Glu Leu Cys Ala Cys Arg Ala Ala
   465                 470                 475                 480
   gga ctg ctg caa cgt ttt aac gtc acg acg tcc gcg caa tct atc gag      1488
   Gly Leu Leu Gln Arg Phe Asn Val Thr Thr Ser Ala Gln Ser Ile Glu
                   485                 490                 495
   caa ctt tcc acc ttc ctt aac gaa cgc tgg aaa ggc gtg caa ccc atc      1536
   Gln Leu Ser Thr Phe Leu Asn Glu Arg Trp Lys Gly Val Gln Pro Ile
               500                 505                 510
   gcc tgg gga gat gca ctg cgc gaa agc gaa ttt acc cgc tgg gtt tat      1584
   Ala Trp Gly Asp Ala Leu Arg Glu Ser Glu Phe Thr Arg Trp Val Tyr
           515                 520                 525
   cag gga ttg tgt ggt ctg gag aag gag cag aaa gat gcg ctt tga          1629
   Gln Gly Leu Cys Gly Leu Glu Lys Glu Gln Lys Asp Ala Leu
       530                 535                 540
<210> 2
   <211> 542
   <212> PRT
   <213> Escherichia coli
<400> 2
   Met Lys Thr Arg Asp Ser Gln Ser Ser Asp Val Ile Ile Ile Gly Gly
   1               5                   10                  15
   Gly Ala Thr Gly Ala Gly Ile Ala Arg Asp Cys Ala Leu Arg Gly Leu
               20                  25                  30
   Arg Val Ile Leu Val Glu Arg His Asp Ile Ala Thr Gly Ala Thr Gly
           35                  40                  45
   Arg Asn His Gly Leu Leu His Ser Gly Ala Arg Tyr Ala Val Thr Asp
   50 55 60
   Ala Glu Ser Ala Arg Glu Cys Ile Ser Glu Asn Gln Ile Leu Lys Arg
   65 70 75 80
   Ile Ala Arg His Cys Val Glu Pro Thr Asn Gly Leu Phe Ile Thr Leu
   85 90 95
   Pro Glu Asp Asp Leu Ser Phe Gln Ala Thr Phe Ile Arg Ala Cys Glu
   100 105 110
   Glu Ala Gly Ile Ser Ala Glu Ala Ile Asp Pro Gln Gln Ala Arg Ile
   115 120 125
   Ile Glu Pro Ala Val Asn Pro Ala Leu Ile Gly Ala Val Lys Val Pro
   130 135 140
   Asp Gly Thr Val Asp Pro Phe Arg Leu Thr Ala Ala Asn Met Leu Asp
   145 150 155 160
   Ala Lys Glu His Gly Ala Val Ile Leu Thr Ala His Glu Val Thr Gly
   165 170 175
   Leu Ile Arg Glu Gly Ala Thr Val Cys Gly Val Arg Val Arg Asn His
   180 185 190
   Leu Thr Gly Glu Thr Gln Ala Leu His Ala Pro Val Val Val Asn Ala
   195 200 205
   Ala Gly Ile Trp Gly Gln His Ile Ala Glu Tyr Ala Asp Leu Arg Ile
   210 215 220
   Arg Met Phe Pro Ala Lys Gly Ser Leu Leu Ile Met Asp His Arg Ile
   225 230 235 240
   Asn Gln His Val Ile Asn Arg Cys Arg Lys Pro Ser Asp Ala Asp Ile
   245 250 255
   Leu Val Pro Gly Asp Thr Ile Ser Leu Ile Gly Thr Thr Ser Leu Arg
   260 265 270
   Ile Asp Tyr Asn Glu Ile Asp Asp Asn Arg Val Thr Ala Glu Glu Val
   275 280 285
   Asp Ile Leu Leu Arg Glu Gly Glu Lys Leu Ala Pro Val Met Ala Lys
   290                 295                 300
   Thr Arg Ile Leu Arg Ala Tyr Ser Gly Val Arg Pro Leu Val Ala Ser
   305                 310                 315                 320
   Asp Asp Asp Pro Ser Gly Arg Asn Val Ser Arg Gly Ile Val Leu Leu
                   325                 330                 335
   Asp His Ala Glu Arg Asp Gly Leu Asp Gly Phe Ile Thr Ile Thr Gly
               340                 345                 350
   Gly Lys Leu Met Thr Tyr Arg Leu Met Ala Glu Trp Ala Thr Asp Ala
           355                 360                 365
   Val Cys Arg Lys Leu Gly Asn Thr Arg Pro Cys Thr Thr Ala Asp Leu
       370                 375                 380
   Ala Leu Pro Gly Ser Gln Glu Pro Ala Glu Val Thr Leu Arg Lys Val
   385                 390                 395                 400
   Ile Ser Leu Pro Ala Pro Leu Arg Gly Ser Ala Val Tyr Arg His Gly
                   405                 410                 415
   Asp Arg Thr Pro Ala Trp Leu Ser Glu Gly Arg Leu His Arg Ser Leu
               420                 425                 430
   Val Cys Glu Cys Glu Ala Val Thr Ala Gly Glu Val Gln Tyr Ala Val
           435                 440                 445
   Glu Asn Leu Asn Val Asn Ser Leu Leu Asp Leu Arg Arg Arg Thr Arg
       450                 455                 460
   Val Gly Met Gly Thr Cys Gln Gly Glu Leu Cys Ala Cys Arg Ala Ala
   465                 470                 475                 480
   Gly Leu Leu Gln Arg Phe Asn Val Thr Thr Ser Ala Gln Ser Ile Glu
                   485                 490                 495
   Gln Leu Ser Thr Phe Leu Asn Glu Arg Trp Lys Gly Val Gln Pro Ile
               500                 505                 510
   Ala Trp Gly Asp Ala Leu Arg Glu Ser Glu Phe Thr Arg Trp Val Tyr
           515                 520                 525
   Gln Gly Leu Cys Gly Leu Glu Lys Glu Gln Lys Asp Ala Leu
       530                 535                 540
<210> 3
   <211> 1260
   <212> DNA
   <213> Escherichia coli
<220>
   <221> CDS
   <222> (1)..(1260)
   <223> glpB Kodierregion
<400> 3
   atg cgc ttt gat act gtc att atg ggc ggc ggc ctc gcc gga tta ctc      48
   Met Arg Phe Asp Thr Val Ile Met Gly Gly Gly Leu Ala Gly Leu Leu
   1               5                   10                  15
   tgt ggc ctg caa ctg caa aaa cac ggc ctg cgc tgt gcc att gtc act      96
   Cys Gly Leu Gln Leu Gln Lys His Gly Leu Arg Cys Ala Ile Val Thr
               20                  25                  30
   cgt ggt caa agc gca ctg cat ttc tca tcc gga tcg ctg gat ttg ctg     144
   Arg Gly Gln Ser Ala Leu His Phe Ser Ser Gly Ser Leu Asp Leu Leu
           35                  40                  45
   agc cat ctg cca gat ggt caa ccg gtg aca gac att cac agt gga ctg     192
   Ser His Leu Pro Asp Gly Gln Pro Val Thr Asp Ile His Ser Gly Leu
       50                  55                  60
   gaa tct ttg cgt cag cag gca cca gcc cat cct tac tcc ctt ctc gag     240
   Glu Ser Leu Arg Gln Gln Ala Pro Ala His Pro Tyr Ser Leu Leu Glu
   65                  70                  75                  80
   cca caa cgc gtg ctc gat ctc gct tgc cag gcg cag gca tta atc gct     288
   Pro Gln Arg Val Leu Asp Leu Ala Cys Gln Ala Gln Ala Leu Ile Ala
                   85                  90                  95
   gaa agc ggt gcg caa ttg cag ggc agc gta gaa ctt gct cac cag cgg     336
   Glu Ser Gly Ala Gln Leu Gln Gly Ser Val Glu Leu Ala His Gln Arg
               100                 105                 110
   gtt acg ccg ctc ggc act ctg cgc tct acc tgg cta agt tcg cca gaa     384
   Val Thr Pro Leu Gly Thr Leu Arg Ser Thr Trp Leu Ser Ser Pro Glu
           115                 120                 125
   gtc ccc gtc tgg ccg ctg ccc gcg aag aaa ata tgt gta gtg gga att     432
   Val Pro Val Trp Pro Leu Pro Ala Lys Lys Ile Cys Val Val Gly Ile
       130                 135                 140
   agc ggc ctg atg gat ttt cag gcg cac ctt gcg gca gct tcg ttg cgt     480
   Ser Gly Leu Met Asp Phe Gln Ala His Leu Ala Ala Ala Ser Leu Arg
   145                 150                 155                 160
   gaa ctc ggc ctt gcc gtt gaa acc gca gaa ata gag ctg ccg gaa ctg     528
   Glu Leu Gly Leu Ala Val Glu Thr Ala Glu Ile Glu Leu Pro Glu Leu
                   165                 170                 175
   gat gtg ctg cgc aat aac gcc acc gaa ttt cgc gcg gtg aat atc gcc     576
   Asp Val Leu Arg Asn Asn Ala Thr Glu Phe Arg Ala Val Asn Ile Ala
               180                 185                 190
   cgt ttc ctt gat aat gaa gaa aac tgg ccg ctg tta ctt gat gcg ctt     624
   Arg Phe Leu Asp Asn Glu Glu Asn Trp Pro Leu Leu Leu Asp Ala Leu
           195                 200                 205
   att cct gtc gcc aat acc tgc gaa atg atc ctg atg ccc gcc tgc ttc         672
   Ile Pro Val Ala Asn Thr Cys Glu Met Ile Leu Met Pro Ala Cys Phe
       210                 215                 220
   ggt ctg gcc gat gac aaa ctg tgg cgt tgg ttg aat gaa aaa cta cct         720
   Gly Leu Ala Asp Asp Lys Leu Trp Arg Trp Leu Asn Glu Lys Leu Pro
   225                 230                 235                 240
   tgt tca ctg atg ctt ttg cca acg ctg ccg cct tcc gtg ctg ggc att         768
   Cys Ser Leu Met Leu Leu Pro Thr Leu Pro Pro Ser Val Leu Gly Ile
                   245                 250                 255
   cgt ctg caa aac cag tta cag cgc cag ttt gtg cgc cag ggt ggc gtg         816
   Arg Leu Gln Asn Gln Leu Gln Arg Gln Phe Val Arg Gln Gly Gly Val
               260                 265                 270
   tgg atg ccg ggc gat gaa gtg aaa aaa gtg acc tgt aaa aat ggc gta         864
   Trp Met Pro Gly Asp Glu Val Lys Lys Val Thr Cys Lys Asn Gly Val
           275                 280                 285
   gtg aac gaa atc tgg acc cgc aat cac gcc gat att ccg cta cgt cca         912
   Val Asn Glu Ile Trp Thr Arg Asn His Ala Asp Ile Pro Leu Arg Pro
       290                 295                 300
   cgt ttc gcg gtt ctc gcc agc ggc agt ttc ttt agt ggc gga ctg gta         960
   Arg Phe Ala Val Leu Ala Ser Gly Ser Phe Phe Ser Gly Gly Leu Val
   305                 310                 315                 320
   gcg gaa cgt aac ggc att cga gag ccg att ctc ggc ctt gat gtg cta        1008
   Ala Glu Arg Asn Gly Ile Arg Glu Pro Ile Leu Gly Leu Asp Val Leu
                   325                 330                 335
   caa acc gcc acg cgg ggt gaa tgg tat aag gga gat ttt ttt gcg ccg        1056
   Gln Thr Ala Thr Arg Gly Glu Trp Tyr Lys Gly Asp Phe Phe Ala Pro
               340                 345                 350
   caa ccg tgg cag cag ttc ggt gta acc act gat gag acg cta cgc ccg        1104
   Gln Pro Trp Gln Gln Phe Gly Val Thr Thr Asp Glu Thr Leu Arg Pro
           355                 360                 365
   tca cag gca ggg caa acc att gaa aac ctg ttt gcc atc ggt tcg gtg        1152
   Ser Gln Ala Gly Gln Thr Ile Glu Asn Leu Phe Ala Ile Gly Ser Val
       370                 375                 380
   ctg ggc gga ttt gat ccc atc gcc cag gga tgc ggc ggc ggt gtt tgt        1200
   Leu Gly Gly Phe Asp Pro Ile Ala Gln Gly Cys Gly Gly Gly Val Cys
   385                 390                 395                 400
   gcc gtc agt gct tta cat gcc gct caa cag att gcc caa cgc gca gga        1248
   Ala Val Ser Ala Leu His Ala Ala Gln Gln Ile Ala Gln Arg Ala Gly
                   405                 410                 415
   ggc caa caa tga                                                        1260
   Gly Gln Gln
<210> 4
   <211> 419
   <212> PRT
   <213> Escherichia coli
<400> 4
   Met Arg Phe Asp Thr Val Ile Met Gly Gly Gly Leu Ala Gly Leu Leu
   1               5                   10                  15
   Cys Gly Leu Gln Leu Gln Lys His Gly Leu Arg Cys Ala Ile Val Thr
               20                  25                  30
   Arg Gly Gln Ser Ala Leu His Phe Ser Ser Gly Ser Leu Asp Leu Leu
           35                  40                  45
   Ser His Leu Pro Asp Gly Gln Pro Val Thr Asp Ile His Ser Gly Leu
       50                  55                  60
   Glu Ser Leu Arg Gln Gln Ala Pro Ala His Pro Tyr Ser Leu Leu Glu
   65                  70                  75                  80
   Pro Gln Arg Val Leu Asp Leu Ala Cys Gln Ala Gln Ala Leu Ile Ala
                   85                  90                  95
   Glu Ser Gly Ala Gln Leu Gln Gly Ser Val Glu Leu Ala His Gln Arg
               100                 105                 110
   Val Thr Pro Leu Gly Thr Leu Arg Ser Thr Trp Leu Ser Ser Pro Glu
           115                 120                 125
   Val Pro Val Trp Pro Leu Pro Ala Lys Lys Ile Cys Val Val Gly Ile
       130                 135                 140
   Ser Gly Leu Met Asp Phe Gln Ala His Leu Ala Ala Ala Ser Leu Arg
   145                 150                 155                 160
   Glu Leu Gly Leu Ala Val Glu Thr Ala Glu Ile Glu Leu Pro Glu Leu
                   165                 170                 175
   Asp Val Leu Arg Asn Asn Ala Thr Glu Phe Arg Ala Val Asn Ile Ala
               180                 185                 190
   Arg Phe Leu Asp Asn Glu Glu Asn Trp Pro Leu Leu Leu Asp Ala Leu
           195                 200                 205
   Ile Pro Val Ala Asn Thr Cys Glu Met Ile Leu Met Pro Ala Cys Phe
       210                 215                 220
   Gly Leu Ala Asp Asp Lys Leu Trp Arg Trp Leu Asn Glu Lys Leu Pro
   225                 230                 235                 240
   Cys Ser Leu Met Leu Leu Pro Thr Leu Pro Pro Ser Val Leu Gly Ile
                   245                 250                 255
   Arg Leu Gln Asn Gln Leu Gln Arg Gln Phe Val Arg Gln Gly Gly Val
               260                 265                 270
   Trp Met Pro Gly Asp Glu Val Lys Lys Val Thr Cys Lys Asn Gly Val
           275                 280                 285
   Val Asn Glu Ile Trp Thr Arg Asn His Ala Asp Ile Pro Leu Arg Pro
       290                 295                 300
   Arg Phe Ala Val Leu Ala Ser Gly Ser Phe Phe Ser Gly Gly Leu Val
   305                 310                 315                 320
   Ala Glu Arg Asn Gly Ile Arg Glu Pro Ile Leu Gly Leu Asp Val Leu
                   325                 330                 335
   Gln Thr Ala Thr Arg Gly Glu Trp Tyr Lys Gly Asp Phe Phe Ala Pro
               340                 345                 350
   Gln Pro Trp Gln Gln Phe Gly Val Thr Thr Asp Glu Thr Leu Arg Pro
           355                 360                 365
   Ser Gln Ala Gly Gln Thr Ile Glu Asn Leu Phe Ala Ile Gly Ser Val
       370                 375                 380
   Leu Gly Gly Phe Asp Pro Ile Ala Gln Gly Cys Gly Gly Gly Val Cys
   385                 390                 395                 400
   Ala Val Ser Ala Leu His Ala Ala Gln Gln Ile Ala Gln Arg Ala Gly
                   405                 410                 415
   Gly Gln Gln
<210> 5
   <211> 1191
   <212> DNA
   <213> Escherichia coli
<220>
   <221> CDS
   <222> (1)..(1191)
   <223> glpC Kodierregion
<400> 5
   atg aat gac acc agc ttc gaa aac tgc att aag tgc acc gtc tgc acc      48
   Met Asn Asp Thr Ser Phe Glu Asn Cys Ile Lys Cys Thr Val Cys Thr
   1               5                   10                  15
   acc gcc tgc ccg gtg agc cgg gtg aat ccc ggt tat cca ggg cca aaa      96
   Thr Ala Cys Pro Val Ser Arg Val Asn Pro Gly Tyr Pro Gly Pro Lys
               20                  25                  30
   caa gcc ggg ccg gat ggc gag cgt ctg cgt ttg aaa gat ggc gca ctg     144
   Gln Ala Gly Pro Asp Gly Glu Arg Leu Arg Leu Lys Asp Gly Ala Leu
           35                  40                  45
   tat gac gag gcg ctg aaa tat tgc atc aac tgc aaa cgt tgt gaa gtc     192
   Tyr Asp Glu Ala Leu Lys Tyr Cys Ile Asn Cys Lys Arg Cys Glu Val
       50                  55                  60
   gcc tgc ccg tcc gat gtg aag att ggc gat att atc cag cgc gcg cgg     240
   Ala Cys Pro Ser Asp Val Lys Ile Gly Asp Ile Ile Gln Arg Ala Arg
   65                  70                  75                  80
   gcg aaa tat gac acc acg cgc ccg tcg ctg cgt aat ttt gtg ttg agt     288
   Ala Lys Tyr Asp Thr Thr Arg Pro Ser Leu Arg Asn Phe Val Leu Ser
                   85                  90                  95
   cat acc gac ctg atg ggt agc gtt tcc acg ccg ttc gca cca atc gtc     336
   His Thr Asp Leu Met Gly Ser Val Ser Thr Pro Phe Ala Pro Ile Val
               100                 105                 110
   aac acc gct acc tcg ctg aaa ccg gtg cgg cag ctg ctt gat gcg gcg     384
   Asn Thr Ala Thr Ser Leu Lys Pro Val Arg Gln Leu Leu Asp Ala Ala
           115                 120                 125
   tta aaa atc gat cat cgc cgc acg cta ccg aaa tac tcc ttc ggc acg     432
   Leu Lys Ile Asp His Arg Arg Thr Leu Pro Lys Tyr Ser Phe Gly Thr
       130                 135                 140
   ttc cgt cgc tgg tat cgc agc gtg gcg gct cag caa gca caa tat aaa     480
   Phe Arg Arg Trp Tyr Arg Ser Val Ala Ala Gln Gln Ala Gln Tyr Lys
   145                 150                 155                 160
   gac cag gtc gct ttc ttt cac ggc tgc ttc gtt aac tac aac cat ccg     528
   Asp Gln Val Ala Phe Phe His Gly Cys Phe Val Asn Tyr Asn His Pro
                   165                 170                 175
   cag tta ggt aaa gat tta att aaa gtg ctc aac gca atg ggt acc ggt     576
   Gln Leu Gly Lys Asp Leu Ile Lys Val Leu Asn Ala Met Gly Thr Gly
               180                 185                 190
   gta caa ctg ctc agc aaa gaa aaa tgc tgc ggc gta ccg cta atc gcc     624
   Val Gln Leu Leu Ser Lys Glu Lys Cys Cys Gly Val Pro Leu Ile Ala
           195                 200                 205
   aac ggc ttt acc gat aaa gca cgc aaa cag gca att acg aat gta gag     672
   Asn Gly Phe Thr Asp Lys Ala Arg Lys Gln Ala Ile Thr Asn Val Glu
       210                 215                 220
   tcg atc cgc gaa gct gtg gga gta aaa ggc att ccg gtg att gcc acc     720
   Ser Ile Arg Glu Ala Val Gly Val Lys Gly Ile Pro Val Ile Ala Thr
   225                 230                 235                 240
   tcc tca acc tgt aca ttt gcc ctg cgc gac gaa tac ccg gaa gtg ctg     768
   Ser Ser Thr Cys Thr Phe Ala Leu Arg Asp Glu Tyr Pro Glu Val Leu
                   245                 250                 255
   aat gtc gac aac aaa ggc ttg cgc gat cat atc gaa ctg gca acc cgc      816
   Asn Val Asp Asn Lys Gly Leu Arg Asp His Ile Glu Leu Ala Thr Arg
               260                 265                 270
   tgg ctg tgg cgc aag ctg gac gaa ggc aaa acg tta ccg ctg aaa ccg      864
   Trp Leu Trp Arg Lys Leu Asp Glu Gly Lys Thr Leu Pro Leu Lys Pro
           275                 280                 285
   ctg ccg ctg aaa gtg gtt tat cac act ccg tgc cat atg gaa aaa atg      912
   Leu Pro Leu Lys Val Val Tyr His Thr Pro Cys His Met Glu Lys Met
       290                 295                 300
   ggc tgg acg ctc tac acc ctg gag ctg ttg cgt aac atc ccg ggg ctt      960
   Gly Trp Thr Leu Tyr Thr Leu Glu Leu Leu Arg Asn Ile Pro Gly Leu
   305                 310                 315                 320
   gag tta acg gtg ctg gat tcc cag tgc tgc ggt att gcg ggt act tac     1008
   Glu Leu Thr Val Leu Asp Ser Gln Cys Cys Gly Ile Ala Gly Thr Tyr
                   325                 330                 335
   ggt ttc aaa aaa gag aac tac ccc acc tca caa gcc atc ggc gca cca     1056
   Gly Phe Lys Lys Glu Asn Tyr Pro Thr Ser Gln Ala Ile Gly Ala Pro
               340                 345                 350
   ctg ttc cgc cag ata gaa gaa agc ggc gca gat ctg gtg gtc acc gac     1104
   Leu Phe Arg Gln Ile Glu Glu Ser Gly Ala Asp Leu Val Val Thr Asp
           355                 360                 365
   tgc gaa acc tgt aaa tgg cag att gag atg tcc aca agt ctt cgc tgc     1152
   Cys Glu Thr Cys Lys Trp Gln Ile Glu Met Ser Thr Ser Leu Arg Cys
       370                 375                 380
   gaa cat ccg att acg cta ctg gcc cag gcg ctg gct taa                 1191
   Glu His Pro Ile Thr Leu Leu Ala Gln Ala Leu Ala
   385                 390                 395
<210> 6
   <211> 396
   <212> PRT
   <213> Escherichia coli
<400> 6
   Met Asn Asp Thr Ser Phe Glu Asn Cys Ile Lys Cys Thr Val Cys Thr
   1               5                   10                  15
   Thr Ala Cys Pro Val Ser Arg Val Asn Pro Gly Tyr Pro Gly Pro Lys
               20                  25                  30
   Gln Ala Gly Pro Asp Gly Glu Arg Leu Arg Leu Lys Asp Gly Ala Leu
           35                  40                  45
   Tyr Asp Glu Ala Leu Lys Tyr Cys Ile Asn Cys Lys Arg Cys Glu Val
       50                  55                  60
   Ala Cys Pro Ser Asp Val Lys Ile Gly Asp Ile Ile Gln Arg Ala Arg
   65                  70                  75                  80
   Ala Lys Tyr Asp Thr Thr Arg Pro Ser Leu Arg Asn Phe Val Leu Ser
                   85                  90                  95
   His Thr Asp Leu Met Gly Ser Val Ser Thr Pro Phe Ala Pro Ile Val
               100                 105                 110
   Asn Thr Ala Thr Ser Leu Lys Pro Val Arg Gln Leu Leu Asp Ala Ala
           115                 120                 125
   Leu Lys Ile Asp His Arg Arg Thr Leu Pro Lys Tyr Ser Phe Gly Thr
       130                 135                 140
   Phe Arg Arg Trp Tyr Arg Ser Val Ala Ala Gln Gln Ala Gln Tyr Lys
   145                 150                 155                 160
   Asp Gln Val Ala Phe Phe His Gly Cys Phe Val Asn Tyr Asn His Pro
                   165                 170                 175
   Gln Leu Gly Lys Asp Leu Ile Lys Val Leu Asn Ala Met Gly Thr Gly
               180                 185                 190
   Val Gln Leu Leu Ser Lys Glu Lys Cys Cys Gly Val Pro Leu Ile Ala
           195                 200                 205
   Asn Gly Phe Thr Asp Lys Ala Arg Lys Gln Ala Ile Thr Asn Val Glu
       210                 215                 220
   Ser Ile Arg Glu Ala Val Gly Val Lys Gly Ile Pro Val Ile Ala Thr
   225                 230                 235                 240
   Ser Ser Thr Cys Thr Phe Ala Leu Arg Asp Glu Tyr Pro Glu Val Leu
                   245                 250                 255
   Asn Val Asp Asn Lys Gly Leu Arg Asp His Ile Glu Leu Ala Thr Arg
               260                 265                 270
   Trp Leu Trp Arg Lys Leu Asp Glu Gly Lys Thr Leu Pro Leu Lys Pro
           275                 280                 285
   Leu Pro Leu Lys Val Val Tyr His Thr Pro Cys His Met Glu Lys Met
       290                 295                 300
   Gly Trp Thr Leu Tyr Thr Leu Glu Leu Leu Arg Asn Ile Pro Gly Leu
   305                 310                 315                 320
   Glu Leu Thr Val Leu Asp Ser Gln Cys Cys Gly Ile Ala Gly Thr Tyr
                   325                 330                 335
   Gly Phe Lys Lys Glu Asn Tyr Pro Thr Ser Gln Ala Ile Gly Ala Pro
               340                 345                 350
   Leu Phe Arg Gln Ile Glu Glu Ser Gly Ala Asp Leu Val Val Thr Asp
           355                 360                 365
   Cys Glu Thr Cys Lys Trp Gln Ile Glu Met Ser Thr Ser Leu Arg Cys
       370                 375                 380
   Glu His Pro Ile Thr Leu Leu Ala Gln Ala Leu Ala
   385                 390                 395
<210> 7
   <211> 1506
   <212> DNA
   <213> Escherichia coli
<220>
   <221> CDS
   <222> (1)..(1506)
   <223> glpD Kodierregion
<400> 7
   atg gaa acc aaa gat ctg att gtg ata ggg ggc ggc atc aat ggt gct       48
   Met Glu Thr Lys Asp Leu Ile Val Ile Gly Gly Gly Ile Asn Gly Ala
   1               5                   10                  15
   ggt atc gcg gca gac gcc gct gga cgc ggt tta tcc gtg ctg atg ctg       96
   Gly Ile Ala Ala Asp Ala Ala Gly Arg Gly Leu Ser Val Leu Met Leu
               20                  25                  30
   gag gcg cag gat ctc gct tgc gcg acc tct tcc gcc agt tca aaa ctc      144
   Glu Ala Gln Asp Leu Ala Cys Ala Thr Ser Ser Ala Ser Ser Lys Leu
           35                  40                  45
   att cac ggt ggc ctg cgc tac ctt gag cac tat gaa ttc cgc ctg gtc      192
   Ile His Gly Gly Leu Arg Tyr Leu Glu His Tyr Glu Phe Arg Leu Val
       50                  55                  60
   agc gag gcg ctg gct gaa cgt gaa gtg ctg ctg aaa atg gcc ccg cat      240
   Ser Glu Ala Leu Ala Glu Arg Glu Val Leu Leu Lys Met Ala Pro His
   65                  70                  75                  80
   atc gcc ttc ccg atg cgt ttt cgc ctg cca cat cgt ccg cat ctg cgc      288
   Ile Ala Phe Pro Met Arg Phe Arg Leu Pro His Arg Pro His Leu Arg
                   85                  90                  95
   ccg gcg tgg atg att cgc att ggt ctg ttt atg tac gat cat ctg ggt      336
   Pro Ala Trp Met Ile Arg Ile Gly Leu Phe Met Tyr Asp His Leu Gly
               100                 105                 110
   aaa cgc acc agc ttg ccg gga tca act ggt ttg cgt ttt ggc gca aat      384
   Lys Arg Thr Ser Leu Pro Gly Ser Thr Gly Leu Arg Phe Gly Ala Asn
           115                 120                 125
   tca gtg tta aaa ccg gaa att aag cgc gga ttc gaa tat tct gac tgt      432
   Ser Val Leu Lys Pro Glu Ile Lys Arg Gly Phe Glu Tyr Ser Asp Cys
       130                 135                 140
   tgg gta gac gac gcc cgt ctg gta ctc gcc aac gcc cag atg gtg gtg      480
   Trp Val Asp Asp Ala Arg Leu Val Leu Ala Asn Ala Gln Met Val Val
   145                 150                 155                 160
   cgt aaa ggc ggc gaa gtg ctt act cgg act cgc gcc acc tct gct cgc      528
   Arg Lys Gly Gly Glu Val Leu Thr Arg Thr Arg Ala Thr Ser Ala Arg
                   165                 170                 175
   cgc gaa aac ggc ctg tgg att gtg gaa gcg gaa gat atc gat acc ggc      576
   Arg Glu Asn Gly Leu Trp Ile Val Glu Ala Glu Asp Ile Asp Thr Gly
               180                 185                 190
   aaa aaa tat agc tgg caa gcg cgc ggc ttg gtt aac gcc acc ggc ccg      624
   Lys Lys Tyr Ser Trp Gln Ala Arg Gly Leu Val Asn Ala Thr Gly Pro
           195                 200                 205
   tgg gtg aaa cag ttc ttc gac gac ggg atg cat ctg cct tcg cct tat      672
   Trp Val Lys Gln Phe Phe Asp Asp Gly Met His Leu Pro Ser Pro Tyr
       210                 215                 220
   ggc att cgc ctg atc aaa ggc agc cat att gtg gtg ccg cgc gtg cat      720
   Gly Ile Arg Leu Ile Lys Gly Ser His Ile Val Val Pro Arg Val His
   225                 230                 235                 240
   acc cag aag caa gcc tac att ctg caa aac gaa gat aaa cgt att gtg      768
   Thr Gln Lys Gln Ala Tyr Ile Leu Gln Asn Glu Asp Lys Arg Ile Val
                   245                 250                 255
   ttc gtg atc ccg tgg atg gac gag ttt tcc atc atc ggc act acc gat      816
   Phe Val Ile Pro Trp Met Asp Glu Phe Ser Ile Ile Gly Thr Thr Asp
               260                 265                 270
   gtc gag tac aaa ggc gat ccg aaa gcg gtg aag att gaa gag agt gaa      864
   Val Glu Tyr Lys Gly Asp Pro Lys Ala Val Lys Ile Glu Glu Ser Glu
           275                 280                 285
   atc aat tac ctg ctg aat gtg tat aac acg cac ttt aaa aag cag tta      912
   Ile Asn Tyr Leu Leu Asn Val Tyr Asn Thr His Phe Lys Lys Gln Leu
       290                 295                 300
   agc cgt gac gat atc gtc tgg acc tac tcc ggt gtg cgt ccg ctg tgt      960
   Ser Arg Asp Asp Ile Val Trp Thr Tyr Ser Gly Val Arg Pro Leu Cys
   305                 310                 315                 320
   gat gat gag tcc gac tcg ccg cag gct att acc cgt gat tac acc ctt     1008
   Asp Asp Glu Ser Asp Ser Pro Gln Ala Ile Thr Arg Asp Tyr Thr Leu
                   325                 330                 335
   gat att cat gat gaa aat ggc aaa gca ccg ctg ctg tcg gta ttc ggc     1056
   Asp Ile His Asp Glu Asn Gly Lys Ala Pro Leu Leu Ser Val Phe Gly
               340                 345                 350
   ggt aag ctg acc acc tac cga aaa ctg gcg gaa cat gcg ctg gaa aaa     1104
   Gly Lys Leu Thr Thr Tyr Arg Lys Leu Ala Glu His Ala Leu Glu Lys
           355                 360                 365
   cta acg ccg tat tat cag ggt att ggc ccg gca tgg acg aaa gag agt      1152
   Leu Thr Pro Tyr Tyr Gln Gly Ile Gly Pro Ala Trp Thr Lys Glu Ser
       370                 375                 380
   gtg cta ccg ggt ggc gcc att gaa ggc gac cgc gac gat tat gcc gct      1200
   Val Leu Pro Gly Gly Ala Ile Glu Gly Asp Arg Asp Asp Tyr Ala Ala
   385                 390                 395                 400
   cgc ctg cgc cgc cgc tat ccg ttc ctg act gaa tcg ctg gcg cgt cat      1248
   Arg Leu Arg Arg Arg Tyr Pro Phe Leu Thr Glu Ser Leu Ala Arg His
                   405                 410                 415
   tac gct cgc act tac ggc agc aac agc gag ctg ctg ctc ggc aat gcg      1296
   Tyr Ala Arg Thr Tyr Gly Ser Asn Ser Glu Leu Leu Leu Gly Asn Ala
               420                 425                 430
   gga acg gta agc gat ctc ggg gaa gat ttc ggt cat gag ttc tac gaa      1344
   Gly Thr Val Ser Asp Leu Gly Glu Asp Phe Gly His Glu Phe Tyr Glu
           435                 440                 445
   gcg gag ctg aaa tac ctg gtg gat cac gaa tgg gtc cgc cgc gcc gac      1392
   Ala Glu Leu Lys Tyr Leu Val Asp His Glu Trp Val Arg Arg Ala Asp
       450                 455                 460
   gac gcc ctg tgg cgt cgc aca aaa caa ggc atg tgg cta aat gcg gat      1440
   Asp Ala Leu Trp Arg Arg Thr Lys Gln Gly Met Trp Leu Asn Ala Asp
   465                 470                 475                 480
   caa caa tct cgt gtg agt cag tgg ctg gtg gag tat acg cag cag agg      1488
   Gln Gln Ser Arg Val Ser Gln Trp Leu Val Glu Tyr Thr Gln Gln Arg
                   485                 490                 495
   tta tcg ctg gcg tcg taa                                              1506
   Leu Ser Leu Ala Ser
               500
<210> 8
   <211> 501
   <212> PRT
   <213> Escherichia coli
<400> 8
   Met Glu Thr Lys Asp Leu Ile Val Ile Gly Gly Gly Ile Asn Gly Ala
   1               5                   10                  15
   Gly Ile Ala Ala Asp Ala Ala Gly Arg Gly Leu Ser Val Leu Met Leu
               20                  25                  30
   Glu Ala Gln Asp Leu Ala Cys Ala Thr Ser Ser Ala Ser Ser Lys Leu
           35                  40                  45
   Ile His Gly Gly Leu Arg Tyr Leu Glu His Tyr Glu Phe Arg Leu Val
       50                  55                  60
   Ser Glu Ala Leu Ala Glu Arg Glu Val Leu Leu Lys Met Ala Pro His
   65                  70                  75                  80
   Ile Ala Phe Pro Met Arg Phe Arg Leu Pro His Arg Pro His Leu Arg
                   85                  90                  95
   Pro Ala Trp Met Ile Arg Ile Gly Leu Phe Met Tyr Asp His Leu Gly
               100                 105                 110
   Lys Arg Thr Ser Leu Pro Gly Ser Thr Gly Leu Arg Phe Gly Ala Asn
           115                 120                 125
   Ser Val Leu Lys Pro Glu Ile Lys Arg Gly Phe Glu Tyr Ser Asp Cys
       130                 135                 140
   Trp Val Asp Asp Ala Arg Leu Val Leu Ala Asn Ala Gln Met Val Val
   145                 150                 155                 160
   Arg Lys Gly Gly Glu Val Leu Thr Arg Thr Arg Ala Thr Ser Ala Arg
                   165                 170                 175
   Arg Glu Asn Gly Leu Trp Ile Val Glu Ala Glu Asp Ile Asp Thr Gly
               180                 185                 190
   Lys Lys Tyr Ser Trp Gln Ala Arg Gly Leu Val Asn Ala Thr Gly Pro
           195                 200                 205
   Trp Val Lys Gln Phe Phe Asp Asp Gly Met His Leu Pro Ser Pro Tyr
       210                 215                 220
   Gly Ile Arg Leu Ile Lys Gly Ser His Ile Val Val Pro Arg Val His
   225                 230                 235                 240
   Thr Gln Lys Gln Ala Tyr Ile Leu Gln Asn Glu Asp Lys Arg Ile Val
                   245                 250                 255
   Phe Val Ile Pro Trp Met Asp Glu Phe Ser Ile Ile Gly Thr Thr Asp
               260                 265                 270
   Val Glu Tyr Lys Gly Asp Pro Lys Ala Val Lys Ile Glu Glu Ser Glu
           275                 280                 285
   Ile Asn Tyr Leu Leu Asn Val Tyr Asn Thr His Phe Lys Lys Gln Leu
       290                 295                 300
   Ser Arg Asp Asp Ile Val Trp Thr Tyr Ser Gly Val Arg Pro Leu Cys
   305                 310                 315                 320
   Asp Asp Glu Ser Asp Ser Pro Gln Ala Ile Thr Arg Asp Tyr Thr Leu
                   325                 330                 335
   Asp Ile His Asp Glu Asn Gly Lys Ala Pro Leu Leu Ser Val Phe Gly
               340                 345                 350
   Gly Lys Leu Thr Thr Tyr Arg Lys Leu Ala Glu His Ala Leu Glu Lys
           355                 360                 365
   Leu Thr Pro Tyr Tyr Gln Gly Ile Gly Pro Ala Trp Thr Lys Glu Ser
       370                 375                 380
   Val Leu Pro Gly Gly Ala Ile Glu Gly Asp Arg Asp Asp Tyr Ala Ala
   385                 390                 395                 400
   Arg Leu Arg Arg Arg Tyr Pro Phe Leu Thr Glu Ser Leu Ala Arg His
                   405                 410                 415
   Tyr Ala Arg Thr Tyr Gly Ser Asn Ser Glu Leu Leu Leu Gly Asn Ala
               420                 425                 430
   Gly Thr Val Ser Asp Leu Gly Glu Asp Phe Gly His Glu Phe Tyr Glu
           435                 440                 445
   Ala Glu Leu Lys Tyr Leu Val Asp His Glu Trp Val Arg Arg Ala Asp
       450                 455                 460
   Asp Ala Leu Trp Arg Arg Thr Lys Gln Gly Met Trp Leu Asn Ala Asp
   465                 470                 475                 480
   Gln Gln Ser Arg Val Ser Gln Trp Leu Val Glu Tyr Thr Gln Gln Arg
                   485                 490                 495
   Leu Ser Leu Ala Ser
               500
<210> 9
   <211> 327
   <212> DNA
   <213> Escherichia coli
<220>
   <221> CDS
   <222> (1)..(327)
   <223> glpE Kodierregion
<210> 10
   <211> 108
   <212> PRT
   <213> Escherichia coli
<400> 10
<210> 11
   <211> 846
   <212> DNA
   <213> Escherichia coli
<220>
   <221> CDS
   <222> (1)..(846)
   <223> glpF Kodierregion
<400> 11
   atg agt caa aca tca acc ttg aaa ggc cag tgc att gct gaa ttc ctc        48
   Met Ser Gln Thr Ser Thr Leu Lys Gly Gln Cys Ile Ala Glu Phe Leu
   1               5                   10                  15
   ggt acc ggg ttg ttg att ttc ttc ggt gtg ggt tgc gtt gca gca cta        96
   Gly Thr Gly Leu Leu Ile Phe Phe Gly Val Gly Cys Val Ala Ala Leu
               20                  25                  30
   aaa gtc gct ggt gcg tct ttt ggt cag tgg gaa atc agt gtc att tgg       144
   Lys Val Ala Gly Ala Ser Phe Gly Gln Trp Glu Ile Ser Val Ile Trp
           35                  40                  45
   gga ctg ggg gtg gca atg gcc atc tac ctg acc gca ggg gtt tcc ggc       192
   Gly Leu Gly Val Ala Met Ala Ile Tyr Leu Thr Ala Gly Val Ser Gly
       50                  55                  60
   gcg cat ctt aat ccc gct gtt acc att gca ttg tgg ctg ttt gcc tgt       240
   Ala His Leu Asn Pro Ala Val Thr Ile Ala Leu Trp Leu Phe Ala Cys
   65                  70                  75                  80
   ttc gac aag cgc aaa gtt att cct ttt atc gtt tca caa gtt gcc ggc       288
   Phe Asp Lys Arg Lys Val Ile Pro Phe Ile Val Ser Gln Val Ala Gly
                   85                  90                  95
   gct ttc tgt gct gcg gct tta gtt tac ggg ctt tac tac aat tta ttt       336
   Ala Phe Cys Ala Ala Ala Leu Val Tyr Gly Leu Tyr Tyr Asn Leu Phe
               100                 105                 110
   ttc gac ttc gag cag act cat cac att gtt cgc ggc agc gtt gaa agt       384
   Phe Asp Phe Glu Gln Thr His His Ile Val Arg Gly Ser Val Glu Ser
           115                 120                 125
   gtt gat ctg gct ggc act ttc tct act tac cct aat cct cat atc aat       432
   Val Asp Leu Ala Gly Thr Phe Ser Thr Tyr Pro Asn Pro His Ile Asn
       130                 135                 140
   ttt gtg cag gct ttc gca gtt gag atg gtg att acc gct att ctg atg       480
   Phe Val Gln Ala Phe Ala Val Glu Met Val Ile Thr Ala Ile Leu Met
   145                 150                 155                 160
   ggg ctg atc ctg gcg tta acg gac gat ggc aac ggt gta cca cgc ggc       528
   Gly Leu Ile Leu Ala Leu Thr Asp Asp Gly Asn Gly Val Pro Arg Gly
                   165                 170                 175
   cct ttg gct ccc ttg ctg att ggt cta ctg att gcg gtc att ggc gca       576
   Pro Leu Ala Pro Leu Leu Ile Gly Leu Leu Ile Ala Val Ile Gly Ala
               180                 185                 190
   tct atg ggc cca ttg aca ggt ttt gcc atg aac cca gcg cgt gac ttc        624
   Ser Met Gly Pro Leu Thr Gly Phe Ala Met Asn Pro Ala Arg Asp Phe
           195                 200                 205
   ggt ccg aaa gtc ttt gcc tgg ctg gcg ggc tgg ggc aat gtc gcc ttt        672
   Gly Pro Lys Val Phe Ala Trp Leu Ala Gly Trp Gly Asn Val Ala Phe
       210                 215                 220
   acc ggc ggc aga gac att cct tac ttc ctg gtg ccg ctt ttc ggc cct        720
   Thr Gly Gly Arg Asp Ile Pro Tyr Phe Leu Val Pro Leu Phe Gly Pro
   225                 230                 235                 240
   atc gtt ggc gcg att gta ggt gca ttt gcc tac cgc aaa ctg att ggt        768
   Ile Val Gly Ala Ile Val Gly Ala Phe Ala Tyr Arg Lys Leu Ile Gly
                   245                 250                 255
   cgc cat ttg cct tgc gat atc tgt gtt gtg gaa gaa aag gaa acc aca        816
   Arg His Leu Pro Cys Asp Ile Cys Val Val Glu Glu Lys Glu Thr Thr
               260                 265                 270
   act cct tca gaa caa aaa gct tcg ctg taa                                846
   Thr Pro Ser Glu Gln Lys Ala Ser Leu
           275                 280
<210> 12
   <211> 281
   <212> PRT
   <213> Escherichia coli
<400> 12
   Met Ser Gln Thr Ser Thr Leu Lys Gly Gln Cys Ile Ala Glu Phe Leu
   1               5                   10                  15
   Gly Thr Gly Leu Leu Ile Phe Phe Gly Val Gly Cys Val Ala Ala Leu
               20                  25                  30
   Lys Val Ala Gly Ala Ser Phe Gly Gln Trp Glu Ile Ser Val Ile Trp
           35                  40                  45
   Gly Leu Gly Val Ala Met Ala Ile Tyr Leu Thr Ala Gly Val Ser Gly
       50                  55                  60
   Ala His Leu Asn Pro Ala Val Thr Ile Ala Leu Trp Leu Phe Ala Cys
   65                  70                  75                  80
   Phe Asp Lys Arg Lys Val Ile Pro Phe Ile Val Ser Gln Val Ala Gly
                   85                  90                  95
   Ala Phe Cys Ala Ala Ala Leu Val Tyr Gly Leu Tyr Tyr Asn Leu Phe
               100                 105                 110
   Phe Asp Phe Glu Gln Thr His His Ile Val Arg Gly Ser Val Glu Ser
                   115                 120                 125
   Val Asp Leu Ala Gly Thr Phe Ser Thr Tyr Pro Asn Pro His Ile Asn
       130                 135                 140
   Phe Val Gln Ala Phe Ala Val Glu Met Val Ile Thr Ala Ile Leu Met
   145                 150                 155                 160
   Gly Leu Ile Leu Ala Leu Thr Asp Asp Gly Asn Gly Val Pro Arg Gly
                   165                 170                 175
   Pro Leu Ala Pro Leu Leu Ile Gly Leu Leu Ile Ala Val Ile Gly Ala
               180                 185                 190
   Ser Met Gly Pro Leu Thr Gly Phe Ala Met Asn Pro Ala Arg Asp Phe
           195                 200                 205
   Gly Pro Lys Val Phe Ala Trp Leu Ala Gly Trp Gly Asn Val Ala Phe
       210                 215                 220
   Thr Gly Gly Arg Asp Ile Pro Tyr Phe Leu Val Pro Leu Phe Gly Pro
   225                 230                 235                 240
   Ile Val Gly Ala Ile Val Gly Ala Phe Ala Tyr Arg Lys Leu Ile Gly
                   245                 250                 255
   Arg His Leu Pro Cys Asp Ile Cys Val Val Glu Glu Lys Glu Thr Thr
               260                 265                 270
   Thr Pro Ser Glu Gln Lys Ala Ser Leu
           275                 280
<210> 13
   <211> 831
   <212> DNA
   <213> Escherichia coli
<220>
   <221> CDS
   <222> (1)..(831)
   <223> glpG Kodierregion
<400> 13
   atg ttg atg att acc tct ttt gct aac ccc cgc gtg gcg cag gcg ttt      48
   Met Leu Met Ile Thr Ser Phe Ala Asn Pro Arg Val Ala Gln Ala Phe
   1               5                   10                  15
   gtt gat tac atg gcg acg cag ggt gtt atc ctc acg att caa caa cat      96
   Val Asp Tyr Met Ala Thr Gln Gly Val Ile Leu Thr Ile Gln Gln His
               20                  25                  30
   aac caa agc gat gtc tgg ctg gcg gat gag tcc cag gcc gag cgc gta        144
   Asn Gln Ser Asp Val Trp Leu Ala Asp Glu Ser Gln Ala Glu Arg Val
           35                  40                  45
   cgg gcg gag ctg gcg cgt ttt ctc gaa aac ccg gca gat ccg cgt tat        192
   Arg Ala Glu Leu Ala Arg Phe Leu Glu Asn Pro Ala Asp Pro Arg Tyr
       50                  55                  60
   ctg gcg gcg agc tgg cag gca ggc cat acc ggc agt ggc ctg cat tat        240
   Leu Ala Ala Ser Trp Gln Ala Gly His Thr Gly Ser Gly Leu His Tyr
   65                  70                  75                  80
   cgc cgt tat cct ttc ttt gcc gcc ttg cgt gaa cgc gca ggt ccg gta        288
   Arg Arg Tyr Pro Phe Phe Ala Ala Leu Arg Glu Arg Ala Gly Pro Val
                   85                  90                  95
   acc tgg gtg atg atg atc gcc tgc gtg gtg gtg ttt att gcc atg caa        336
   Thr Trp Val Met Met Ile Ala Cys Val Val Val Phe Ile Ala Met Gln
               100                 105                 110
   att ctc ggc gat cag gaa gtg atg tta tgg ctg gcc tgg cca ttc gat        384
   Ile Leu Gly Asp Gln Glu Val Met Leu Trp Leu Ala Trp Pro Phe Asp
           115                 120                 125
   cca aca ctg aaa ttt gag ttc tgg cgt tac ttc acc cac gcg tta atg        432
   Pro Thr Leu Lys Phe Glu Phe Trp Arg Tyr Phe Thr His Ala Leu Met
       130                 135                 140
   cac ttc tcg ctg atg cat atc ctc ttt aac ctg ctc tgg tgg tgg tat        480
   His Phe Ser Leu Met His Ile Leu Phe Asn Leu Leu Trp Trp Trp Tyr
   145                 150                 155                 160
   ctc ggc ggt gcg gtg gaa aaa cgc ctc ggt agc ggt aag cta att gtc        528
   Leu Gly Gly Ala Val Glu Lys Arg Leu Gly Ser Gly Lys Leu Ile Val
                   165                 170                 175
   att acg ctt atc agc gcc ctg tta agc ggc tat gtg cag caa aaa ttc        576
   Ile Thr Leu Ile Ser Ala Leu Leu Ser Gly Tyr Val Gln Gln Lys Phe
               180                 185                 190
   agc ggg ccg tgg ttt ggc ggg ctt tct ggc gtg gtg tat gcg ctg atg        624
   Ser Gly Pro Trp Phe Gly Gly Leu Ser Gly Val Val Tyr Ala Leu Met
           195                 200                 205
   ggc tac gtc tgg cta cgt ggc gaa cgc gat ccg caa agt ggc att tac        672
   Gly Tyr Val Trp Leu Arg Gly Glu Arg Asp Pro Gln Ser Gly Ile Tyr
       210                 215                 220
   ctg caa cgt ggg tta att atc ttt gcg ctg atc tgg att gtc gcc gga        720
   Leu Gln Arg Gly Leu Ile Ile Phe Ala Leu Ile Trp Ile Val Ala Gly
   225                 230                 235                 240
   tgg ttt gat ttg ttt ggg atg tcg atg gcg aac gga gca cac atc gcc        768
   Trp Phe Asp Leu Phe Gly Met Ser Met Ala Asn Gly Ala His Ile Ala
                   245                 250                 255
   ggg tta gcc gtg ggt tta gcg atg gct ttt gtt gat tcg ctc aat gcg        816
   Gly Leu Ala Val Gly Leu Ala Met Ala Phe Val Asp Ser Leu Asn Ala
               260                 265                 270
   cga aaa cga aaa taa                                                    831
                       Arg Lys Arg Lys
                           275
<210> 14
   <211> 276
   <212> PRT
   <213> Escherichia coli
<400> 14
   Met Leu Met Ile Thr Ser Phe Ala Asn Pro Arg Val Ala Gln Ala Phe
   1               5                   10                  15
   Val Asp Tyr Met Ala Thr Gln Gly Val Ile Leu Thr Ile Gln Gln His
               20                  25                  30
   Asn Gln Ser Asp Val Trp Leu Ala Asp Glu Ser Gln Ala Glu Arg Val
           35                  40                  45
   Arg Ala Glu Leu Ala Arg Phe Leu Glu Asn Pro Ala Asp Pro Arg Tyr
       50                  55                  60
   Leu Ala Ala Ser Trp Gln Ala Gly His Thr Gly Ser Gly Leu His Tyr
   65                  70                  75                  80
   Arg Arg Tyr Pro Phe Phe Ala Ala Leu Arg Glu Arg Ala Gly Pro Val
                   85                  90                  95
   Thr Trp Val Met Met Ile Ala Cys Val Val Val Phe Ile Ala Met Gln
               100                 105                 110
   Ile Leu Gly Asp Gln Glu Val Met Leu Trp Leu Ala Trp Pro Phe Asp
           115                 120                 125
   Pro Thr Leu Lys Phe Glu Phe Trp Arg Tyr Phe Thr His Ala Leu Met
       130                 135                 140
   His Phe Ser Leu Met His Ile Leu Phe Asn Leu Leu Trp Trp Trp Tyr
   145                 150                 155                 160
   Leu Gly Gly Ala Val Glu Lys Arg Leu Gly Ser Gly Lys Leu Ile Val
                   165                 170                 175
   Ile Thr Leu Ile Ser Ala Leu Leu Ser Gly Tyr Val Gln Gln Lys Phe
               180                 185                 190
   Ser Gly Pro Trp Phe Gly Gly Leu Ser Gly Val Val Tyr Ala Leu Met
           195                 200                 205
   Gly Tyr Val Trp Leu Arg Gly Glu Arg Asp Pro Gln Ser Gly Ile Tyr
       210                 215                 220
   Leu Gln Arg Gly Leu Ile Ile Phe Ala Leu Ile Trp Ile Val Ala Gly
   225                 230                 235                 240
   Trp Phe Asp Leu Phe Gly Met Ser Met Ala Asn Gly Ala His Ile Ala
                   245                 250                 255
   Gly Leu Ala Val Gly Leu Ala Met Ala Phe Val Asp Ser Leu Asn Ala
               260                 265                 270
   Arg Lys Arg Lys
           275
<210> 15
   <211> 1509
   <212> DNA
   <213> Escherichia coli
<220>
   <221> CDS
   <222> (1)..(1509)
   <223> glpK Kodierregion
<400> 15
   atg act gaa aaa aaa tat atc gtt gcg ctc gac cag ggc acc acc agc        48
   Met Thr Glu Lys Lys Tyr Ile Val Ala Leu Asp Gln Gly Thr Thr Ser
   1               5                   10                  15
   tcc cgc gcg gtc gta atg gat cac gat gcc aat atc att agc gtg tcg        96
   Ser Arg Ala Val Val Met Asp His Asp Ala Asn Ile Ile Ser Val Ser
               20                  25                  30
   cag cgc gaa ttt gag caa atc tac cca aaa cca ggt tgg gta gaa cac       144
   Gln Arg Glu Phe Glu Gln Ile Tyr Pro Lys Pro Gly Trp Val Glu His
           35                  40                  45
   gac cca atg gaa atc tgg gcc acc caa agc tcc acg ctg gta gaa gtg       192
   Asp Pro Met Glu Ile Trp Ala Thr Gln Ser Ser Thr Leu Val Glu Val
       50                  55                  60
   ctg gcg aaa gcc gat atc agt tcc gat caa att gca gct atc ggt att       240
   Leu Ala Lys Ala Asp Ile Ser Ser Asp Gln Ile Ala Ala Ile Gly Ile
   65                  70                  75                  80
   acg aac cag cgt gaa acc act att gtc tgg gaa aaa gaa acc ggc aag       288
   Thr Asn Gln Arg Glu Thr Thr Ile Val Trp Glu Lys Glu Thr Gly Lys
                   85                  90                  95
   cct atc tat aac gcc att gtc tgg cag tgc cgt cgt acc gca gaa atc       336
   Pro Ile Tyr Asn Ala Ile Val Trp Gln Cys Arg Arg Thr Ala Glu Ile
               100                 105                 110
   tgc gag cat tta aaa cgt gac ggt tta gaa gat tat atc cgc agc aat       384
   Cys Glu His Leu Lys Arg Asp Gly Leu Glu Asp Tyr Ile Arg Ser Asn
           115                 120                 125
   acc ggt ctg gtg att gac ccg tac ttt tct ggc acc aaa gtg aag tgg       432
   Thr Gly Leu Val Ile Asp Pro Tyr Phe Ser Gly Thr Lys Val Lys Trp
       130                 135                 140
   atc ctc gac cat gtg gaa ggc tct cgc gag cgt gca cgt cgt ggt gaa       480
   Ile Leu Asp His Val Glu Gly Ser Arg Glu Arg Ala Arg Arg Gly Glu
   145                 150                 155                 160
   ttg ctg ttt ggt acg gtt gat acg tgg ctt atc tgg aaa atg act cag       528
   Leu Leu Phe Gly Thr Val Asp Thr Trp Leu Ile Trp Lys Met Thr Gln
                   165                 170                 175
   ggc cgt gtc cat gtg acc gat tac acc aac gcc tct cgt acc atg ttg       576
   Gly Arg Val His Val Thr Asp Tyr Thr Asn Ala Ser Arg Thr Met Leu
               180                 185                 190
   ttc aac atc cat acc ctg gac tgg gac gac aaa atg ctg gaa gtg ctg       624
   Phe Asn Ile His Thr Leu Asp Trp Asp Asp Lys Met Leu Glu Val Leu
           195                 200                 205
   gat att ccg cgc gag atg ctg cca gaa gtg cgt cgt tct tcc gaa gta       672
   Asp Ile Pro Arg Glu Met Leu Pro Glu Val Arg Arg Ser Ser Glu Val
       210                 215                 220
   tac ggt cag act aac att ggc ggc aaa ggc ggc acg cgt att cca atc       720
   Tyr Gly Gln Thr Asn Ile Gly Gly Lys Gly Gly Thr Arg Ile Pro Ile
   225                 230                 235                 240
   tcc ggg atc gcc ggt gac cag cag gcc gcg ctg ttt ggt cag ttg tgc       768
   Ser Gly Ile Ala Gly Asp Gln Gln Ala Ala Leu Phe Gly Gln Leu Cys
                   245                 250                 255
   gtg aaa gaa ggg atg gcg aag aac acc tat ggc act ggc tgc ttt atg       816
   Val Lys Glu Gly Met Ala Lys Asn Thr Tyr Gly Thr Gly Cys Phe Met
               260                 265                 270
   ctg atg aac act ggc gag aaa gcg gtg aaa tca gaa aac ggc ctg ctg       864
   Leu Met Asn Thr Gly Glu Lys Ala Val Lys Ser Glu Asn Gly Leu Leu
           275                 280                 285
   acc acc atc gcc tgc ggc ccg act ggc gaa gtg aac tat gcg ttg gaa       912
   Thr Thr Ile Ala Cys Gly Pro Thr Gly Glu Val Asn Tyr Ala Leu Glu
       290                 295                 300
   ggt gcg gtg ttt atg gca ggc gca tcc att cag tgg ctg cgc gat gaa       960
   Gly Ala Val Phe Met Ala Gly Ala Ser Ile Gln Trp Leu Arg Asp Glu
   305                 310                 315                 320
   atg aag ttg att aac gac gcc tac gat tcc gaa tat ttc gcc acc aaa      1008
   Met Lys Leu Ile Asn Asp Ala Tyr Asp Ser Glu Tyr Phe Ala Thr Lys
                   325                 330                 335
   gtg caa aac acc aat ggt gtg tat gtg gtt ccg gca ttt acc ggg ctg      1056
   Val Gln Asn Thr Asn Gly Val Tyr Val Val Pro Ala Phe Thr Gly Leu
               340                 345                 350
   ggt gcg ccg tac tgg gac ccg tat gcg cgc ggg gcg att ttc ggt ctg      1104
   Gly Ala Pro Tyr Trp Asp Pro Tyr Ala Arg Gly Ala Ile Phe Gly Leu
           355                 360                 365
   act cgt ggg gtg aac gct aac cac att ata cgc gcg acg ctg gag tct      1152
   Thr Arg Gly Val Asn Ala Asn His Ile Ile Arg Ala Thr Leu Glu Ser
       370                 375                 380
   att gct tat cag acg cgt gac gtg ctg gaa gcg atg cag gcc gac tct      1200
   Ile Ala Tyr Gln Thr Arg Asp Val Leu Glu Ala Met Gln Ala Asp Ser
   385                 390                 395                 400
   ggt atc cgt ctg cac gcc ctg cgc gtg gat ggt ggc gca gta gca aac      1248
   Gly Ile Arg Leu His Ala Leu Arg Val Asp Gly Gly Ala Val Ala Asn
                   405                 410                 415
   aat ttc ctg atg cag ttc cag tcc gat att ctc ggc acc cgc gtt gag      1296
   Asn Phe Leu Met Gln Phe Gln Ser Asp Ile Leu Gly Thr Arg Val Glu
               420                 425                 430
   cgc ccg gaa gtg cgc gaa gtc acc gca ttg ggt gcg gcc tat ctc gca      1344
   Arg Pro Glu Val Arg Glu Val Thr Ala Leu Gly Ala Ala Tyr Leu Ala
           435                 440                 445
   ggc ctg gcg gtt ggc ttc tgg cag aac ctc gac gag ctg caa gag aaa      1392
   Gly Leu Ala Val Gly Phe Trp Gln Asn Leu Asp Glu Leu Gln Glu Lys
       450                 455                 460
   gcg gtg att gag cgc gag ttc cgt cca ggc atc gaa acc act gag cgt      1440
   Ala Val Ile Glu Arg Glu Phe Arg Pro Gly Ile Glu Thr Thr Glu Arg
   465                 470                 475                 480
   aat tac cgt tac gca ggc tgg aaa aaa gcg gtt aaa cgc gcg atg gcg      1488
   Asn Tyr Arg Tyr Ala Gly Trp Lys Lys Ala Val Lys Arg Ala Met Ala
                   485                 490                 495
   tgg gaa gaa cac gac gaa taa                                          1509
   Trp Glu Glu His Asp Glu
               500
<210> 16
   <211> 502
   <212> PRT
   <213> Escherichia coli
<400> 16
   Met Thr Glu Lys Lys Tyr Ile Val Ala Leu Asp Gln Gly Thr Thr Ser
   1               5                   10                  15
   Ser Arg Ala Val Val Met Asp His Asp Ala Asn Ile Ile Ser Val Ser
               20                  25                  30
   Gln Arg Glu Phe Glu Gln Ile Tyr Pro Lys Pro Gly Trp Val Glu His
           35                  40                  45
   Asp Pro Met Glu Ile Trp Ala Thr Gln Ser Ser Thr Leu Val Glu Val
       50                  55                  60
   Leu Ala Lys Ala Asp Ile Ser Ser Asp Gln Ile Ala Ala Ile Gly Ile
   65                  70                  75                  80
   Thr Asn Gln Arg Glu Thr Thr Ile Val Trp Glu Lys Glu Thr Gly Lys
                   85                  90                  95
   Pro Ile Tyr Asn Ala Ile Val Trp Gln Cys Arg Arg Thr Ala Glu Ile
               100                 105                 110
   Cys Glu His Leu Lys Arg Asp Gly Leu Glu Asp Tyr Ile Arg Ser Asn
           115                 120                 125
   Thr Gly Leu Val Ile Asp Pro Tyr Phe Ser Gly Thr Lys Val Lys Trp
       130                 135                 140
   Ile Leu Asp His Val Glu Gly Ser Arg Glu Arg Ala Arg Arg Gly Glu
   145                 150                 155                 160
   Leu Leu Phe Gly Thr Val Asp Thr Trp Leu Ile Trp Lys Met Thr Gln
                   165                 170                 175
   Gly Arg Val His Val Thr Asp Tyr Thr Asn Ala Ser Arg Thr Met Leu
               180                 185                 190
   Phe Asn Ile His Thr Leu Asp Trp Asp Asp Lys Met Leu Glu Val Leu
           195                 200                 205
   Asp Ile Pro Arg Glu Met Leu Pro Glu Val Arg Arg Ser Ser Glu Val
       210                 215                 220
   Tyr Gly Gln Thr Asn Ile Gly Gly Lys Gly Gly Thr Arg Ile Pro Ile
   225                 230                 235                 240
   Ser Gly Ile Ala Gly Asp Gln Gln Ala Ala Leu Phe Gly Gln Leu Cys
                   245                 250                 255
   Val Lys Glu Gly Met Ala Lys Asn Thr Tyr Gly Thr Gly Cys Phe Met
               260                 265                 270
   Leu Met Asn Thr Gly Glu Lys Ala Val Lys Ser Glu Asn Gly Leu Leu
           275                 280                 285
   Thr Thr Ile Ala Cys Gly Pro Thr Gly Glu Val Asn Tyr Ala Leu Glu
       290                 295                 300
   Gly Ala Val Phe Met Ala Gly Ala Ser Ile Gln Trp Leu Arg Asp Glu
   305                 310                 315                 320
   Met Lys Leu Ile Asn Asp Ala Tyr Asp Ser Glu Tyr Phe Ala Thr Lys
   325 330 335
   Val Gln Asn Thr Asn Gly Val Tyr Val Val Pro Ala Phe Thr Gly Leu
   340 345 350
   Gly Ala Pro Tyr Trp Asp Pro Tyr Ala Arg Gly Ala Ile Phe Gly Leu
   355 360 365
   Thr Arg Gly Val Asn Ala Asn His Ile Ile Arg Ala Thr Leu Glu Ser
   370 375 380
   Ile Ala Tyr Gln Thr Arg Asp Val Leu Glu Ala Met Gln Ala Asp Ser
   385 390 395 400
   Gly Ile Arg Leu His Ala Leu Arg Val Asp Gly Gly Ala Val Ala Asn
   405 410 415
   Asn Phe Leu Met Gln Phe Gln Ser Asp Ile Leu Gly Thr Arg Val Glu
   420 425 430
   Arg Pro Glu Val Arg Glu Val Thr Ala Leu Gly Ala Ala Tyr Leu Ala
   435 440 445
   Gly Leu Ala Val Gly Phe Trp Gln Asn Leu Asp Glu Leu Gln Glu Lys
   450 455 460
   Ala Val Ile Glu Arg Glu Phe Arg Pro Gly Ile Glu Thr Thr Glu Arg
   465 470 475 480
   Asn Tyr Arg Tyr Ala Gly Trp Lys Lys Ala Val Lys Arg Ala Met Ala
   485 490 495
   Trp Glu Glu His Asp Glu
   500
<210> 17
   <211> 1077
   <212> DNA
   <213> Escherichia coli
<220>
   <221> CDS
   <222> (1)..(1077)
   <223> glpQ Kodierregion
<400> 17
   atg aaa ttg acg ctg aaa aac ctt agc atg gcg atc atg atg agc act 48
   Met Lys Leu Thr Leu Lys Asn Leu Ser Met Ala Ile Met Met Ser Thr
   1               5                   10                  15
   ata gtc atg gga agc agt gca atg gcg gcg gac agc aac gaa aaa ata        96
   Ile Val Met Gly Ser Ser Ala Met Ala Ala Asp Ser Asn Glu Lys Ile
               20                  25                  30
   gtc atc gcc cat cgc ggt gcc agt gga tat ttg ccg gag cat acg ctg       144
   Val Ile Ala His Arg Gly Ala Ser Gly Tyr Leu Pro Glu His Thr Leu
           35                  40                  45
   cca gca aaa gcg atg gcg tat gcg cag gga gcg gat tat ctg gaa cag       192
   Pro Ala Lys Ala Met Ala Tyr Ala Gln Gly Ala Asp Tyr Leu Glu Gln
       50                  55                  60
   gat ttg gtg atg acc aaa gac gac aat ctg gtt gtt ctg cat gac cat       240
   Asp Leu Val Met Thr Lys Asp Asp Asn Leu Val Val Leu His Asp His
   65                  70                  75                  80
   tac ctc gat cgt gtt act gat gtt gcc gat cgt ttc ccg gat cgg gcg       288
   Tyr Leu Asp Arg Val Thr Asp Val Ala Asp Arg Phe Pro Asp Arg Ala
                   85                  90                  95
   cgc aaa gac ggt cgt tac tac gcg ata gat ttc acg ctg gat gaa att       336
   Arg Lys Asp Gly Arg Tyr Tyr Ala Ile Asp Phe Thr Leu Asp Glu Ile
               100                 105                 110
   aag tcg ttg aaa ttt acc gaa ggt ttc gat att gaa aac ggt aaa aaa       384
   Lys Ser Leu Lys Phe Thr Glu Gly Phe Asp Ile Glu Asn Gly Lys Lys
           115                 120                 125
   gtg cag act tat ccg ggg cgt ttc cca atg ggt aag tcc gac ttc cgg       432
   Val Gln Thr Tyr Pro Gly Arg Phe Pro Met Gly Lys Ser Asp Phe Arg
       130                 135                 140
   gtg cac acc ttt gaa gaa gag att gaa ttt gtt cag ggg tta aat cac       480
   Val His Thr Phe Glu Glu Glu Ile Glu Phe Val Gln Gly Leu Asn His
   145                 150                 155                 160
   tct acc ggg aaa aat atc ggt att tat cca gaa atc aaa gcg ccg tgg       528
   Ser Thr Gly Lys Asn Ile Gly Ile Tyr Pro Glu Ile Lys Ala Pro Trp
                   165                 170                 175
   ttc cat cat cag gaa ggg aag gat att gcg gca aaa acg ctg gaa gtg       576
   Phe His His Gln Glu Gly Lys Asp Ile Ala Ala Lys Thr Leu Glu Val
               180                 185                 190
   ctg aag aaa tat ggt tac acc ggt aaa gac gat aaa gtt tat ttg caa       624
   Leu Lys Lys Tyr Gly Tyr Thr Gly Lys Asp Asp Lys Val Tyr Leu Gln
           195                 200                 205
   tgt ttt gat gct gat gag ctg aag cgt att aag aat gag ctg gaa ccc       672
   Cys Phe Asp Ala Asp Glu Leu Lys Arg Ile Lys Asn Glu Leu Glu Pro
       210                 215                 220
   aaa atg ggc atg gag ctc aat ctg gta cag ctg att gcc tat acc gac       720
   Lys Met Gly Met Glu Leu Asn Leu Val Gln Leu Ile Ala Tyr Thr Asp
   225                 230                 235                 240
   tgg aat gaa acg cag cag aaa cag ccg gat gga agc tgg gtt aat tac       768
   Trp Asn Glu Thr Gln Gln Lys Gln Pro Asp Gly Ser Trp Val Asn Tyr
                   245                 250                 255
   aac tac gac tgg atg ttt aag ccg ggt gcc atg aaa cag gtg gcg gaa      816
   Asn Tyr Asp Trp Met Phe Lys Pro Gly Ala Met Lys Gln Val Ala Glu
               260                 265                 270
   tat gca gat ggt att ggt ccg gat tac cat atg ttg att gag gag aca      864
   Tyr Ala Asp Gly Ile Gly Pro Asp Tyr His Met Leu Ile Glu Glu Thr
           275                 280                 285
   tcg cag ccg ggt aat atc aaa ctc act ggc atg gtg caa gat gct cag      912
   Ser Gln Pro Gly Asn Ile Lys Leu Thr Gly Met Val Gln Asp Ala Gln
       290                 295                 300
   cag aat aaa ctg gta gtg cat cct tat acc gtg cgg tca gat aaa ctg      960
   Gln Asn Lys Leu Val Val His Pro Tyr Thr Val Arg Ser Asp Lys Leu
   305                 310                 315                 320
   cct gaa tac act cct gat gtg aat cag tta tat gat gct ctg tat aac     1008
   Pro Glu Tyr Thr Pro Asp Val Asn Gln Leu Tyr Asp Ala Leu Tyr Asn
                   325                 330                 335
   aaa gcg ggt gta aat ggg ctg ttt act gat ttc cct gat aag gca gta     1056
   Lys Ala Gly Val Asn Gly Leu Phe Thr Asp Phe Pro Asp Lys Ala Val
               340                 345                 350
   aaa ttt ctt aat aaa gag taa                                         1077
   Lys Phe Leu Asn Lys Glu
           355
<210> 18
   <211> 358
   <212> PRT
   <213> Escherichia coli
<400> 18
   Met Lys Leu Thr Leu Lys Asn Leu Ser Met Ala Ile Met Met Ser Thr
   1               5                   10                  15
   Ile Val Met Gly Ser Ser Ala Met Ala Ala Asp Ser Asn Glu Lys Ile
               20                  25                  30
   Val Ile Ala His Arg Gly Ala Ser Gly Tyr Leu Pro Glu His Thr Leu
           35                  40                  45
   Pro Ala Lys Ala Met Ala Tyr Ala Gln Gly Ala Asp Tyr Leu Glu Gln
       50                  55                  60
   Asp Leu Val Met Thr Lys Asp Asp Asn Leu Val Val Leu His Asp His
   65                  70                  75                  80
   Tyr Leu Asp Arg Val Thr Asp Val Ala Asp Arg Phe Pro Asp Arg Ala
                   85                  90                  95
   Arg Lys Asp Gly Arg Tyr Tyr Ala Ile Asp Phe Thr Leu Asp Glu Ile
           100                 105                 110
   Lys Ser Leu Lys Phe Thr Glu Gly Phe Asp Ile Glu Asn Gly Lys Lys
           115                 120                 125
   Val Gln Thr Tyr Pro Gly Arg Phe Pro Met Gly Lys Ser Asp Phe Arg
       130                 135                 140
   Val His Thr Phe Glu Glu Glu Ile Glu Phe Val Gln Gly Leu Asn His
   145                 150                 155                 160
   Ser Thr Gly Lys Asn Ile Gly Ile Tyr Pro Glu Ile Lys Ala Pro Trp
                   165                 170                 175
   Phe His His Gln Glu Gly Lys Asp Ile Ala Ala Lys Thr Leu Glu Val
               180                 185                 190
   Leu Lys Lys Tyr Gly Tyr Thr Gly Lys Asp Asp Lys Val Tyr Leu Gln
           195                 200                 205
   Cys Phe Asp Ala Asp Glu Leu Lys Arg Ile Lys Asn Glu Leu Glu Pro
       210                 215                 220
   Lys Met Gly Met Glu Leu Asn Leu Val Gln Leu Ile Ala Tyr Thr Asp
   225                 230                 235                 240
   Trp Asn Glu Thr Gln Gln Lys Gln Pro Asp Gly Ser Trp Val Asn Tyr
                   245                 250                 255
   Asn Tyr Asp Trp Met Phe Lys Pro Gly Ala Met Lys Gln Val Ala Glu
               260                 265                 270
   Tyr Ala Asp Gly Ile Gly Pro Asp Tyr His Met Leu Ile Glu Glu Thr
           275                 280                 285
   Ser Gln Pro Gly Asn Ile Lys Leu Thr Gly Met Val Gln Asp Ala Gln
       290                 295                 300
   Gln Asn Lys Leu Val Val His Pro Tyr Thr Val Arg Ser Asp Lys Leu
   305                 310                 315                 320
   Pro Glu Tyr Thr Pro Asp Val Asn Gln Leu Tyr Asp Ala Leu Tyr Asn
                   325                 330                 335
   Lys Ala Gly Val Asn Gly Leu Phe Thr Asp Phe Pro Asp Lys Ala Val
               340                 345                 350
   Lys Phe Leu Asn Lys Glu
           355
<210> 19
   <211> 1359
   <212> DNA
   <213> Escherichia coli
<220>
   <221> CDS
   <222> (1)..(1359)
   <223> glpT Kodierregion
<400> 19
   atg ttg agt att ttt aaa cca gcg cca cac aaa gcg cgc tta cct gcc        48
   Met Leu Ser Ile Phe Lys Pro Ala Pro His Lys Ala Arg Leu Pro Ala
   1               5                   10                  15
   gcg gag atc gat ccg act tat cgt cga ttg cgc tgg caa att ttc ctg        96
   Ala Glu Ile Asp Pro Thr Tyr Arg Arg Leu Arg Trp Gln Ile Phe Leu
               20                  25                  30
   ggg ata ttc ttt ggc tat gcg gct tac tat ttg gtt cgt aag aac ttt       144
   Gly Ile Phe Phe Gly Tyr Ala Ala Tyr Tyr Leu Val Arg Lys Asn Phe
           35                  40                  45
   gcg ctt gct atg cct tat ctg gtt gag cag gga ttc tca cgc ggt gat       192
   Ala Leu Ala Met Pro Tyr Leu Val Glu Gln Gly Phe Ser Arg Gly Asp
       50                  55                  60
   tta ggt ttt gcc ctt tcg ggg atc tcg att gct tat gga ttt tcg aaa       240
   Leu Gly Phe Ala Leu Ser Gly Ile Ser Ile Ala Tyr Gly Phe Ser Lys
   65                  70                  75                  80
   ttc atc atg ggt tcg gta tcg gat cgc tcg aat ccg cgc gtt ttc ctg       288
   Phe Ile Met Gly Ser Val Ser Asp Arg Ser Asn Pro Arg Val Phe Leu
                   85                  90                  95
   ccc gca ggt ttg att ctg gcg gcg gca gtg atg ttg ttt atg ggc ttt       336
   Pro Ala Gly Leu Ile Leu Ala Ala Ala Val Met Leu Phe Met Gly Phe
               100                 105                 110
   gtg cca tgg gcg acg tcg agc att gcg gtg atg ttt gta ctg ttg ttc       384
   Val Pro Trp Ala Thr Ser Ser Ile Ala Val Met Phe Val Leu Leu Phe
           115                 120                 125
   ctc tgc ggt tgg ttc cag ggg atg ggg tgg ccg ccg tgt ggt cgt act       432
   Leu Cys Gly Trp Phe Gln Gly Met Gly Trp Pro Pro Cys Gly Arg Thr
       130                 135                 140
   atg gtg cac tgg tgg tcg cag aaa gaa cgt ggc ggc att gtg tca gtg       480
   Met Val His Trp Trp Ser Gln Lys Glu Arg Gly Gly Ile Val Ser Val
   145                 150                 155                 160
   tgg aac tgt gcg cac aac gtc ggt ggt ggt att ccg ccg ctg ctg ttc       528
   Trp Asn Cys Ala His Asn Val Gly Gly Gly Ile Pro Pro Leu Leu Phe
                   165                 170                 175
   ctg ctg ggg atg gcc tgg ttc aat gac tgg cat gcg gcg ctc tat atg       576
   Leu Leu Gly Met Ala Trp Phe Asn Asp Trp His Ala Ala Leu Tyr Met
               180                 185                 190
   cct gct ttc tgc gcc att ctg gtg gca tta ttc gcc ttt gcg atg atg       624
   Pro Ala Phe Cys Ala Ile Leu Val Ala Leu Phe Ala Phe Ala Met Met
           195                 200                 205
   cgc gat acc ccg caa tcc tgt ggc ttg ccg ccg atc gaa gag tac aaa       672
   Arg Asp Thr Pro Gln Ser Cys Gly Leu Pro Pro Ile Glu Glu Tyr Lys
       210                 215                 220
   aat gat tat ccg gac gac tat aac gaa aaa gcg gaa cag gag ctg acg       720
   Asn Asp Tyr Pro Asp Asp Tyr Asn Glu Lys Ala Glu Gln Glu Leu Thr
   225                 230                 235                 240
   gcg aag caa atc ttc atg cag tac gta ctg ccg aac aaa ctg ctg tgg       768
   Ala Lys Gln Ile Phe Met Gln Tyr Val Leu Pro Asn Lys Leu Leu Trp
                   245                 250                 255
   tat atc gcc atc gcc aac gtg ttc gtt tat ctg ctg cgt tac ggc atc       816
   Tyr Ile Ala Ile Ala Asn Val Phe Val Tyr Leu Leu Arg Tyr Gly Ile
               260                 265                 270
   ctc gac tgg tca ccg act tat ctg aaa gag gtt aag cat ttc gcg cta       864
   Leu Asp Trp Ser Pro Thr Tyr Leu Lys Glu Val Lys His Phe Ala Leu
           275                 280                 285
   gat aaa tcc tcc tgg gcc tac ttc ctt tat gaa tat gca ggt att ccg       912
   Asp Lys Ser Ser Trp Ala Tyr Phe Leu Tyr Glu Tyr Ala Gly Ile Pro
       290                 295                 300
   ggc act ctg ctg tgc ggc tgg atg tcg gat aaa gtc ttc cgt ggc aac       960
   Gly Thr Leu Leu Cys Gly Trp Met Ser Asp Lys Val Phe Arg Gly Asn
   305                 310                 315                 320
   cgt ggg gca acc ggc gtt ttc ttt atg aca ctg gtg acc atc gcg act      1008
   Arg Gly Ala Thr Gly Val Phe Phe Met Thr Leu Val Thr Ile Ala Thr
                   325                 330                 335
   atc gtt tac tgg atg aac ccg gca ggt aac cca acc gtc gat atg att      1056
   Ile Val Tyr Trp Met Asn Pro Ala Gly Asn Pro Thr Val Asp Met Ile
               340                 345                 350
   tgt atg att gtt atc ggc ttc ctg atc tac ggt cct gtg atg ctg atc      1104
   Cys Met Ile Val Ile Gly Phe Leu Ile Tyr Gly Pro Val Met Leu Ile
           355                 360                 365
   ggt ctg cat gcg ctg gaa ctg gca ccg aaa aaa gcg gca ggt acg gca      1152
   Gly Leu His Ala Leu Glu Leu Ala Pro Lys Lys Ala Ala Gly Thr Ala
       370                 375                 380
   gcg ggc ttt acc ggg ctg ttt ggt tac ctg ggc ggt tcg gtg gcg gcg      1200
   Ala Gly Phe Thr Gly Leu Phe Gly Tyr Leu Gly Gly Ser Val Ala Ala
   385                 390                 395                 400
   agc gcg att gtt ggc tac acc gtg gac ttc ttc ggc tgg gat ggc ggc      1248
   Ser Ala Ile Val Gly Tyr Thr Val Asp Phe Phe Gly Trp Asp Gly Gly
                   405                 410                 415
   ttt atg gta atg att ggc ggc agc att ctg gcg gtt atc ttg ttg att      1296
   Phe Met Val Met Ile Gly Gly Ser Ile Leu Ala Val Ile Leu Leu Ile
               420                 425                 430
   gtt gtg atg att ggc gaa aaa cgt cgc cat gaa caa tta ctg caa gaa      1344
   Val Val Met Ile Gly Glu Lys Arg Arg His Glu Gln Leu Leu Gln Glu
           435                 440                 445
   cgc aac gga ggc taa                                                  1359
   Arg Asn Gly Gly
       450
<210> 20
   <211> 452
   <212> PRT
   <213> Escherichia coli
<400> 20
   Met Leu Ser Ile Phe Lys Pro Ala Pro His Lys Ala Arg Leu Pro Ala
   1               5                   10                  15
   Ala Glu Ile Asp Pro Thr Tyr Arg Arg Leu Arg Trp Gln Ile Phe Leu
               20                  25                  30
   Gly Ile Phe Phe Gly Tyr Ala Ala Tyr Tyr Leu Val Arg Lys Asn Phe
           35                  40                  45
   Ala Leu Ala Met Pro Tyr Leu Val Glu Gln Gly Phe Ser Arg Gly Asp
       50                  55                  60
   Leu Gly Phe Ala Leu Ser Gly Ile Ser Ile Ala Tyr Gly Phe Ser Lys
   65                  70                  75                  80
   Phe Ile Met Gly Ser Val Ser Asp Arg Ser Asn Pro Arg Val Phe Leu
                   85                  90                  95
   Pro Ala Gly Leu Ile Leu Ala Ala Ala Val Met Leu Phe Met Gly Phe
               100                 105                 110
   Val Pro Trp Ala Thr Ser Ser Ile Ala Val Met Phe Val Leu Leu Phe
           115                 120                 125
   Leu Cys Gly Trp Phe Gln Gly Met Gly Trp Pro Pro Cys Gly Arg Thr
       130                 135                 140
   Met Val His Trp Trp Ser Gln Lys Glu Arg Gly Gly Ile Val Ser Val
   145                 150                 155                 160
   Trp Asn Cys Ala His Asn Val Gly Gly Gly Ile Pro Pro Leu Leu Phe
                   165                 170                 175
   Leu Leu Gly Met Ala Trp Phe Asn Asp Trp His Ala Ala Leu Tyr Met
   180 185 190
   Pro Ala Phe Cys Ala Ile Leu Val Ala Leu Phe Ala Phe Ala Met Met
   195 200 205
   Arg Asp Thr Pro Gln Ser Cys Gly Leu Pro Pro Ile Glu Glu Tyr Lys
   210 215 220
   Asn Asp Tyr Pro Asp Asp Tyr Asn Glu Lys Ala Glu Gln Glu Leu Thr
   225 230 235 240
   Ala Lys Gln Ile Phe Met Gln Tyr Val Leu Pro Asn Lys Leu Leu Trp
   245 250 255
   Tyr Ile Ala Ile Ala Asn Val Phe Val Tyr Leu Leu Arg Tyr Gly Ile
   260 265 270
   Leu Asp Trp Ser Pro Thr Tyr Leu Lys Glu Val Lys His Phe Ala Leu
   275 280 285
   Asp Lys Ser Ser Trp Ala Tyr Phe Leu Tyr Glu Tyr Ala Gly Ile Pro
   290 295 300
   Gly Thr Leu Leu Cys Gly Trp Met Ser Asp Lys Val Phe Arg Gly Asn
   305 310 315 320
   Arg Gly Ala Thr Gly Val Phe Phe Met Thr Leu Val Thr Ile Ala Thr
   325 330 335
   Ile Val Tyr Trp Met Asn Pro Ala Gly Asn Pro Thr Val Asp Met Ile
   340 345 350
   Cys Met Ile Val Ile Gly Phe Leu Ile Tyr Gly Pro Val Met Leu Ile
   355 360 365
   Gly Leu His Ala Leu Glu Leu Ala Pro Lys Lys Ala Ala Gly Thr Ala
   370 375 380
   Ala Gly Phe Thr Gly Leu Phe Gly Tyr Leu Gly Gly Ser Val Ala Ala
   385 390 395 400
   Ser Ala Ile Val Gly Tyr Thr Val Asp Phe Phe Gly Trp Asp Gly Gly
   405 410 415
   Phe Met Val Met Ile Gly Gly Ser Ile Leu Ala Val Ile Leu Leu Ile
   420 425 430
   Val Val Met Ile Gly Glu Lys Arg Arg His Glu Gln Leu Leu Gln Glu
   435 440 445
   Arg Asn Gly Gly
   450
<210> 21
   <211> 1011
   <212> DNA
   <213> Escherichia coli
<220>
   <221> CDS
   <222> (1)..(1011)
   <223> glpX Kodierregion
<400> 21
   atg aga cga gaa ctt gcc atc gaa ttt tcc cgc gtc acc gaa tca gcg        48
   Met Arg Arg Glu Leu Ala Ile Glu Phe Ser Arg Val Thr Glu Ser Ala
   1               5                   10                  15
   gcg ctg gct ggc tac aaa tgg tta gga cgc ggc gat aaa aac acc gcg        96
   Ala Leu Ala Gly Tyr Lys Trp Leu Gly Arg Gly Asp Lys Asn Thr Ala
               20                  25                  30
   gac ggc gcg gcg gta aac gcc atg cgt att atg ctc aac cag gtc aac       144
   Asp Gly Ala Ala Val Asn Ala Met Arg Ile Met Leu Asn Gln Val Asn
           35                  40                  45
   att gac ggc acc atc gtc att ggt gaa ggt gaa atc gac gaa gca ccg       192
   Ile Asp Gly Thr Ile Val Ile Gly Glu Gly Glu Ile Asp Glu Ala Pro
       50                  55                  60
   atg ctc tac att ggt gaa aaa gtc ggt act ggt cgc ggc gac gcg gta       240
   Met Leu Tyr Ile Gly Glu Lys Val Gly Thr Gly Arg Gly Asp Ala Val
   65                  70                  75                  80
   gat att gct gtt gat ccg att gaa ggc acg cgc atg acg gcg atg ggc       288
   Asp Ile Ala Val Asp Pro Ile Glu Gly Thr Arg Met Thr Ala Met Gly
                   85                  90                  95
   cag gct aac gcg ctg gcg gtg ctg gca gta ggc gat aaa ggc tgc ttc       336
   Gln Ala Asn Ala Leu Ala Val Leu Ala Val Gly Asp Lys Gly Cys Phe
               100                 105                 110
   ctc aat gcg ccg gat atg tat atg gag aag ctg att gtc ggg ccg gga       384
   Leu Asn Ala Pro Asp Met Tyr Met Glu Lys Leu Ile Val Gly Pro Gly
           115                 120                 125
   gcc aaa ggc acc att gat ctg aac ctg ccg ctg gcg gat aac ctg cgc       432
   Ala Lys Gly Thr Ile Asp Leu Asn Leu Pro Leu Ala Asp Asn Leu Arg
       130                 135                 140
   aat gta gcg gcg gcg ctc ggc aaa ccg ttg agc gaa ctg acg gta acg       480
   Asn Val Ala Ala Ala Leu Gly Lys Pro Leu Ser Glu Leu Thr Val Thr
   145                 150                 155                 160
   att ctg gct aaa cca cgc cac gat gcc gtt atc gct gaa atg cag caa       528
   Ile Leu Ala Lys Pro Arg His Asp Ala Val Ile Ala Glu Met Gln Gln
                   165                 170                 175
   ctc ggc gta cgc gta ttt gct att ccg gac ggc gac gtt gcg gcc tca      576
   Leu Gly Val Arg Val Phe Ala Ile Pro Asp Gly Asp Val Ala Ala Ser
               180                 185                 190
   att ctc acc tgt atg cca gac agc gaa gtt gac gtg ctg tac ggt att      624
   Ile Leu Thr Cys Met Pro Asp Ser Glu Val Asp Val Leu Tyr Gly Ile
           195                 200                 205
   ggt ggc gcg ccg gaa ggc gta gtt tct gcg gcg gtg atc cgc gca tta      672
   Gly Gly Ala Pro Glu Gly Val Val Ser Ala Ala Val Ile Arg Ala Leu
       210                 215                 220
   gat ggc gac atg aac ggt cgt ctg ctg gcg cgt cat gac gtc aaa ggc      720
   Asp Gly Asp Met Asn Gly Arg Leu Leu Ala Arg His Asp Val Lys Gly
   225                 230                 235                 240
   gac aac gaa gag aat cgt cgc att ggc gag cag gag ctg gca cgc tgc      768
   Asp Asn Glu Glu Asn Arg Arg Ile Gly Glu Gln Glu Leu Ala Arg Cys
                   245                 250                 255
   aaa gcg atg ggc atc gaa gcc ggt aaa gta ttg cgc ctg ggc gat atg      816
   Lys Ala Met Gly Ile Glu Ala Gly Lys Val Leu Arg Leu Gly Asp Met
               260                 265                 270
   gcg cgc agc gat aac gtc atc ttc tct gcc acc ggt att acc aaa ggc      864
   Ala Arg Ser Asp Asn Val Ile Phe Ser Ala Thr Gly Ile Thr Lys Gly
           275                 280                 285
   gat ctg ctg gaa ggc att agc cgc aaa ggc aat atc gcg act acc gaa      912
   Asp Leu Leu Glu Gly Ile Ser Arg Lys Gly Asn Ile Ala Thr Thr Glu
       290                 295                 300
   acg ctg ctg atc cgc ggc aag tca cgc acc att cgc cgc att cag tcc      960
   Thr Leu Leu Ile Arg Gly Lys Ser Arg Thr Ile Arg Arg Ile Gln Ser
   305                 310                 315                 320
   atc cac tat ctg gat cgc aaa gac ccg gaa atg cag gtg cac atc ctc     1008
   Ile His Tyr Leu Asp Arg Lys Asp Pro Glu Met Gln Val His Ile Leu
                   325                 330                 335
   tga                                                                 1011
<210> 22
   <211> 336
   <212> PRT
   <213> Escherichia coli
<400> 22
   Met Arg Arg Glu Leu Ala Ile Glu Phe Ser Arg Val Thr Glu Ser Ala
   1               5                   10                  15
   Ala Leu Ala Gly Tyr Lys Trp Leu Gly Arg Gly Asp Lys Asn Thr Ala
               20                  25                  30
   Asp Gly Ala Ala Val Asn Ala Met Arg Ile Met Leu Asn Gln Val Asn
           35                  40                  45
   Ile Asp Gly Thr Ile Val Ile Gly Glu Gly Glu Ile Asp Glu Ala Pro
       50                  55                  60
   Met Leu Tyr Ile Gly Glu Lys Val Gly Thr Gly Arg Gly Asp Ala Val
   65                  70                  75                  80
   Asp Ile Ala Val Asp Pro Ile Glu Gly Thr Arg Met Thr Ala Met Gly
                   85                  90                  95
   Gln Ala Asn Ala Leu Ala Val Leu Ala Val Gly Asp Lys Gly Cys Phe
               100                 105                 110
   Leu Asn Ala Pro Asp Met Tyr Met Glu Lys Leu Ile Val Gly Pro Gly
           115                 120                 125
   Ala Lys Gly Thr Ile Asp Leu Asn Leu Pro Leu Ala Asp Asn Leu Arg
       130                 135                 140
   Asn Val Ala Ala Ala Leu Gly Lys Pro Leu Ser Glu Leu Thr Val Thr
   145                 150                 155                 160
   Ile Leu Ala Lys Pro Arg His Asp Ala Val Ile Ala Glu Met Gln Gln
                   165                 170                 175
   Leu Gly Val Arg Val Phe Ala Ile Pro Asp Gly Asp Val Ala Ala Ser
               180                 185                 190
   Ile Leu Thr Cys Met Pro Asp Ser Glu Val Asp Val Leu Tyr Gly Ile
           195                 200                 205
   Gly Gly Ala Pro Glu Gly Val Val Ser Ala Ala Val Ile Arg Ala Leu
       210                 215                 220
   Asp Gly Asp Met Asn Gly Arg Leu Leu Ala Arg His Asp Val Lys Gly
   225                 230                 235                 240
   Asp Asn Glu Glu Asn Arg Arg Ile Gly Glu Gln Glu Leu Ala Arg Cys
                   245                 250                 255
   Lys Ala Met Gly Ile Glu Ala Gly Lys Val Leu Arg Leu Gly Asp Met
               260                 265                 270
   Ala Arg Ser Asp Asn Val Ile Phe Ser Ala Thr Gly Ile Thr Lys Gly
           275                 280                 285
   Asp Leu Leu Glu Gly Ile Ser Arg Lys Gly Asn Ile Ala Thr Thr Glu
       290                 295                 300
   Thr Leu Leu Ile Arg Gly Lys Ser Arg Thr Ile Arg Arg Ile Gln Ser
   305                 310                 315                 320
   Ile His Tyr Leu Asp Arg Lys Asp Pro Glu Met Gln Val His Ile Leu
                   325                 330                 335
<210> 23
   <211> 1143
   <212> DNA
   <213> Escherichia coli
<220>
   <221> CDS
   <222> (1)..(1143)
   <223> gldA Kodierregion
<400> 23
   atg ccg cat ttg gca cta ctc atc tct aaa gga gca att atg gac cgc       48
   Met Pro His Leu Ala Leu Leu Ile Ser Lys Gly Ala Ile Met Asp Arg
   1               5                   10                  15
   att att caa tca ccg ggt aaa tac atc cag ggc gct gat gtg att aat       96
   Ile Ile Gln Ser Pro Gly Lys Tyr Ile Gln Gly Ala Asp Val Ile Asn
               20                  25                  30
   cgt ctg ggc gaa tac ctg aag ccg ctg gca gaa cgc tgg tta gtg gtg      144
   Arg Leu Gly Glu Tyr Leu Lys Pro Leu Ala Glu Arg Trp Leu Val Val
           35                  40                  45
   ggt gac aaa ttt gtt tta ggt ttt gct caa tcc act gtc gag aaa agc      192
   Gly Asp Lys Phe Val Leu Gly Phe Ala Gln Ser Thr Val Glu Lys Ser
       50                  55                  60
   ttt aaa gat gct gga ctg gta gta gaa att gcg ccg ttt ggc ggt gaa      240
   Phe Lys Asp Ala Gly Leu Val Val Glu Ile Ala Pro Phe Gly Gly Glu
   65                  70                  75                  80
   tgt tcg caa aat gag atc gac cgt ctg cgt ggc atc gcg gag act gcg      288
   Cys Ser Gln Asn Glu Ile Asp Arg Leu Arg Gly Ile Ala Glu Thr Ala
                   85                  90                  95
   cag tgt ggc gca att ctc ggt atc ggt ggc gga aaa acc ctc gat act      336
   Gln Cys Gly Ala Ile Leu Gly Ile Gly Gly Gly Lys Thr Leu Asp Thr
               100                 105                 110
   gcc aaa gca ctg gca cat ttc atg ggt gtt ccg gta gcg atc gca ccg      384
   Ala Lys Ala Leu Ala His Phe Met Gly Val Pro Val Ala Ile Ala Pro
           115                 120                 125
   act atc gcc tct acc gat gca ccg tgc agc gca ttg tct gtt atc tac      432
   Thr Ile Ala Ser Thr Asp Ala Pro Cys Ser Ala Leu Ser Val Ile Tyr
       130                 135                 140
   acc gat gag ggt gag ttt gac cgc tat ctg ctg ttg cca aat aac ccg      480
   Thr Asp Glu Gly Glu Phe Asp Arg Tyr Leu Leu Leu Pro Asn Asn Pro
   145                 150                 155                 160
   aat atg gtc att gtc gac acc aaa atc gtc gct ggc gca cct gca cgt      528
   Asn Met Val Ile Val Asp Thr Lys Ile Val Ala Gly Ala Pro Ala Arg
                   165                 170                 175
   ctg tta gcg gcg ggt atc ggc gat gcg ctg gca acc tgg ttt gaa gcg      576
   Leu Leu Ala Ala Gly Ile Gly Asp Ala Leu Ala Thr Trp Phe Glu Ala
               180                 185                 190
   cgt gcc tgc tct cgt agc ggc gcg acc acc atg gcg ggc ggc aag tgc      624
   Arg Ala Cys Ser Arg Ser Gly Ala Thr Thr Met Ala Gly Gly Lys Cys
           195                 200                 205
   acc cag gct gcg ctg gca ctg gct gaa ctg tgc tac aac acc ctg ctg      672
   Thr Gln Ala Ala Leu Ala Leu Ala Glu Leu Cys Tyr Asn Thr Leu Leu
       210                 215                 220
   gaa gaa ggc gaa aaa gcg atg ctt gct gcc gaa cag cat gta gtg act      720
   Glu Glu Gly Glu Lys Ala Met Leu Ala Ala Glu Gln His Val Val Thr
   225                 230                 235                 240
   ccg gcg ctg gag cgc gtg att gaa gcg aac acc tat ttg agc ggt gtt      768
   Pro Ala Leu Glu Arg Val Ile Glu Ala Asn Thr Tyr Leu Ser Gly Val
                   245                 250                 255
   ggt ttt gaa agt ggt ggt ctg gct gcg gcg cac gca gtg cat aac ggc      816
   Gly Phe Glu Ser Gly Gly Leu Ala Ala Ala His Ala Val His Asn Gly
               260                 265                 270
   ctg acc gct atc ccg gac gcg cat cac tat tat cac ggt gaa aaa gtg      864
   Leu Thr Ala Ile Pro Asp Ala His His Tyr Tyr His Gly Glu Lys Val
           275                 280                 285
   gca ttc ggt acg ctg acg cag ctg gtt ctg gaa aat gcg ccg gtg gag      912
   Ala Phe Gly Thr Leu Thr Gln Leu Val Leu Glu Asn Ala Pro Val Glu
       290                 295                 300
   gaa atc gaa acc gta gct gcc ctt agc cat gcg gta ggt ttg cca ata      960
   Glu Ile Glu Thr Val Ala Ala Leu Ser His Ala Val Gly Leu Pro Ile
   305                 310                 315                 320
   act ctc gct caa ctg gat att aaa gaa gat gtc ccg gcg aaa atg cga     1008
   Thr Leu Ala Gln Leu Asp Ile Lys Glu Asp Val Pro Ala Lys Met Arg
                   325                 330                 335
   att gtg gca gaa gcg gca tgt gca gaa ggt gaa acc att cac aac atg     1056
   Ile Val Ala Glu Ala Ala Cys Ala Glu Gly Glu Thr Ile His Asn Met
               340                 345                 350
   cct ggc ggc gcg acg cca gat cag gtt tac gcc gct ctg ctg gta gcc     1104
   Pro Gly Gly Ala Thr Pro Asp Gln Val Tyr Ala Ala Leu Leu Val Ala
           355                 360                 365
   gac cag tac ggt cag cgt ttc ctg caa gag tgg gaa taa                 1143
   Asp Gln Tyr Gly Gln Arg Phe Leu Gln Glu Trp Glu
       370                 375                 380
<210> 24
   <211> 380
   <212> PRT
   <213> Escherichia coli
<400> 24
   Met Pro His Leu Ala Leu Leu Ile Ser Lys Gly Ala Ile Met Asp Arg
   1               5                   10                  15
   Ile Ile Gln Ser Pro Gly Lys Tyr Ile Gln Gly Ala Asp Val Ile Asn
               20                  25                  30
   Arg Leu Gly Glu Tyr Leu Lys Pro Leu Ala Glu Arg Trp Leu Val Val
           35                  40                  45
   Gly Asp Lys Phe Val Leu Gly Phe Ala Gln Ser Thr Val Glu Lys Ser
       50                  55                  60
   Phe Lys Asp Ala Gly Leu Val Val Glu Ile Ala Pro Phe Gly Gly Glu
   65                  70                  75                  80
   Cys Ser Gln Asn Glu Ile Asp Arg Leu Arg Gly Ile Ala Glu Thr Ala
                   85                  90                  95
   Gln Cys Gly Ala Ile Leu Gly Ile Gly Gly Gly Lys Thr Leu Asp Thr
               100                 105                 110
   Ala Lys Ala Leu Ala His Phe Met Gly Val Pro Val Ala Ile Ala Pro
           115                 120                 125
   Thr Ile Ala Ser Thr Asp Ala Pro Cys Ser Ala Leu Ser Val Ile Tyr
       130                 135                 140
   Thr Asp Glu Gly Glu Phe Asp Arg Tyr Leu Leu Leu Pro Asn Asn Pro
   145                 150                 155                 160
   Asn Met Val Ile Val Asp Thr Lys Ile Val Ala Gly Ala Pro Ala Arg
                   165                 170                 175
   Leu Leu Ala Ala Gly Ile Gly Asp Ala Leu Ala Thr Trp Phe Glu Ala
               180                 185                 190
   Arg Ala Cys Ser Arg Ser Gly Ala Thr Thr Met Ala Gly Gly Lys Cys
           195                 200                 205
   Thr Gln Ala Ala Leu Ala Leu Ala Glu Leu Cys Tyr Asn Thr Leu Leu
       210                 215                 220
   Glu Glu Gly Glu Lys Ala Met Leu Ala Ala Glu Gln His Val Val Thr
   225                 230                 235                 240
   Pro Ala Leu Glu Arg Val Ile Glu Ala Asn Thr Tyr Leu Ser Gly Val
                   245                 250                 255
   Gly Phe Glu Ser Gly Gly Leu Ala Ala Ala His Ala Val His Asn Gly
               260                 265                 270
   Leu Thr Ala Ile Pro Asp Ala His His Tyr Tyr His Gly Glu Lys Val
           275                 280                 285
   Ala Phe Gly Thr Leu Thr Gln Leu Val Leu Glu Asn Ala Pro Val Glu
       290                 295                 300
   Glu Ile Glu Thr Val Ala Ala Leu Ser His Ala Val Gly Leu Pro Ile
   305                 310                 315                 320
   Thr Leu Ala Gln Leu Asp Ile Lys Glu Asp Val Pro Ala Lys Met Arg
                   325                 330                 335
   Ile Val Ala Glu Ala Ala Cys Ala Glu Gly Glu Thr Ile His Asn Met
               340                 345                 350
   Pro Gly Gly Ala Thr Pro Asp Gln Val Tyr Ala Ala Leu Leu Val Ala
           355                 360                 365
   Asp Gln Tyr Gly Gln Arg Phe Leu Gln Glu Trp Glu
       370                 375                 380
<210> 25
   <211> 1101
   <212> DNA
   <213> Escherichia coli
<220>
   <221> CDS
   <222> (1)..(1101)
   <223> dhaK Kodierregion
<400> 25
   atg ccc tac cgt aat tgc tgg agc aaa ata atg aaa aaa ttg atc aat       48
   Met Pro Tyr Arg Asn Cys Trp Ser Lys Ile Met Lys Lys Leu Ile Asn
   1               5                   10                  15
   gat gtg caa gac gta ctg gac gaa caa ctg gca gga ctg gcg aaa gcg       96
   Asp Val Gln Asp Val Leu Asp Glu Gln Leu Ala Gly Leu Ala Lys Ala
               20                  25                  30
   cat cca tcg ctg aca ctg cat cag gat ccg gtg tat gtc acc cga gct      144
   His Pro Ser Leu Thr Leu His Gln Asp Pro Val Tyr Val Thr Arg Ala
           35                  40                  45
   gat gcc cct gtt gca gga aaa gtc gcc ctg ctg tcg ggt ggc ggc agc      192
   Asp Ala Pro Val Ala Gly Lys Val Ala Leu Leu Ser Gly Gly Gly Ser
       50                  55                  60
   gga cac gag ccg atg cac tgt ggt tat atc ggt cag ggg atg ctt tcg      240
   Gly His Glu Pro Met His Cys Gly Tyr Ile Gly Gln Gly Met Leu Ser
   65                  70                  75                  80
   ggg gcc tgt ccg ggc gaa att ttc acc tca ccg acg ccc gat aaa atc      288
   Gly Ala Cys Pro Gly Glu Ile Phe Thr Ser Pro Thr Pro Asp Lys Ile
                   85                  90                  95
   ttt gaa tgc gcc atg caa gtt gat ggc ggc gaa ggt gta ctg ttg att      336
   Phe Glu Cys Ala Met Gln Val Asp Gly Gly Glu Gly Val Leu Leu Ile
               100                 105                 110
   atc aaa aat tac acc ggc gat att ctt aac ttt gaa aca gcg acc gag      384
   Ile Lys Asn Tyr Thr Gly Asp Ile Leu Asn Phe Glu Thr Ala Thr Glu
           115                 120                 125
   tta ctg cac gat agc ggc gta aaa gtg acc act gtg gtc att gat gac      432
   Leu Leu His Asp Ser Gly Val Lys Val Thr Thr Val Val Ile Asp Asp
       130                 135                 140
   gac gtt gcg gta aaa gac agt ctt tat act gcc ggg cga cgc ggc gtt      480
   Asp Val Ala Val Lys Asp Ser Leu Tyr Thr Ala Gly Arg Arg Gly Val
   145                 150                 155                 160
   gcc aac acc gta tta att gaa aaa ctc gta ggc gca gcg gcg gag cgt      528
   Ala Asn Thr Val Leu Ile Glu Lys Leu Val Gly Ala Ala Ala Glu Arg
                   165                 170                 175
   ggc gac tca ctg gac gcc tgt gcg gaa ctg ggg cgt aag ctg aat aat      576
   Gly Asp Ser Leu Asp Ala Cys Ala Glu Leu Gly Arg Lys Leu Asn Asn
               180                 185                 190
   caa ggc cac tca ata ggt atc gct ctc ggt gcc tgt acc gtt cct gcc      624
   Gln Gly His Ser Ile Gly Ile Ala Leu Gly Ala Cys Thr Val Pro Ala
           195                 200                 205
   gcg ggc aaa cct tct ttt acc ctg gcg gat aat gag atg gag ttt ggc      672
   Ala Gly Lys Pro Ser Phe Thr Leu Ala Asp Asn Glu Met Glu Phe Gly
       210                 215                 220
   gtc ggc att cat ggt gag ccg ggt att gac cgc cgc ccc ttc tct tcc      720
   Val Gly Ile His Gly Glu Pro Gly Ile Asp Arg Arg Pro Phe Ser Ser
   225                 230                 235                 240
   ctt gat caa acc gtc gat gaa atg ttc gac acc ctg ctg gta aat ggc      768
   Leu Asp Gln Thr Val Asp Glu Met Phe Asp Thr Leu Leu Val Asn Gly
                   245                 250                 255
   tca tac cat cgc act ttg cgt ttc tgg gat tat caa caa ggc agt tgg      816
   Ser Tyr His Arg Thr Leu Arg Phe Trp Asp Tyr Gln Gln Gly Ser Trp
               260                 265                 270
   cag gaa gaa caa caa acc aaa caa ccg ctc cag tct ggc gat cgg gtg      864
   Gln Glu Glu Gln Gln Thr Lys Gln Pro Leu Gln Ser Gly Asp Arg Val
           275                 280                 285
   att gcg ctg gtt aac aat ctt ggc gca act ccg ctt tct gag ctg tac      912
   Ile Ala Leu Val Asn Asn Leu Gly Ala Thr Pro Leu Ser Glu Leu Tyr
       290                 295                 300
   ggc gtc tat aac cgc ctg acc aca cgt tgc cag caa gcg gga ttg act      960
   Gly Val Tyr Asn Arg Leu Thr Thr Arg Cys Gln Gln Ala Gly Leu Thr
   305                 310                 315                 320
   atc gaa cgt aat tta att ggc gcg tac tgc acc tca ctg gat atg acc     1008
   Ile Glu Arg Asn Leu Ile Gly Ala Tyr Cys Thr Ser Leu Asp Met Thr
                   325                 330                 335
   ggt ttc tca atc acc tta ctg aaa gtt gat gac gaa acg ctg gca ctc     1056
   Gly Phe Ser Ile Thr Leu Leu Lys Val Asp Asp Glu Thr Leu Ala Leu
               340                 345                 350
   tgg gac gcc ccg gtc cac acc ccg gcc ctt aac tgg ggt aaa taa         1101
   Trp Asp Ala Pro Val His Thr Pro Ala Leu Asn Trp Gly Lys
           355                 360                 365
<210> 26
   <211> 366
   <212> PRT
   <213> Escherichia coli
<400> 26
   Met Pro Tyr Arg Asn Cys Trp Ser Lys Ile Met Lys Lys Leu Ile Asn
   1               5                   10                  15
   Asp Val Gln Asp Val Leu Asp Glu Gln Leu Ala Gly Leu Ala Lys Ala
               20                  25                  30
   His Pro Ser Leu Thr Leu His Gln Asp Pro Val Tyr Val Thr Arg Ala
           35                  40                  45
   Asp Ala Pro Val Ala Gly Lys Val Ala Leu Leu Ser Gly Gly Gly Ser
       50                  55                  60
   Gly His Glu Pro Met His Cys Gly Tyr Ile Gly Gln Gly Met Leu Ser
   65                  70                  75                  80
   Gly Ala Cys Pro Gly Glu Ile Phe Thr Ser Pro Thr Pro Asp Lys Ile
                   85                  90                  95
   Phe Glu Cys Ala Met Gln Val Asp Gly Gly Glu Gly Val Leu Leu Ile
               100                 105                 110
   Ile Lys Asn Tyr Thr Gly Asp Ile Leu Asn Phe Glu Thr Ala Thr Glu
           115                 120                 125
   Leu Leu His Asp Ser Gly Val Lys Val Thr Thr Val Val Ile Asp Asp
       130                 135                 140
   Asp Val Ala Val Lys Asp Ser Leu Tyr Thr Ala Gly Arg Arg Gly Val
   145                 150                 155                 160
   Ala Asn Thr Val Leu Ile Glu Lys Leu Val Gly Ala Ala Ala Glu Arg
                   165                 170                 175
   Gly Asp Ser Leu Asp Ala Cys Ala Glu Leu Gly Arg Lys Leu Asn Asn
               180                 185                 190
   Gln Gly His Ser Ile Gly Ile Ala Leu Gly Ala Cys Thr Val Pro Ala
           195                 200                 205
   Ala Gly Lys Pro Ser Phe Thr Leu Ala Asp Asn Glu Met Glu Phe Gly
       210                 215                 220
   Val Gly Ile His Gly Glu Pro Gly Ile Asp Arg Arg Pro Phe Ser Ser
   225                 230                 235                 240
   Leu Asp Gln Thr Val Asp Glu Met Phe Asp Thr Leu Leu Val Asn Gly
                   245                 250                 255
   Ser Tyr His Arg Thr Leu Arg Phe Trp Asp Tyr Gln Gln Gly Ser Trp
               260                 265                 270
   Gln Glu Glu Gln Gln Thr Lys Gln Pro Leu Gln Ser Gly Asp Arg Val
           275                 280                 285
   Ile Ala Leu Val Asn Asn Leu Gly Ala Thr Pro Leu Ser Glu Leu Tyr
       290                 295                 300
   Gly Val Tyr Asn Arg Leu Thr Thr Arg Cys Gln Gln Ala Gly Leu Thr
   305                 310                 315                 320
   Ile Glu Arg Asn Leu Ile Gly Ala Tyr Cys Thr Ser Leu Asp Met Thr
                   325                 330                 335
   Gly Phe Ser Ile Thr Leu Leu Lys Val Asp Asp Glu Thr Leu Ala Leu
               340                 345                 350
   Trp Asp Ala Pro Val His Thr Pro Ala Leu Asn Trp Gly Lys
           355                 360                 365
<210> 27
   <211> 633
   <212> DNA
   <213> Escherichia coli
<220>
   <221> CDS
   <222> (1)..(633)
   <223> dhaL Kodierregion
<400> 27
   atg tca ctg agc aga act caa att gtt aac tgg ctc act cgt tgt ggc      48
   Met Ser Leu Ser Arg Thr Gln Ile Val Asn Trp Leu Thr Arg Cys Gly
   1               5                   10                  15
   gat att ttc agc acc gag agc gag tat ctt acc gga ctg gat cgc gaa      96
   Asp Ile Phe Ser Thr Glu Ser Glu Tyr Leu Thr Gly Leu Asp Arg Glu
               20                  25                  30
   att ggc gat gct gac cac ggg cta aat atg aac cga ggc ttt agc aaa     144
   Ile Gly Asp Ala Asp His Gly Leu Asn Met Asn Arg Gly Phe Ser Lys
           35                  40                  45
   gtg gtg gaa aaa ctc cct gct atc gca gat aaa gat atc ggt ttc att     192
   Val Val Glu Lys Leu Pro Ala Ile Ala Asp Lys Asp Ile Gly Phe Ile
       50                  55                  60
   ctc aag aat acc ggt atg acg ctg ctt tcc agc gtc ggt ggt gcc agt     240
   Leu Lys Asn Thr Gly Met Thr Leu Leu Ser Ser Val Gly Gly Ala Ser
   65                  70                  75                  80
   ggt ccg ctg ttc ggt acc ttc ttt atc cgc gcc gca cag gcg acc cag     288
   Gly Pro Leu Phe Gly Thr Phe Phe Ile Arg Ala Ala Gln Ala Thr Gln
                   85                  90                  95
   gca cgg caa agc ctg aca ctg gaa gag ctt tat cag atg ttc cgc gat     336
   Ala Arg Gln Ser Leu Thr Leu Glu Glu Leu Tyr Gln Met Phe Arg Asp
               100                 105                 110
   ggc gcg gac ggc gta atc agt cgc ggg aaa gcc gaa cct ggc gat aaa     384
   Gly Ala Asp Gly Val Ile Ser Arg Gly Lys Ala Glu Pro Gly Asp Lys
           115                 120                 125
   acc atg tgt gat gtg tgg gtg ccg gtg gtg gaa tcg tta cgt cag tcc     432
   Thr Met Cys Asp Val Trp Val Pro Val Val Glu Ser Leu Arg Gln Ser
       130                 135                 140
   agc gag caa aat ctc tct gtt ccg gtg gcg ctc gaa gct gcc agt agc     480
   Ser Glu Gln Asn Leu Ser Val Pro Val Ala Leu Glu Ala Ala Ser Ser
   145                 150                 155                 160
   atc gcc gaa tcc gct gca caa agt acg att acg atg caa gcc cgc aaa     528
   Ile Ala Glu Ser Ala Ala Gln Ser Thr Ile Thr Met Gln Ala Arg Lys
                   165                 170                 175
   ggc cgc gcc agt tat ctc ggt gaa cgc agt att ggt cac cag gat ccc     576
   Gly Arg Ala Ser Tyr Leu Gly Glu Arg Ser Ile Gly His Gln Asp Pro
               180                 185                 190
   ggc gcg acc tcg gtg atg ttt atg atg caa atg ttg gcg tta gcc gca     624
   Gly Ala Thr Ser Val Met Phe Met Met Gln Met Leu Ala Leu Ala Ala
           195                 200                 205
   aaa gag taa                                                         633
   Lys Glu
       210
<210> 28
   <211> 210
   <212> PRT
   <213> Escherichia coli
<400> 28
   Met Ser Leu Ser Arg Thr Gln Ile Val Asn Trp Leu Thr Arg Cys Gly
   1               5                   10                  15
   Asp Ile Phe Ser Thr Glu Ser Glu Tyr Leu Thr Gly Leu Asp Arg Glu
               20                  25                  30
   Ile Gly Asp Ala Asp His Gly Leu Asn Met Asn Arg Gly Phe Ser Lys
           35                  40                  45
   Val Val Glu Lys Leu Pro Ala Ile Ala Asp Lys Asp Ile Gly Phe Ile
       50                  55                  60
   Leu Lys Asn Thr Gly Met Thr Leu Leu Ser Ser Val Gly Gly Ala Ser
   65                  70                  75                  80
   Gly Pro Leu Phe Gly Thr Phe Phe Ile Arg Ala Ala Gln Ala Thr Gln
                   85                  90                  95
   Ala Arg Gln Ser Leu Thr Leu Glu Glu Leu Tyr Gln Met Phe Arg Asp
               100                 105                 110
   Gly Ala Asp Gly Val Ile Ser Arg Gly Lys Ala Glu Pro Gly Asp Lys
           115                 120                 125
   Thr Met Cys Asp Val Trp Val Pro Val Val Glu Ser Leu Arg Gln Ser
       130                 135                 140
   Ser Glu Gln Asn Leu Ser Val Pro Val Ala Leu Glu Ala Ala Ser Ser
   145                 150                 155                 160
   Ile Ala Glu Ser Ala Ala Gln Ser Thr Ile Thr Met Gln Ala Arg Lys
                   165                 170                 175
   Gly Arg Ala Ser Tyr Leu Gly Glu Arg Ser Ile Gly His Gln Asp Pro
               180                 185                 190
   Gly Ala Thr Ser Val Met Phe Met Met Gln Met Leu Ala Leu Ala Ala
           195                 200                 205
   Lys Glu
                                 210
<210> 29
   <211> 1422
   <212> DNA
   <213> Escherichia coli
<220>
   <221> CDS
   <222> (1)..(1422)
   <223> dhaM Kodierregion
<400> 29
   gtg atg gta aac ctg gtc ata gtt tca cat agc agc cga ctg gga gaa 48
   Met Met Val Asn Leu Val Ile Val Ser His Ser Ser Arg Leu Gly Glu
   1 5 10 15
   ggt gtc ggt gaa tta gcc cgt cag atg tta atg agt gat agt tgt aaa 96
   Gly Val Gly Glu Leu Ala Arg Gln Met Leu Met Ser Asp Ser Cys Lys
   20 25 30
   atc gcc att gcc gcg gga att gac gat cca caa aat ccc att ggt acc 144
   Ile Ala Ile Ala Ala Gly Ile Asp Asp Pro Gln Asn Pro Ile Gly Thr
   35 40 45
   gat gcc gtc aaa gtg atg gag gcc atc gaa tct gtt gct gat gcc gac 192
   Asp Ala Val Lys Val Met Glu Ala Ile Glu Ser Val Ala Asp Ala Asp
   50 55 60
   cat gtg ctg gtc atg atg gat atg ggt agc gca tta ttg agt gct gaa 240
   His Val Leu Val Met Met Asp Met Gly Ser Ala Leu Leu Ser Ala Glu
   65 70 75 80
   act gcg ctg gaa ttg ctg gct ccc gag atc gcc gca aaa gta cgt ttg 288
   Thr Ala Leu Glu Leu Leu Ala Pro Glu Ile Ala Ala Lys Val Arg Leu
   85 90 95
   tgt gct gcg ccg ttg gtc gaa ggt aca ctg gca gca acg gtc agc gcg 336
   Cys Ala Ala Pro Leu Val Glu Gly Thr Leu Ala Ala Thr Val Ser Ala
   100 105 110
   gcc tcg ggg gcg gat atc gac aaa gtt atc ttt gac gcc atg cat gcg 384
   Ala Ser Gly Ala Asp Ile Asp Lys Val Ile Phe Asp Ala Met His Ala
   115 120 125
   ctg gaa gcc aaa cgt gaa caa ctg ggt tta ccg tcc tcc gac act gaa 432
   Leu Glu Ala Lys Arg Glu Gln Leu Gly Leu Pro Ser Ser Asp Thr Glu
   130 135 140
   atc tct gac aca tgt cct gcg tac gat gaa gaa gcc cgt tct ctg gcg 480
   Ile Ser Asp Thr Cys Pro Ala Tyr Asp Glu Glu Ala Arg Ser Leu Ala
   145 150 155 160
   gtg gtc ata aaa aac cgt aac ggc ctg cat gta cgt ccg gcc tcc cgg 528
   Val Val Ile Lys Asn Arg Asn Gly Leu His Val Arg Pro Ala Ser Arg
   165 170 175
   ctg gtt tat acc tta tcg aca ttt aat gcc gat atg ttg ctg gaa aaa 576
   Leu Val Tyr Thr Leu Ser Thr Phe Asn Ala Asp Met Leu Leu Glu Lys
   180 185 190
   aac ggc aaa tgc gtc aca cca gag agt att aac cag att gcg tta cta       624
   Asn Gly Lys Cys Val Thr Pro Glu Ser Ile Asn Gln Ile Ala Leu Leu
           195                 200                 205
   caa gtt cgc tat aac gat acg ctg cgc ctg att gcg aaa ggg cca gaa       672
   Gln Val Arg Tyr Asn Asp Thr Leu Arg Leu Ile Ala Lys Gly Pro Glu
       210                 215                 220
   gct gaa gag gca ctg atc gct ttc cgt cag ctg gct gaa gat aac ttt       720
   Ala Glu Glu Ala Leu Ile Ala Phe Arg Gln Leu Ala Glu Asp Asn Phe
   225                 230                 235                 240
   ggt gaa acg gag gaa gtc gct cca cct act ctg cgt ccc gtt ccg cct       768
   Gly Glu Thr Glu Glu Val Ala Pro Pro Thr Leu Arg Pro Val Pro Pro
                   245                 250                 255
   gtt tcg ggt aaa gcc ttt tat tat caa cca gtt tta tgt acg gta cag       816
   Val Ser Gly Lys Ala Phe Tyr Tyr Gln Pro Val Leu Cys Thr Val Gln
               260                 265                 270
   gca aaa tca acc ctg acc gtg gaa gaa gaa caa gat cga tta cgc cag       864
   Ala Lys Ser Thr Leu Thr Val Glu Glu Glu Gln Asp Arg Leu Arg Gln
           275                 280                 285
   gct att gac ttc acg tta tta gat ctg atg acg tta aca gcg aaa gca       912
   Ala Ile Asp Phe Thr Leu Leu Asp Leu Met Thr Leu Thr Ala Lys Ala
       290                 295                 300
   gaa gcc agc ggg ctt gac gat att gcc gca atc ttt tct ggt cac cat       960
   Glu Ala Ser Gly Leu Asp Asp Ile Ala Ala Ile Phe Ser Gly His His
   305                 310                 315                 320
   aca ctg tta gat gat ccg gaa ctg ctg gcg gcg gca agc gaa ctc ctt      1008
   Thr Leu Leu Asp Asp Pro Glu Leu Leu Ala Ala Ala Ser Glu Leu Leu
                   325                 330                 335
   cag cat gaa cat tgc acg gca gaa tat gcc tgg cag caa gtt ctt aaa      1056
   Gln His Glu His Cys Thr Ala Glu Tyr Ala Trp Gln Gln Val Leu Lys
               340                 345                 350
   gaa ctt agc cag caa tac cag caa ctg gat gat gaa tat cta caa gct      1104
   Glu Leu Ser Gln Gln Tyr Gln Gln Leu Asp Asp Glu Tyr Leu Gln Ala
           355                 360                 365
   cgc tat att gat gtg gac gat ctt ctg cat cgc acc ctg gtc cac ctg      1152
   Arg Tyr Ile Asp Val Asp Asp Leu Leu His Arg Thr Leu Val His Leu
       370                 375                 380
   acc caa acg aaa gaa gaa ctc ccg cag ttt aac tcg cca act att cta      1200
   Thr Gln Thr Lys Glu Glu Leu Pro Gln Phe Asn Ser Pro Thr Ile Leu
   385                 390                 395                 400
   ctg gcg gag aac att tat cct tcc aca gta ctg caa ctg gat ccg gcg      1248
   Leu Ala Glu Asn Ile Tyr Pro Ser Thr Val Leu Gln Leu Asp Pro Ala
                   405                 410                 415
   gtt gta aaa ggt atc tgc ctt agc gcc gga agt ccg gta tcc cac agc      1296
   Val Val Lys Gly Ile Cys Leu Ser Ala Gly Ser Pro Val Ser His Ser
               420                 425                 430
   gcc cta atc gcc cgt gaa ctg ggg att ggc tgg att tgc cag cag ggt      1344
   Ala Leu Ile Ala Arg Glu Leu Gly Ile Gly Trp Ile Cys Gln Gln Gly
           435                 440                 445
   gag aaa ctg tat gcg ata caa cca gaa gaa acg cta acg ctg gac gtt      1392
   Glu Lys Leu Tyr Ala Ile Gln Pro Glu Glu Thr Leu Thr Leu Asp Val
       450                 455                 460
   aaa acg caa cgt ttc aac cgt cag ggt taa                              1422
   Lys Thr Gln Arg Phe Asn Arg Gln Gly
   465                 470
<210> 30
   <211> 473
   <212> PRT
   <213> Escherichia coli
<400> 30
   Met Met Val Asn Leu Val Ile Val Ser His Ser Ser Arg Leu Gly Glu
   1               5                   10                  15
   Gly Val Gly Glu Leu Ala Arg Gln Met Leu Met Ser Asp Ser Cys Lys
               20                  25                  30
   Ile Ala Ile Ala Ala Gly Ile Asp Asp Pro Gln Asn Pro Ile Gly Thr
           35                  40                  45
   Asp Ala Val Lys Val Met Glu Ala Ile Glu Ser Val Ala Asp Ala Asp
       50                  55                  60
   His Val Leu Val Met Met Asp Met Gly Ser Ala Leu Leu Ser Ala Glu
   65                  70                  75                  80
   Thr Ala Leu Glu Leu Leu Ala Pro Glu Ile Ala Ala Lys Val Arg Leu
                   85                  90                  95
   Cys Ala Ala Pro Leu Val Glu Gly Thr Leu Ala Ala Thr Val Ser Ala
               100                 105                 110
   Ala Ser Gly Ala Asp Ile Asp Lys Val Ile Phe Asp Ala Met His Ala
           115                 120                 125
   Leu Glu Ala Lys Arg Glu Gln Leu Gly Leu Pro Ser Ser Asp Thr Glu
       130                 135                 140
   Ile Ser Asp Thr Cys Pro Ala Tyr Asp Glu Glu Ala Arg Ser Leu Ala
   145                 150                 155                 160
   Val Val Ile Lys Asn Arg Asn Gly Leu His Val Arg Pro Ala Ser Arg
                   165                 170                 175
   Leu Val Tyr Thr Leu Ser Thr Phe Asn Ala Asp Met Leu Leu Glu Lys
               180                 185                 190
   Asn Gly Lys Cys Val Thr Pro Glu Ser Ile Asn Gln Ile Ala Leu Leu
           195                 200                 205
   Gln Val Arg Tyr Asn Asp Thr Leu Arg Leu Ile Ala Lys Gly Pro Glu
       210                 215                 220
   Ala Glu Glu Ala Leu Ile Ala Phe Arg Gln Leu Ala Glu Asp Asn Phe
   225                 230                 235                 240
   Gly Glu Thr Glu Glu Val Ala Pro Pro Thr Leu Arg Pro Val Pro Pro
                   245                 250                 255
   Val Ser Gly Lys Ala Phe Tyr Tyr Gln Pro Val Leu Cys Thr Val Gln
               260                 265                 270
   Ala Lys Ser Thr Leu Thr Val Glu Glu Glu Gln Asp Arg Leu Arg Gln
           275                 280                 285
   Ala Ile Asp Phe Thr Leu Leu Asp Leu Met Thr Leu Thr Ala Lys Ala
       290                 295                 300
   Glu Ala Ser Gly Leu Asp Asp Ile Ala Ala Ile Phe Ser Gly His His
   305                 310                 315                 320
   Thr Leu Leu Asp Asp Pro Glu Leu Leu Ala Ala Ala Ser Glu Leu Leu
                   325                 330                 335
   Gln His Glu His Cys Thr Ala Glu Tyr Ala Trp Gln Gln Val Leu Lys
               340                 345                 350
   Glu Leu Ser Gln Gln Tyr Gln Gln Leu Asp Asp Glu Tyr Leu Gln Ala
           355                 360                 365
   Arg Tyr Ile Asp Val Asp Asp Leu Leu His Arg Thr Leu Val His Leu
       370                 375                 380
   Thr Gln Thr Lys Glu Glu Leu Pro Gln Phe Asn Ser Pro Thr Ile Leu
   385                 390                 395                 400
   Leu Ala Glu Asn Ile Tyr Pro Ser Thr Val Leu Gln Leu Asp Pro Ala
                   405                 410                 415
   Val Val Lys Gly Ile Cys Leu Ser Ala Gly Ser Pro Val Ser His Ser
                   420                 425                 430
   Ala Leu Ile Ala Arg Glu Leu Gly Ile Gly Trp Ile Cys Gln Gln Gly
           435                 440                 445
   Glu Lys Leu Tyr Ala Ile Gln Pro Glu Glu Thr Leu Thr Leu Asp Val
       450                 455                 460
   Lys Thr Gln Arg Phe Asn Arg Gln Gly
   465                 470
<210> 31
   <211> 1929
   <212> DNA
   <213> Escherichia coli
<220>
   <221> CDS
   <222> (1)..(1929)
   <223> dhaR Kodierregion
<400> 31
   atg cgg gat atg agt ggc gct ttt aac aac gat ggt cgg ggc ata tct       48
   Met Arg Asp Met Ser Gly Ala Phe Asn Asn Asp Gly Arg Gly Ile Ser
   1               5                   10                  15
   ccc tta att gca acc tcc tgg gag cga tgc aat aag ctg atg aaa cgg       96
   Pro Leu Ile Ala Thr Ser Trp Glu Arg Cys Asn Lys Leu Met Lys Arg
               20                  25                  30
   gag aca tgg aac gta cca cat cag gcc cag ggc gtg aca ttt gct tct      144
   Glu Thr Trp Asn Val Pro His Gln Ala Gln Gly Val Thr Phe Ala Ser
           35                  40                  45
   att tat cgg cgt aag aaa gcg atg ctg acg ctc ggg cag gct gcg ctg      192
   Ile Tyr Arg Arg Lys Lys Ala Met Leu Thr Leu Gly Gln Ala Ala Leu
       50                  55                  60
   gaa gat gcc tgg gaa tat atg gca ccg cga gag tgt gcg ctg ttt atc      240
   Glu Asp Ala Trp Glu Tyr Met Ala Pro Arg Glu Cys Ala Leu Phe Ile
   65                  70                  75                  80
   ctc gat gaa acc gcc tgc att ctc agc cgt aat ggc gat ccg caa acc      288
   Leu Asp Glu Thr Ala Cys Ile Leu Ser Arg Asn Gly Asp Pro Gln Thr
                   85                  90                  95
   ttg cag cag cta agt gca ctg gga ttc aat gac ggc acg tat tgc gcc      336
   Leu Gln Gln Leu Ser Ala Leu Gly Phe Asn Asp Gly Thr Tyr Cys Ala
               100                 105                 110
   gag gga att att ggt act tgt gcg cta tcg tta gcg gct atc tct ggt      384
   Glu Gly Ile Ile Gly Thr Cys Ala Leu Ser Leu Ala Ala Ile Ser Gly
           115                 120                 125
   cag gcc gtg aaa acg atg gcc gat caa cat ttc aaa cag gta ctc tgg      432
   Gln Ala Val Lys Thr Met Ala Asp Gln His Phe Lys Gln Val Leu Trp
       130                 135                 140
   aac tgg gcc ttt tgt gca acg ccg ttg ttt gac agc aag ggc cga ttg      480
   Asn Trp Ala Phe Cys Ala Thr Pro Leu Phe Asp Ser Lys Gly Arg Leu
   145                 150                 155                 160
   acg gga aca ata gcg ctg gcg tgt ccg gtt gag caa act acc gca gct      528
   Thr Gly Thr Ile Ala Leu Ala Cys Pro Val Glu Gln Thr Thr Ala Ala
                   165                 170                 175
   gat ttg ccg ttg acg ttg gca atc gcc cgc gag gtc gga aat tta ctg      576
   Asp Leu Pro Leu Thr Leu Ala Ile Ala Arg Glu Val Gly Asn Leu Leu
               180                 185                 190
   ctg acg gac agt ttg ctc gct gaa act aac cgt cat tta aat caa ctt      624
   Leu Thr Asp Ser Leu Leu Ala Glu Thr Asn Arg His Leu Asn Gln Leu
           195                 200                 205
   aat gcc ctg tta gaa agt atg gat gat ggc gtg att agc tgg gac gag      672
   Asn Ala Leu Leu Glu Ser Met Asp Asp Gly Val Ile Ser Trp Asp Glu
       210                 215                 220
   cag ggt aat ttg caa ttt att aat gcc cag gcg gcg cgg gtc ttg cgc      720
   Gln Gly Asn Leu Gln Phe Ile Asn Ala Gln Ala Ala Arg Val Leu Arg
   225                 230                 235                 240
   ctt gac gcg acg gca agt cag gga cgg gca atc act gaa ctc tta acg      768
   Leu Asp Ala Thr Ala Ser Gln Gly Arg Ala Ile Thr Glu Leu Leu Thr
                   245                 250                 255
   tta ccc gcc gta ttg caa caa gca ata aaa cag gca cat ccg ctc aaa      816
   Leu Pro Ala Val Leu Gln Gln Ala Ile Lys Gln Ala His Pro Leu Lys
               260                 265                 270
   cac gta gaa gca acc ttt gaa agc cag cac cag ttt att gat gcg gtg      864
   His Val Glu Ala Thr Phe Glu Ser Gln His Gln Phe Ile Asp Ala Val
           275                 280                 285
   ata acc ctt aaa ccg ata ata gaa acg cag gga acc agc ttt att ttg      912
   Ile Thr Leu Lys Pro Ile Ile Glu Thr Gln Gly Thr Ser Phe Ile Leu
       290                 295                 300
   ttg ctc cat cct gtg gaa cag atg cgg cag ttg atg acc agt caa tta      960
   Leu Leu His Pro Val Glu Gln Met Arg Gln Leu Met Thr Ser Gln Leu
   305                 310                 315                 320
   gga aaa gtc agc cat acc ttc gct cat atg cca cag gac gat ccg caa     1008
   Gly Lys Val Ser His Thr Phe Ala His Met Pro Gln Asp Asp Pro Gln
                   325                 330                 335
   acc cgc cgc ttg att cat ttt ggt cgc cag gcg gcg cgc agt agc ttt     1056
   Thr Arg Arg Leu Ile His Phe Gly Arg Gln Ala Ala Arg Ser Ser Phe
               340                 345                 350
   cct gtc ctg ctt tgt gga gaa gag ggc gtg ggc aag gca ctg cta agt     1104
   Pro Val Leu Leu Cys Gly Glu Glu Gly Val Gly Lys Ala Leu Leu Ser
           355                 360                 365
   cag gca att cat aat gaa agc gag cgt gct gca ggt cct tat atc gcc     1152
   Gln Ala Ile His Asn Glu Ser Glu Arg Ala Ala Gly Pro Tyr Ile Ala
       370                 375                 380
   gtc aat tgt gag tta tat ggt gat gct gcg ctg gcg gaa gaa ttt att     1200
   Val Asn Cys Glu Leu Tyr Gly Asp Ala Ala Leu Ala Glu Glu Phe Ile
   385                 390                 395                 400
   ggt ggc gat cgc acg gac aat gaa aat ggc cgt ctg agt cgg ctg gaa      1248
   Gly Gly Asp Arg Thr Asp Asn Glu Asn Gly Arg Leu Ser Arg Leu Glu
               405                 410                 415
   ctg gca cac ggc ggc acg ctg ttt ctt gaa aag att gaa tat ctg gcg      1296
   Leu Ala His Gly Gly Thr Leu Phe Leu Glu Lys Ile Glu Tyr Leu Ala
           420                 425                 430
   gtg gag tta cag tct gct ttg ctt cag gtt atc aag cag ggg gtt atc      1344
   Val Glu Leu Gln Ser Ala Leu Leu Gln Val Ile Lys Gln Gly Val Ile
       435                 440                 445
   acg cga ctg gat gcg cgg cgt tta ata cca att gat gtc aaa gtg att      1392
   Thr Arg Leu Asp Ala Arg Arg Leu Ile Pro Ile Asp Val Lys Val Ile
       450                 455                 460
   gca aca acg acc gcg gac ctc gca atg ctg gtg gaa caa aat cgt ttt      1440
   Ala Thr Thr Thr Ala Asp Leu Ala Met Leu Val Glu Gln Asn Arg Phe
   465                 470                 475                 480
   agt cgc cag ctg tat tac gcg ctg cat gca ttt gaa att acc atc ccg      1488
   Ser Arg Gln Leu Tyr Tyr Ala Leu His Ala Phe Glu Ile Thr Ile Pro
               485                 490                 495
   cct ctg cgt atg cgg cgt ggc agc att ccg gcg ctg gtg aat aac aaa      1536
   Pro Leu Arg Met Arg Arg Gly Ser Ile Pro Ala Leu Val Asn Asn Lys
           500                 505                 510
   tta cgc agt ctt gaa aaa cgc ttc tct acg cgg ctg aaa att gat gac      1584
   Leu Arg Ser Leu Glu Lys Arg Phe Ser Thr Arg Leu Lys Ile Asp Asp
       515                 520                 525
   gat gcc ctc gct cgc ctg gtt tct tgt gca tgg cca ggc aac gat ttt      1632
   Asp Ala Leu Ala Arg Leu Val Ser Cys Ala Trp Pro Gly Asn Asp Phe
       530                 535                 540
   gaa ctt tac agc gtc atc gag aat ctt gct ctg agt agt gat aac ggg      1680
   Glu Leu Tyr Ser Val Ile Glu Asn Leu Ala Leu Ser Ser Asp Asn Gly
   545                 550                 555                 560
   cgc att cgc gtc agt gat ttg ccg gaa cat ctg ttt acc gag cag gcg      1728
   Arg Ile Arg Val Ser Asp Leu Pro Glu His Leu Phe Thr Glu Gln Ala
               565                 570                 575
   aca gat gat gtc agc gcc acc cgc ctt tcc acc agt ctg tca ttt gcg      1776
   Thr Asp Asp Val Ser Ala Thr Arg Leu Ser Thr Ser Leu Ser Phe Ala
           580                 585                 590
   gaa gtt gaa aaa gag gca att att aac gca gcc cag gtc aca ggc ggt      1824
   Glu Val Glu Lys Glu Ala Ile Ile Asn Ala Ala Gln Val Thr Gly Gly
       595                 600                 605
   cgc att cag gaa atg tcg gct tta ctt ggg atc ggc cgc act acg ctg      1872
   Arg Ile Gln Glu Met Ser Ala Leu Leu Gly Ile Gly Arg Thr Thr Leu
       610                 615                 620
   tgg cgg aaa atg aag caa cat ggc att gat gca ggg cag ttt aag cgc      1920
   Trp Arg Lys Met Lys Gln His Gly Ile Asp Ala Gly Gln Phe Lys Arg
   625                 630                 635                 640
   cgg gta tga                                                          1929
   Arg Val
<210> 32
   <211> 642
   <212> PRT
   <213> Escherichia coli
<400> 32
   Met Arg Asp Met Ser Gly Ala Phe Asn Asn Asp Gly Arg Gly Ile Ser
   1               5                   10                  15
   Pro Leu Ile Ala Thr Ser Trp Glu Arg Cys Asn Lys Leu Met Lys Arg
               20                  25                  30
   Glu Thr Trp Asn Val Pro His Gln Ala Gln Gly Val Thr Phe Ala Ser
           35                  40                  45
   Ile Tyr Arg Arg Lys Lys Ala Met Leu Thr Leu Gly Gln Ala Ala Leu
       50                  55                  60
   Glu Asp Ala Trp Glu Tyr Met Ala Pro Arg Glu Cys Ala Leu Phe Ile
   65                  70                  75                  80
   Leu Asp Glu Thr Ala Cys Ile Leu Ser Arg Asn Gly Asp Pro Gln Thr
                   85                  90                  95
   Leu Gln Gln Leu Ser Ala Leu Gly Phe Asn Asp Gly Thr Tyr Cys Ala
               100                 105                 110
   Glu Gly Ile Ile Gly Thr Cys Ala Leu Ser Leu Ala Ala Ile Ser Gly
           115                 120                 125
   Gln Ala Val Lys Thr Met Ala Asp Gln His Phe Lys Gln Val Leu Trp
       130                 135                 140
   Asn Trp Ala Phe Cys Ala Thr Pro Leu Phe Asp Ser Lys Gly Arg Leu
   145                 150                 155                 160
   Thr Gly Thr Ile Ala Leu Ala Cys Pro Val Glu Gln Thr Thr Ala Ala
                   165                 170                 175
   Asp Leu Pro Leu Thr Leu Ala Ile Ala Arg Glu Val Gly Asn Leu Leu
               180                 185                 190
   Leu Thr Asp Ser Leu Leu Ala Glu Thr Asn Arg His Leu Asn Gln Leu
           195                 200                 205
   Asn Ala Leu Leu Glu Ser Met Asp Asp Gly Val Ile Ser Trp Asp Glu
       210                 215                 220
   Gln Gly Asn Leu Gln Phe Ile Asn Ala Gln Ala Ala Arg Val Leu Arg
   225                 230                 235                 240
   Leu Asp Ala Thr Ala Ser Gln Gly Arg Ala Ile Thr Glu Leu Leu Thr
                   245                 250                 255
   Leu Pro Ala Val Leu Gln Gln Ala Ile Lys Gln Ala His Pro Leu Lys
               260                 265                 270
   His Val Glu Ala Thr Phe Glu Ser Gln His Gln Phe Ile Asp Ala Val
           275                 280                 285
   Ile Thr Leu Lys Pro Ile Ile Glu Thr Gln Gly Thr Ser Phe Ile Leu
       290                 295                 300
   Leu Leu His Pro Val Glu Gln Met Arg Gln Leu Met Thr Ser Gln Leu
   305                 310                 315                 320
   Gly Lys Val Ser His Thr Phe Ala His Met Pro Gln Asp Asp Pro Gln
                   325                 330                 335
   Thr Arg Arg Leu Ile His Phe Gly Arg Gln Ala Ala Arg Ser Ser Phe
               340                 345                 350
   Pro Val Leu Leu Cys Gly Glu Glu Gly Val Gly Lys Ala Leu Leu Ser
           355                 360                 365
   Gln Ala Ile His Asn Glu Ser Glu Arg Ala Ala Gly Pro Tyr Ile Ala
       370                 375                 380
   Val Asn Cys Glu Leu Tyr Gly Asp Ala Ala Leu Ala Glu Glu Phe Ile
   385                 390                 395                 400
   Gly Gly Asp Arg Thr Asp Asn Glu Asn Gly Arg Leu Ser Arg Leu Glu
                   405                 410                 415
   Leu Ala His Gly Gly Thr Leu Phe Leu Glu Lys Ile Glu Tyr Leu Ala
               420                 425                 430
   Val Glu Leu Gln Ser Ala Leu Leu Gln Val Ile Lys Gln Gly Val Ile
           435                 440                 445
   Thr Arg Leu Asp Ala Arg Arg Leu Ile Pro Ile Asp Val Lys Val Ile
       450                 455                 460
   Ala Thr Thr Thr Ala Asp Leu Ala Met Leu Val Glu Gln Asn Arg Phe
   465                 470                 475                 480
   Ser Arg Gln Leu Tyr Tyr Ala Leu His Ala Phe Glu Ile Thr Ile Pro
                   485                 490                 495
   Pro Leu Arg Met Arg Arg Gly Ser Ile Pro Ala Leu Val Asn Asn Lys
               500                 505                 510
   Leu Arg Ser Leu Glu Lys Arg Phe Ser Thr Arg Leu Lys Ile Asp Asp
           515                 520                 525
   Asp Ala Leu Ala Arg Leu Val Ser Cys Ala Trp Pro Gly Asn Asp Phe
       530                 535                 540
   Glu Leu Tyr Ser Val Ile Glu Asn Leu Ala Leu Ser Ser Asp Asn Gly
   545                 550                 555                 560
   Arg Ile Arg Val Ser Asp Leu Pro Glu His Leu Phe Thr Glu Gln Ala
                   565                 570                 575
   Thr Asp Asp Val Ser Ala Thr Arg Leu Ser Thr Ser Leu Ser Phe Ala
               580                 585                 590
   Glu Val Glu Lys Glu Ala Ile Ile Asn Ala Ala Gln Val Thr Gly Gly
           595                 600                 605
   Arg Ile Gln Glu Met Ser Ala Leu Leu Gly Ile Gly Arg Thr Thr Leu
       610                 615                 620
   Trp Arg Lys Met Lys Gln His Gly Ile Asp Ala Gly Gln Phe Lys Arg
   625                 630                 635                 640
   Arg Val
<210> 33
   <211> 735
   <212> DNA
   <213> Escherichia coli
<220>
   <221> CDS
   <222> (1)..(735)
   <223> fsa Kodierregion
<400> 33
   gtg cgt caa ctg ttc aga gag ttt tcc cgt gat agt cta cat tca gac       48
   Met Arg Gln Leu Phe Arg Glu Phe Ser Arg Asp Ser Leu His Ser Asp
   1               5                   10                  15
   aaa aag tac att ttg agg atg gtt atg gaa ctg tat ctg gat act tca       96
   Lys Lys Tyr Ile Leu Arg Met Val Met Glu Leu Tyr Leu Asp Thr Ser
               20                  25                  30
   gac gtt gtt gcg gtg aag gcg ctg tca cgt att ttt ccg ctg gcg ggt      144
   Asp Val Val Ala Val Lys Ala Leu Ser Arg Ile Phe Pro Leu Ala Gly
           35                  40                  45
   gtg acc act aac cca agc att atc gcc gcg ggt aaa aaa ccg ctg gat      192
   Val Thr Thr Asn Pro Ser Ile Ile Ala Ala Gly Lys Lys Pro Leu Asp
       50                  55                  60
   gtt gtg ctt ccg caa ctt cat gaa gcg atg ggc ggt cag ggg cgt ctg      240
   Val Val Leu Pro Gln Leu His Glu Ala Met Gly Gly Gln Gly Arg Leu
   65                  70                  75                  80
   ttt gcc cag gta atg gct acc act gcc gaa ggg atg gtt aat gac gcg      288
   Phe Ala Gln Val Met Ala Thr Thr Ala Glu Gly Met Val Asn Asp Ala
                   85                  90                  95
   ctt aag ctg cgt tct att att gcg gat atc gtg gtg aaa gtt ccg gtg      336
   Leu Lys Leu Arg Ser Ile Ile Ala Asp Ile Val Val Lys Val Pro Val
               100                 105                 110
   acc gcc gag ggg ctg gca gct att aag atg tta aaa gcg gaa ggg att      384
   Thr Ala Glu Gly Leu Ala Ala Ile Lys Met Leu Lys Ala Glu Gly Ile
           115                 120                 125
   ccg acg ctg gga acc gcg gta tat ggc gca gca caa ggg ctg ctg tcg      432
   Pro Thr Leu Gly Thr Ala Val Tyr Gly Ala Ala Gln Gly Leu Leu Ser
       130                 135                 140
   gcg ctg gca ggt gcg gaa tat gtt gcg cct tac gtt aat cgt att gat      480
   Ala Leu Ala Gly Ala Glu Tyr Val Ala Pro Tyr Val Asn Arg Ile Asp
   145                 150                 155                 160
   gct cag ggc ggt agc ggc att cag act gtg acc gac tta cac cag tta      528
   Ala Gln Gly Gly Ser Gly Ile Gln Thr Val Thr Asp Leu His Gln Leu
                   165                 170                 175
   ttg aaa atg cat gcg ccg cag gcg aaa gtg ctg gca gcg agt ttc aaa      576
   Leu Lys Met His Ala Pro Gln Ala Lys Val Leu Ala Ala Ser Phe Lys
               180                 185                 190
   acc ccg cgt cag gcg ctg gac tgc tta ctg gca gga tgt gaa tca att      624
   Thr Pro Arg Gln Ala Leu Asp Cys Leu Leu Ala Gly Cys Glu Ser Ile
           195                 200                 205
   act ctg cca ctg gat gtg gca caa cag atg att agc tat ccg gcg gtt      672
   Thr Leu Pro Leu Asp Val Ala Gln Gln Met Ile Ser Tyr Pro Ala Val
       210                 215                 220
   gat gcc gct gtg gcg aag ttt gag cag gac tgg cag gga gcg ttt ggc      720
   Asp Ala Ala Val Ala Lys Phe Glu Gln Asp Trp Gln Gly Ala Phe Gly
   225                 230                 235                 240
   aga acg tcg att taa                                                  735
   Arg Thr Ser Ile
<210> 34
   <211> 244
   <212> PRT
   <213> Escherichia coli
<400> 34
   Met Arg Gln Leu Phe Arg Glu Phe Ser Arg Asp Ser Leu His Ser Asp
   1               5                   10                  15
   Lys Lys Tyr Ile Leu Arg Met Val Met Glu Leu Tyr Leu Asp Thr Ser
               20                  25                  30
   Asp Val Val Ala Val Lys Ala Leu Ser Arg Ile Phe Pro Leu Ala Gly
           35                  40                  45
   Val Thr Thr Asn Pro Ser Ile Ile Ala Ala Gly Lys Lys Pro Leu Asp
       50                  55                  60
   Val Val Leu Pro Gln Leu His Glu Ala Met Gly Gly Gln Gly Arg Leu
   65                  70                  75                  80
   Phe Ala Gln Val Met Ala Thr Thr Ala Glu Gly Met Val Asn Asp Ala
                   85                  90                  95
   Leu Lys Leu Arg Ser Ile Ile Ala Asp Ile Val Val Lys Val Pro Val
               100                 105                 110
   Thr Ala Glu Gly Leu Ala Ala Ile Lys Met Leu Lys Ala Glu Gly Ile
           115                 120                 125
   Pro Thr Leu Gly Thr Ala Val Tyr Gly Ala Ala Gln Gly Leu Leu Ser
       130                 135                 140
   Ala Leu Ala Gly Ala Glu Tyr Val Ala Pro Tyr Val Asn Arg Ile Asp
   145                 150                 155                 160
   Ala Gln Gly Gly Ser Gly Ile Gln Thr Val Thr Asp Leu His Gln Leu
                   165                 170                 175
   Leu Lys Met His Ala Pro Gln Ala Lys Val Leu Ala Ala Ser Phe Lys
               180                 185                 190
   Thr Pro Arg Gln Ala Leu Asp Cys Leu Leu Ala Gly Cys Glu Ser Ile
           195                 200                 205
   Thr Leu Pro Leu Asp Val Ala Gln Gln Met Ile Ser Tyr Pro Ala Val
       210                 215                 220
   Asp Ala Ala Val Ala Lys Phe Glu Gln Asp Trp Gln Gly Ala Phe Gly
   225                 230                 235                 240
   Arg Thr Ser Ile
<210> 35
   <211> 663
   <212> DNA
   <213> Escherichia coli
<220>
   <221> CDS
   <222> (1)..(663)
   <223> talC Kodierregion
<400> 35
   atg gaa ctg tat ctg gac acc gct aac gtc gca gaa gtc gaa cgt ctg        48
   Met Glu Leu Tyr Leu Asp Thr Ala Asn Val Ala Glu Val Glu Arg Leu
   1               5                   10                  15
   gca cgc ata ttc ccc att gcc ggg gtg aca act aac ccg agc att atc        96
   Ala Arg Ile Phe Pro Ile Ala Gly Val Thr Thr Asn Pro Ser Ile Ile
               20                  25                  30
   gct gcc agc aag gag tcc ata tgg gaa gtg ctg ccg cgt ctg caa aaa       144
   Ala Ala Ser Lys Glu Ser Ile Trp Glu Val Leu Pro Arg Leu Gln Lys
           35                  40                  45
   gcg att ggt gat gag ggc att ctg ttt gct cag acc atg agc cgc gac       192
   Ala Ile Gly Asp Glu Gly Ile Leu Phe Ala Gln Thr Met Ser Arg Asp
       50                  55                  60
   gcg cag ggg atg gtg gaa gaa gcg aag cgc ctg cgc gac gct att ccg       240
   Ala Gln Gly Met Val Glu Glu Ala Lys Arg Leu Arg Asp Ala Ile Pro
   65                  70                  75                  80
   ggt att gtg gtg aaa atc ccg gtg act tcc gaa ggt ctg gca gca att       288
   Gly Ile Val Val Lys Ile Pro Val Thr Ser Glu Gly Leu Ala Ala Ile
                   85                  90                  95
   aaa ata ctg aaa aaa gag ggt att act aca ctt ggc act gct gta tat       336
   Lys Ile Leu Lys Lys Glu Gly Ile Thr Thr Leu Gly Thr Ala Val Tyr
               100                 105                 110
   agc gcc gca caa ggg tta tta gcc gca ctg gca ggg gca aaa tac gtt       384
   Ser Ala Ala Gln Gly Leu Leu Ala Ala Leu Ala Gly Ala Lys Tyr Val
           115                 120                 125
   gct ccg tat gtt aac cgc gta gat gcc cag ggc gga gac ggc att cgt       432
   Ala Pro Tyr Val Asn Arg Val Asp Ala Gln Gly Gly Asp Gly Ile Arg
       130                 135                 140
   acg gtt cag gag ctg caa acg ctg tta gaa atg cac gcg cca gaa agc       480
   Thr Val Gln Glu Leu Gln Thr Leu Leu Glu Met His Ala Pro Glu Ser
   145                 150                 155                 160
   atg gtg ctg gca gcc agc ttt aaa acg ccg cgt cag gcg ctg gac tgt       528
   Met Val Leu Ala Ala Ser Phe Lys Thr Pro Arg Gln Ala Leu Asp Cys
                   165                 170                 175
   tta ctg gca gga tgt gaa tcc atc acc ctg ccc tta gat gta gcg caa       576
   Leu Leu Ala Gly Cys Glu Ser Ile Thr Leu Pro Leu Asp Val Ala Gln
               180                 185                 190
   caa atg ctc aac acc cct gcg gta gag tca gct ata gag aag ttc gaa       624
   Gln Met Leu Asn Thr Pro Ala Val Glu Ser Ala Ile Glu Lys Phe Glu
           195                 200                 205
   cac gac tgg aat gcc gca ttt ggc act act cat ctc taa                   663
   His Asp Trp Asn Ala Ala Phe Gly Thr Thr His Leu
       210                 215                 220
<210> 36
   <211> 220
   <212> PRT
   <213> Escherichia coli
<400> 36
   Met Glu Leu Tyr Leu Asp Thr Ala Asn Val Ala Glu Val Glu Arg Leu
   1               5                   10                  15
   Ala Arg Ile Phe Pro Ile Ala Gly Val Thr Thr Asn Pro Ser Ile Ile
               20                  25                  30
   Ala Ala Ser Lys Glu Ser Ile Trp Glu Val Leu Pro Arg Leu Gln Lys
           35                  40                  45
   Ala Ile Gly Asp Glu Gly Ile Leu Phe Ala Gln Thr Met Ser Arg Asp
       50                  55                  60
   Ala Gln Gly Met Val Glu Glu Ala Lys Arg Leu Arg Asp Ala Ile Pro
   65                  70                  75                  80
   Gly Ile Val Val Lys Ile Pro Val Thr Ser Glu Gly Leu Ala Ala Ile
                   85                  90                  95
   Lys Ile Leu Lys Lys Glu Gly Ile Thr Thr Leu Gly Thr Ala Val Tyr
               100                 105                 110
   Ser Ala Ala Gln Gly Leu Leu Ala Ala Leu Ala Gly Ala Lys Tyr Val
           115                 120                 125
   Ala Pro Tyr Val Asn Arg Val Asp Ala Gln Gly Gly Asp Gly Ile Arg
       130                 135                 140
   Thr Val Gln Glu Leu Gln Thr Leu Leu Glu Met His Ala Pro Glu Ser
   145                 150                 155                 160
   Met Val Leu Ala Ala Ser Phe Lys Thr Pro Arg Gln Ala Leu Asp Cys
                   165                 170                 175
   Leu Leu Ala Gly Cys Glu Ser Ile Thr Leu Pro Leu Asp Val Ala Gln
               180                 185                 190
   Gln Met Leu Asn Thr Pro Ala Val Glu Ser Ala Ile Glu Lys Phe Glu
           195                 200                 205
   His Asp Trp Asn Ala Ala Phe Gly Thr Thr His Leu
       210                 215                 220
<210> 37
   <211> 1530
   <212> DNA
   <213> Corynebacterium glutamicum
<220>
   <221> CDS
   <222> (1)..(1530)
   <223> glpK Kodierregion
<400> 37
   atg aga atc tca aag gcc aat gcg tat gtt gca gcg att gac caa ggc       48
   Met Arg Ile Ser Lys Ala Asn Ala Tyr Val Ala Ala Ile Asp Gln Gly
   1               5                   10                  15
   acc act tcc act cgg tgc atc ttc att gat gcc caa gga aaa gtg gtg       96
   Thr Thr Ser Thr Arg Cys Ile Phe Ile Asp Ala Gln Gly Lys Val Val
               20                  25                  30
   tct tct gct tcc aag gag cac cgc caa atc ttc cca caa cag ggc tgg      144
   Ser Ser Ala Ser Lys Glu His Arg Gln Ile Phe Pro Gln Gln Gly Trp
           35                  40                  45
   gta gag cac gat cct gaa gaa att tgg gac aac atc cga tct gtc gtc      192
   Val Glu His Asp Pro Glu Glu Ile Trp Asp Asn Ile Arg Ser Val Val
       50                  55                  60
   agc cag gcg atg gtc tcc att gac atc acc cca cac gag gtt gca tcg      240
   Ser Gln Ala Met Val Ser Ile Asp Ile Thr Pro His Glu Val Ala Ser
   65                  70                  75                  80
   ctg gga gtc acc aac cag cgc gaa acc acc gtg gtg tgg gac aag cac      288
   Leu Gly Val Thr Asn Gln Arg Glu Thr Thr Val Val Trp Asp Lys His
                   85                  90                  95
   acc ggc gaa cct gtc tac aac gca atc gtg tgg caa gac acc cgc acc      336
   Thr Gly Glu Pro Val Tyr Asn Ala Ile Val Trp Gln Asp Thr Arg Thr
               100                 105                 110
   tct gac att tgc cta gag atc gcg ggc gaa gaa ggc cag gaa aag tgg      384
   Ser Asp Ile Cys Leu Glu Ile Ala Gly Glu Glu Gly Gln Glu Lys Trp
           115                 120                 125
   ctt gac cgc acc ggc ctg ctg atc aac tcc tac cca tcg ggg ccc aaa      432
   Leu Asp Arg Thr Gly Leu Leu Ile Asn Ser Tyr Pro Ser Gly Pro Lys
       130                 135                 140
   atc aag tgg att ctc gac aac gtt gag gga gct cgc gaa cgc gcc gaa      480
   Ile Lys Trp Ile Leu Asp Asn Val Glu Gly Ala Arg Glu Arg Ala Glu
   145                 150                 155                 160
   aag ggc gac ctt ttg ttt ggc acc atg gat acc tgg gtg ctg tgg aac      528
   Lys Gly Asp Leu Leu Phe Gly Thr Met Asp Thr Trp Val Leu Trp Asn
                   165                 170                 175
   ctg acc ggc ggt gtc cgc ggc gac gac ggt gat gat gcc atc cac gtc      576
   Leu Thr Gly Gly Val Arg Gly Asp Asp Gly Asp Asp Ala Ile His Val
               180                 185                 190
   acc gat gtc acc aac gca tcc cgc aca cta ttg atg gat ctc cgc acg      624
   Thr Asp Val Thr Asn Ala Ser Arg Thr Leu Leu Met Asp Leu Arg Thr
           195                 200                 205
   caa cag tgg gat cca gaa cta tgc gaa gcc cta gac att ccg atg tcc      672
   Gln Gln Trp Asp Pro Glu Leu Cys Glu Ala Leu Asp Ile Pro Met Ser
       210                 215                 220
   atg ctc cct gag att cgt ccc tcc gtc gga gaa ttc cgc tcc gtg cgc      720
   Met Leu Pro Glu Ile Arg Pro Ser Val Gly Glu Phe Arg Ser Val Arg
   225                 230                 235                 240
   cac cgc gga acc cta gcc gac gtc ccg att act ggc gtg ctc ggc gac      768
   His Arg Gly Thr Leu Ala Asp Val Pro Ile Thr Gly Val Leu Gly Asp
                   245                 250                 255
   cag caa gcg gcc ctt ttt ggt cag ggc gga ttc cac gaa ggt gct gct      816
   Gln Gln Ala Ala Leu Phe Gly Gln Gly Gly Phe His Glu Gly Ala Ala
               260                 265                 270
   aaa aat acc tac ggc acc ggc ctc ttc ctg ctg atg aac acc ggc acc      864
   Lys Asn Thr Tyr Gly Thr Gly Leu Phe Leu Leu Met Asn Thr Gly Thr
           275                 280                 285
   tcg ttg aag att tcc gag cac ggc ctg ctg tcc acc atc gcc tat caa      912
   Ser Leu Lys Ile Ser Glu His Gly Leu Leu Ser Thr Ile Ala Tyr Gln
       290                 295                 300
   cgg gaa gga tcc gct ccg gtc tac gcg ctg gaa ggt tcc gta tcc atg      960
   Arg Glu Gly Ser Ala Pro Val Tyr Ala Leu Glu Gly Ser Val Ser Met
   305                 310                 315                 320
   ggc ggt tcc ttg gtg cag tgg ctg cgc gac aac cta cag cta atc ccc      1008
   Gly Gly Ser Leu Val Gln Trp Leu Arg Asp Asn Leu Gln Leu Ile Pro
                   325                 330                 335
   aac gca cca gcg att gaa aac ctc gcc cga gaa gtc gaa gac aac ggt      1056
   Asn Ala Pro Ala Ile Glu Asn Leu Ala Arg Glu Val Glu Asp Asn Gly
                   340                 345                 350
   ggc gtt cat gtt gtc cca gca ttc acc gga ctg ttc gca cca cgt tgg      1104
   Gly Val His Val Val Pro Ala Phe Thr Gly Leu Phe Ala Pro Arg Trp
               355                 360                 365
   cgc ccc gat gct cgt ggc gtc att aca ggc ctc acc cgt ttt gcc aac      1152
   Arg Pro Asp Ala Arg Gly Val Ile Thr Gly Leu Thr Arg Phe Ala Asn
       370                 375                 380
   cgc aaa cac atc gcc cgc gca gtc ctt gaa gcc aac gcc ttc caa acc      1200
   Arg Lys His Ile Ala Arg Ala Val Leu Glu Ala Asn Ala Phe Gln Thr
   385                 390                 395                 400
   cgc gaa gtt gtg gac gcc atg gcc aaa gac gca ggc aaa gcc ctc gaa      1248
   Arg Glu Val Val Asp Ala Met Ala Lys Asp Ala Gly Lys Ala Leu Glu
                   405                 410                 415
   tcc ctc cgc gtc gac ggt gcg atg gtg gaa aat gac ctc ctc atg caa      1296
   Ser Leu Arg Val Asp Gly Ala Met Val Glu Asn Asp Leu Leu Met Gln
               420                 425                 430
   atg caa gcc gac ttc ctc ggc atc gac gtc caa cgt ctc gag gac gta      1344
   Met Gln Ala Asp Phe Leu Gly Ile Asp Val Gln Arg Leu Glu Asp Val
           435                 440                 445
   gaa acc acc gcc gtc ggc gtc gca ttc gct gca ggt ctc ggc tct gga      1392
   Glu Thr Thr Ala Val Gly Val Ala Phe Ala Ala Gly Leu Gly Ser Gly
       450                 455                 460
   ttc ttc aaa aca act gac gag atc gaa aaa ctt att gca gtg aag aaa      1440
   Phe Phe Lys Thr Thr Asp Glu Ile Glu Lys Leu Ile Ala Val Lys Lys
   465                 470                 475                 480
   gtc tgg aac cct gac atg agc gaa gaa gag cgc gaa cgt cgc tat gcc      1488
   Val Trp Asn Pro Asp Met Ser Glu Glu Glu Arg Glu Arg Arg Tyr Ala
                   485                 490                 495
   gaa tgg aat agg gca gtg gag cat tct tat gac cag gcc tag              1530
   Glu Trp Asn Arg Ala Val Glu His Ser Tyr Asp Gln Ala
               500                 505
<210> 38
   <211> 509
   <212> PRT
   <213> Corynebacterium glutamicum
<400> 38
   Met Arg Ile Ser Lys Ala Asn Ala Tyr Val Ala Ala Ile Asp Gln Gly
   1               5                   10                  15
   Thr Thr Ser Thr Arg Cys Ile Phe Ile Asp Ala Gln Gly Lys Val Val
               20                  25                  30
   Ser Ser Ala Ser Lys Glu His Arg Gln Ile Phe Pro Gln Gln Gly Trp
           35                  40                  45
   Val Glu His Asp Pro Glu Glu Ile Trp Asp Asn Ile Arg Ser Val Val
       50                  55                  60
   Ser Gln Ala Met Val Ser Ile Asp Ile Thr Pro His Glu Val Ala Ser
   65                  70                  75                  80
   Leu Gly Val Thr Asn Gln Arg Glu Thr Thr Val Val Trp Asp Lys His
                   85                  90                  95
   Thr Gly Glu Pro Val Tyr Asn Ala Ile Val Trp Gln Asp Thr Arg Thr
               100                 105                 110
   Ser Asp Ile Cys Leu Glu Ile Ala Gly Glu Glu Gly Gln Glu Lys Trp
           115                 120                 125
   Leu Asp Arg Thr Gly Leu Leu Ile Asn Ser Tyr Pro Ser Gly Pro Lys
       130                 135                 140
   Ile Lys Trp Ile Leu Asp Asn Val Glu Gly Ala Arg Glu Arg Ala Glu
   145                 150                 155                 160
   Lys Gly Asp Leu Leu Phe Gly Thr Met Asp Thr Trp Val Leu Trp Asn
                   165                 170                 175
   Leu Thr Gly Gly Val Arg Gly Asp Asp Gly Asp Asp Ala Ile His Val
               180                 185                 190
   Thr Asp Val Thr Asn Ala Ser Arg Thr Leu Leu Met Asp Leu Arg Thr
           195                 200                 205
   Gln Gln Trp Asp Pro Glu Leu Cys Glu Ala Leu Asp Ile Pro Met Ser
       210                 215                 220
   Met Leu Pro Glu Ile Arg Pro Ser Val Gly Glu Phe Arg Ser Val Arg
   225                 230                 235                 240
   His Arg Gly Thr Leu Ala Asp Val Pro Ile Thr Gly Val Leu Gly Asp
                   245                 250                 255
   Gln Gln Ala Ala Leu Phe Gly Gln Gly Gly Phe His Glu Gly Ala Ala
               260                 265                 270
   Lys Asn Thr Tyr Gly Thr Gly Leu Phe Leu Leu Met Asn Thr Gly Thr
           275                 280                 285
   Ser Leu Lys Ile Ser Glu His Gly Leu Leu Ser Thr Ile Ala Tyr Gln
               290                 295                 300
   Arg Glu Gly Ser Ala Pro Val Tyr Ala Leu Glu Gly Ser Val Ser Met
   305                 310                 315                 320
   Gly Gly Ser Leu Val Gln Trp Leu Arg Asp Asn Leu Gln Leu Ile Pro
                   325                 330                 335
   Asn Ala Pro Ala Ile Glu Asn Leu Ala Arg Glu Val Glu Asp Asn Gly
               340                 345                 350
   Gly Val His Val Val Pro Ala Phe Thr Gly Leu Phe Ala Pro Arg Trp
           355                 360                 365
   Arg Pro Asp Ala Arg Gly Val Ile Thr Gly Leu Thr Arg Phe Ala Asn
       370                 375                 380
   Arg Lys His Ile Ala Arg Ala Val Leu Glu Ala Asn Ala Phe Gln Thr
   385                 390                 395                 400
   Arg Glu Val Val Asp Ala Met Ala Lys Asp Ala Gly Lys Ala Leu Glu
                   405                 410                 415
   Ser Leu Arg Val Asp Gly Ala Met Val Glu Asn Asp Leu Leu Met Gln
               420                 425                 430
   Met Gln Ala Asp Phe Leu Gly Ile Asp Val Gln Arg Leu Glu Asp Val
           435                 440                 445
   Glu Thr Thr Ala Val Gly Val Ala Phe Ala Ala Gly Leu Gly Ser Gly
       450                 455                 460
   Phe Phe Lys Thr Thr Asp Glu Ile Glu Lys Leu Ile Ala Val Lys Lys
   465                 470                 475                 480
   Val Trp Asn Pro Asp Met Ser Glu Glu Glu Arg Glu Arg Arg Tyr Ala
                   485                 490                 495
   Glu Trp Asn Arg Ala Val Glu His Ser Tyr Asp Gln Ala
               500                 505
<210> 39
   <211> 1725
   <212> DNA
   <213> Corynebacterium glutamicum
<220>
   <221> CDS
   <222> (1)..(1725)
   <223> glpD Kodierregion
<400> 39
   atg tgg gtt ggc ggt ttt cct att agg ctc act ttt atg acg agc gca      48
   Met Trp Val Gly Gly Phe Pro Ile Arg Leu Thr Phe Met Thr Ser Ala
   1               5                   10                  15
   cac ttt gaa tcc cgc cgg att ggc cct ccg ctt cgc gat aat tat gac      96
   His Phe Glu Ser Arg Arg Ile Gly Pro Pro Leu Arg Asp Asn Tyr Asp
               20                  25                  30
   gtc att gtg att ggc ggt ggt atc tca ggt gta cag att gcg cga cat     144
   Val Ile Val Ile Gly Gly Gly Ile Ser Gly Val Gln Ile Ala Arg His
           35                  40                  45
   gct caa ggc cgc ggt tta cgc act gtg atg ttt gag gcc aga gat tat     192
   Ala Gln Gly Arg Gly Leu Arg Thr Val Met Phe Glu Ala Arg Asp Tyr
       50                  55                  60
   tct tct gga aca tca tcg aca acc tcc aag atg att cat ggt ggt ttg     240
   Ser Ser Gly Thr Ser Ser Thr Thr Ser Lys Met Ile His Gly Gly Leu
   65                  70                  75                  80
   cgc tat ttg gag cag tac gat ttc ggc gtg gtc cag gaa gcc gtg aag     288
   Arg Tyr Leu Glu Gln Tyr Asp Phe Gly Val Val Gln Glu Ala Val Lys
                   85                  90                  95
   gaa cgc cgg tac cta ggt atc gcc gct ccg cat ttg gtg gct cca cgc     336
   Glu Arg Arg Tyr Leu Gly Ile Ala Ala Pro His Leu Val Ala Pro Arg
               100                 105                 110
   agt ttc atg ctc acg gcg ttt gat tgg tca gag ccg aaa gcc cct atg     384
   Ser Phe Met Leu Thr Ala Phe Asp Trp Ser Glu Pro Lys Ala Pro Met
           115                 120                 125
   ttg ggt gct ggt gtg gcg ttg tat gaa acg atg gcg tgg cag cgt aac     432
   Leu Gly Ala Gly Val Ala Leu Tyr Glu Thr Met Ala Trp Gln Arg Asn
       130                 135                 140
   cag ggg caa tcg aag gaa aac cac tcg ccg cgt ttc cgg tgg att cct     480
   Gln Gly Gln Ser Lys Glu Asn His Ser Pro Arg Phe Arg Trp Ile Pro
   145                 150                 155                 160
   aaa aat gca ctg ctc aag gaa gtc ccg tgg ctt gac ccg gag ggc ttg     528
   Lys Asn Ala Leu Leu Lys Glu Val Pro Trp Leu Asp Pro Glu Gly Leu
                   165                 170                 175
   aag gga gcg tgg cgc cac gat gat acg ttg aat ctc cat gca gaa cga     576
   Lys Gly Ala Trp Arg His Asp Asp Thr Leu Asn Leu His Ala Glu Arg
               180                 185                 190
   ctc ctc ctc gcg gtg att aaa gct ttt gcg gca gat ggc gga acg gcg     624
   Leu Leu Leu Ala Val Ile Lys Ala Phe Ala Ala Asp Gly Gly Thr Ala
           195                 200                 205
   atc aac cac gcc aaa gtc act cgc att ctc cgg aac gtg gaa gaa ggc     672
   Ile Asn His Ala Lys Val Thr Arg Ile Leu Arg Asn Val Glu Glu Gly
       210                 215                 220
   cgc gtc aag ggt gta gaa gtg act gat cag gtc acc aac acc acg cat     720
   Arg Val Lys Gly Val Glu Val Thr Asp Gln Val Thr Asn Thr Thr His
   225                 230                 235                 240
   gag gtg aat gcc cct gtg gtg atc aac gct gcg ggt ccg tgg gtt gcg      768
   Glu Val Asn Ala Pro Val Val Ile Asn Ala Ala Gly Pro Trp Val Ala
                   245                 250                 255
   cag gcg ttg ggt gat ttg gcg gag gta acc aag ttg aag gtg cgc caa      816
   Gln Ala Leu Gly Asp Leu Ala Glu Val Thr Lys Leu Lys Val Arg Gln
               260                 265                 270
   tcc aag gga gtg cat ttg ctc act ggt gat ttg ggc agc cag agt ggc      864
   Ser Lys Gly Val His Leu Leu Thr Gly Asp Leu Gly Ser Gln Ser Gly
           275                 280                 285
   gtg ttt gtg cgt ggc aaa aac ggc aag cat gtg atc gtg aat ccg tgg      912
   Val Phe Val Arg Gly Lys Asn Gly Lys His Val Ile Val Asn Pro Trp
       290                 295                 300
   atg ggg cgc acc ctt att ggt cca acc gac acc atg att gac ggt gac      960
   Met Gly Arg Thr Leu Ile Gly Pro Thr Asp Thr Met Ile Asp Gly Asp
   305                 310                 315                 320
   gct gat gat gcg gct gca gat gaa agc gat atc gat ttg ctg ctt gag     1008
   Ala Asp Asp Ala Ala Ala Asp Glu Ser Asp Ile Asp Leu Leu Leu Glu
                   325                 330                 335
   acc atc gat tcg gta cgc gct aca ccg ctt gat cgc aaa gag atc atc     1056
   Thr Ile Asp Ser Val Arg Ala Thr Pro Leu Asp Arg Lys Glu Ile Ile
               340                 345                 350
   tcc acg ctg gtg ggt gtg cgc ccg ctt gtt gat gac ggc acc gac acc     1104
   Ser Thr Leu Val Gly Val Arg Pro Leu Val Asp Asp Gly Thr Asp Thr
           355                 360                 365
   tac acg tcc tct cgc cgt ttc gat att tcc gat cac gcc aac gtc ggc     1152
   Tyr Thr Ser Ser Arg Arg Phe Asp Ile Ser Asp His Ala Asn Val Gly
       370                 375                 380
   att gat ggt ttg gtg tct gtc tct ggc ggc aag tgg acc act tcg cgc     1200
   Ile Asp Gly Leu Val Ser Val Ser Gly Gly Lys Trp Thr Thr Ser Arg
   385                 390                 395                 400
   gtg atg ggg tac aag gtg att gag cat gtg gtg gag cac caa gct gcg     1248
   Val Met Gly Tyr Lys Val Ile Glu His Val Val Glu His Gln Ala Ala
                   405                 410                 415
   gtg tta cct ccg ctg cgc cac ttt gac tcc agg cag atg ccg ttg agt     1296
   Val Leu Pro Pro Leu Arg His Phe Asp Ser Arg Gln Met Pro Leu Ser
               420                 425                 430
   act tct ttt ggc gcg tat gag tcc gtg gct gat tcc ttt gag tca gcg     1344
   Thr Ser Phe Gly Ala Tyr Glu Ser Val Ala Asp Ser Phe Glu Ser Ala
           435                 440                 445
   ctt cgc agc cac ccc gag ctg gat gtg gat gat gaa atc cgc gtg cat     1392
   Leu Arg Ser His Pro Glu Leu Asp Val Asp Asp Glu Ile Arg Val His
       450                 455                 460
   ctg gcc aga ctg tat gga act gag cat gaa aaa gtg ctg gat ctc gtc     1440
   Leu Ala Arg Leu Tyr Gly Thr Glu His Glu Lys Val Leu Asp Leu Val
   465                 470                 475                 480
   gca aag caa ccc gac ctg ggg cgc cga ctt gac cca gac aac ctt gat      1488
   Ala Lys Gln Pro Asp Leu Gly Arg Arg Leu Asp Pro Asp Asn Leu Asp
                   485                 490                 495
   atc gcg gcg cag gcc gtt ttt gct gtc gcc gag gag gcg gcc gtc gac      1536
   Ile Ala Ala Gln Ala Val Phe Ala Val Ala Glu Glu Ala Ala Val Asp
               500                 505                 510
   ctg gcg gac gtg ctg gat cgt cgc atc gtg ctc ggc acg ctg ggt tat      1584
   Leu Ala Asp Val Leu Asp Arg Arg Ile Val Leu Gly Thr Leu Gly Tyr
           515                 520                 525
   gtg caa ccg gct gcc gtg cgt gcg acg gcc gaa gca atg gcg cag gtc      1632
   Val Gln Pro Ala Ala Val Arg Ala Thr Ala Glu Ala Met Ala Gln Val
       530                 535                 540
   acc ggg tgg tca gct gag ctt atc gac gcc cag tgc cag tcc tac ctc      1680
   Thr Gly Trp Ser Ala Glu Leu Ile Asp Ala Gln Cys Gln Ser Tyr Leu
   545                 550                 555                 560
   gcc aag caa gac aaa atc caa gcc gtg tta aag ccg tac cgc taa          1725
   Ala Lys Gln Asp Lys Ile Gln Ala Val Leu Lys Pro Tyr Arg
                   565                 570
<210> 40
   <211> 574
   <212> PRT
   <213> Corynebacterium glutamicum
<400> 40
   Met Trp Val Gly Gly Phe Pro Ile Arg Leu Thr Phe Met Thr Ser Ala
   1               5                   10                  15
   His Phe Glu Ser Arg Arg Ile Gly Pro Pro Leu Arg Asp Asn Tyr Asp
               20                  25                  30
   Val Ile Val Ile Gly Gly Gly Ile Ser Gly Val Gln Ile Ala Arg His
           35                  40                  45
   Ala Gln Gly Arg Gly Leu Arg Thr Val Met Phe Glu Ala Arg Asp Tyr
       50                  55                  60
   Ser Ser Gly Thr Ser Ser Thr Thr Ser Lys Met Ile His Gly Gly Leu
   65                  70                  75                  80
   Arg Tyr Leu Glu Gln Tyr Asp Phe Gly Val Val Gln Glu Ala Val Lys
                   85                  90                  95
   Glu Arg Arg Tyr Leu Gly Ile Ala Ala Pro His Leu Val Ala Pro Arg
               100                 105                 110
   Ser Phe Met Leu Thr Ala Phe Asp Trp Ser Glu Pro Lys Ala Pro Met
           115                 120                 125
   Leu Gly Ala Gly Val Ala Leu Tyr Glu Thr Met Ala Trp Gln Arg Asn
       130                 135                 140
   Gln Gly Gln Ser Lys Glu Asn His Ser Pro Arg Phe Arg Trp Ile Pro
   145                 150                 155                 160
   Lys Asn Ala Leu Leu Lys Glu Val Pro Trp Leu Asp Pro Glu Gly Leu
                   165                 170                 175
   Lys Gly Ala Trp Arg His Asp Asp Thr Leu Asn Leu His Ala Glu Arg
               180                 185                 190
   Leu Leu Leu Ala Val Ile Lys Ala Phe Ala Ala Asp Gly Gly Thr Ala
           195                 200                 205
   Ile Asn His Ala Lys Val Thr Arg Ile Leu Arg Asn Val Glu Glu Gly
       210                 215                 220
   Arg Val Lys Gly Val Glu Val Thr Asp Gln Val Thr Asn Thr Thr His
   225                 230                 235                 240
   Glu Val Asn Ala Pro Val Val Ile Asn Ala Ala Gly Pro Trp Val Ala
                   245                 250                 255
   Gln Ala Leu Gly Asp Leu Ala Glu Val Thr Lys Leu Lys Val Arg Gln
               260                 265                 270
   Ser Lys Gly Val His Leu Leu Thr Gly Asp Leu Gly Ser Gln Ser Gly
           275                 280                 285
   Val Phe Val Arg Gly Lys Asn Gly Lys His Val Ile Val Asn Pro Trp
       290                 295                 300
   Met Gly Arg Thr Leu Ile Gly Pro Thr Asp Thr Met Ile Asp Gly Asp
   305                 310                 315                 320
   Ala Asp Asp Ala Ala Ala Asp Glu Ser Asp Ile Asp Leu Leu Leu Glu
                   325                 330                 335
   Thr Ile Asp Ser Val Arg Ala Thr Pro Leu Asp Arg Lys Glu Ile Ile
               340                 345                 350
   Ser Thr Leu Val Gly Val Arg Pro Leu Val Asp Asp Gly Thr Asp Thr
           355                 360                 365
   Tyr Thr Ser Ser Arg Arg Phe Asp Ile Ser Asp His Ala Asn Val Gly
       370                 375                 380
   Ile Asp Gly Leu Val Ser Val Ser Gly Gly Lys Trp Thr Thr Ser Arg
   385                 390                 395                 400
   Val Met Gly Tyr Lys Val Ile Glu His Val Val Glu His Gln Ala Ala
                   405                 410                 415
   Val Leu Pro Pro Leu Arg His Phe Asp Ser Arg Gln Met Pro Leu Ser
               420                 425                 430
   Thr Ser Phe Gly Ala Tyr Glu Ser Val Ala Asp Ser Phe Glu Ser Ala
           435                 440                 445
   Leu Arg Ser His Pro Glu Leu Asp Val Asp Asp Glu Ile Arg Val His
       450                 455                 460
   Leu Ala Arg Leu Tyr Gly Thr Glu His Glu Lys Val Leu Asp Leu Val
   465                 470                 475                 480
   Ala Lys Gln Pro Asp Leu Gly Arg Arg Leu Asp Pro Asp Asn Leu Asp
                   485                 490                 495
   Ile Ala Ala Gln Ala Val Phe Ala Val Ala Glu Glu Ala Ala Val Asp
               500                 505                 510
   Leu Ala Asp Val Leu Asp Arg Arg Ile Val Leu Gly Thr Leu Gly Tyr
           515                 520                 525
   Val Gln Pro Ala Ala Val Arg Ala Thr Ala Glu Ala Met Ala Gln Val
       530                 535                 540
   Thr Gly Trp Ser Ala Glu Leu Ile Asp Ala Gln Cys Gln Ser Tyr Leu
   545                 550                 555                 560
   Ala Lys Gln Asp Lys Ile Gln Ala Val Leu Lys Pro Tyr Arg
                   565                 570